(19) Europäisches Patentamt
European Patent Office
Office européen des brevets

(11) Numéro de publication : **0 341 158 B1**

(12) # FASCICULE DE BREVET EUROPEEN

(45) Date de publication du fascicule du brevet : **07.01.93 Bulletin 93/01**

(21) Numéro de dépôt : **89401262.4**

(22) Date de dépôt : **03.05.89**

(51) Int. Cl.$^5$ : **C07C 401/00,** G01N 33/82, A61K 31/59, A61K 49/02, C07K 15/00, C07B 59/00

(54) **Nouveaux dérivés de la vitamine D : applications thérapeutiques et aux dosages des métabolites de la vitamine D.**

(30) Priorité : **04.05.88 FR 8805985**

(43) Date de publication de la demande : **08.11.89 Bulletin 89/45**

(45) Mention de la délivrance du brevet : **07.01.93 Bulletin 93/01**

(84) Etats contractants désignés : **AT BE CH DE ES FR GB GR IT LI LU NL SE**

(56) Documents cités :
**EP-A- 0 092 004**
**DE-A- 2 440 406**
**FR-A- 2 376 863**
**FR-A- 2 376 864**
**US-A- 2 407 672**
**J. ORG. CHEM., vol. 47, 1982, pages 337-342, Am. Chem. Soc.; K. PARKER et al, "New approahces to the synthesis of vitamin Dmetabolites. 1. Stereocontrol in the intramolecular Diels-Alder reaction"**

(73) Titulaire : **MEDGENIX DIAGNOSTIC**
**B-6220 Fleurus (BE)**

(72) Inventeur : **Bouillon, Roger**
**Schoonzichtlaan, 22**
**B-3009 Herent (BE)**
Inventeur : **De Clercq, Pierre Jean**
**Vijvermeerspark, 22**
**B-9000 Gent (BE)**
Inventeur : **Eliard, Pierre**
**Rue du Broeck, 35**
**B-1070 Bruxelles (BE)**
Inventeur : **Vandewalle, Mauritz**
**Nekkersberglaan 7**
**B-9000 Gent (BE)**

(74) Mandataire : **Warcoin, Jacques et al**
**Cabinet Régimbeau 26, avenue Kléber**
**F-75116 Paris (FR)**

**EP 0 341 158 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la vitamine D, un procédé pour leur préparation, leur application thérapeutique et leur application comme agent de diagnostic, à savoir les traceurs et immunogènes obtenus à l'aide de ces dérivés, les anticorps obtenus à l'aide de ces immunogènes ainsi qu'un procédé et un kit de dosage utilisant ces traceurs et anticorps.

La vitamine D est une hormone dont le précurseur est synthétisé au niveau cutané sous la dépendance d'une réaction photochimique faisant intervenir l'irradiation U.V. Une insuffisance d'irradiation peut être compensée par un apport exogène de vitamine $D_3$ (cholécalciférol) ou $D_2$ (ergocalciférol).

La vitamine $D_3$ et la vitamine $D_2$ répondent aux formules suivantes

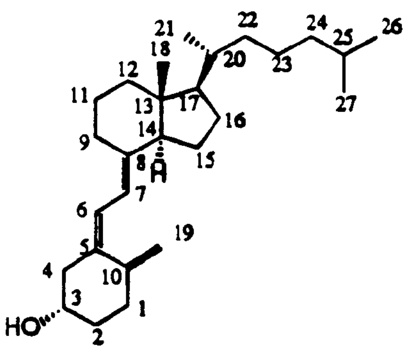

Vitamine $D_3$ ou Cholécalciférol

(9.10-séco-3.7(19)-cholestatriène-3β-ol)

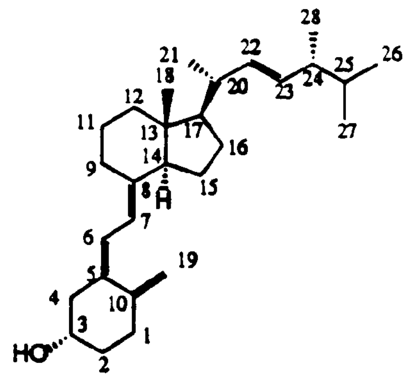

Vitamine $D_2$

La vitamine D subit une première hydroxylation au niveau hépatique en position 25 : la 25-OH-vitamine D est une des formes principales de vitamine D circulante. Ce métabolite présente une haute affinité pour une protéine plasmatique porteuse, la "vitamin D binding protein" (DBP), mais une faible affinité pour le récepteur intracellulaire de la vitamine D. Après hydroxylation en position 1α au niveau rénal, la 25-OH-vitamine D est transformée en 1α-25-$(OH)_2$ vitamine D qui est le principal métabolite actif responsable de l'activité biologique. Ce métabolite a une faible affinité pour la DBP et une forte affinité pour le récepteur intracellulaire. Son taux sanguin est régulé dans des limites très étroites.

Le métabolisme de la vitamine D produit toute une série d'autres métabolites (par exemple 24-25 $(OH)_2$ vitamine D ; 1, 24, 25 $(OH)_3$ vitamine D ; 25, 26 $(OH)_2$ vitamine D, etc.) dont le rôle physiologique, s'il existe, est encore mal défini. Une production extrarénale de 1,25$(OH)_2$ vitamine D est décrite au niveau du placenta, mais aussi au niveau de kératinocytes et de certaines cellules hématopoiétiques (monocytes, macrophages), ce qui expliquerait la production excessive de vitamine D (accompagnée éventuellement d'hypercalcémie, d'hypercalciurie, et de leurs conséquences) que l'on observe dans certains états inflammatoires (maladies granulomateuses, telle la sarcoidose).

Actuellement, les pathologies associées à la vitamine D se classent en déficiences ou en excès de vitamine D. Les déficiences en vitamine D sont dues soit à un ensoleillement insuffisant associé à un déficit d'apport exogène par la nourriture, soit à des anomalies du métabolisme de la vitamine D. Sont décrites des anomalies génétiques au niveau de l'hydroxylase rénale (1α hydroxylase) ou au niveau du récepteur de la vitamine D (résistance à la vitamine D). Le métabolisme peut également être perturbé au cours de différentes pathologies, et en particulier l'insuffisance rénale et l'hypoparathyroïdie, ou par des interactions pharmacologiques, par exemple avec des médicaments antiépileptiques et des corticoïdes.

Les manifestations cliniques de ces déficiences en vitamine D apparaissent le plus clairement au niveau osseux : rachitisme, ostéomalacie, et peut-être participation aux phénomènes d'ostéoporose. Il existe d'autres troubles associés plus discrets, par exemple un déficit immunitaire, une incidence plus élevée de certains cancers, de troubles vasculaires, endocriniens.

Les excès en vitamine D se rencontrent essentiellement lors d'intoxications par la vitamine D ou lors d'une production ectopique de métabolites actifs, par exemple au cours de maladies granulomateuses (sarcoïdose). L'hyperparathyroïdie stimule la production excessive de 1,25$(OH)_2$ vitamine D, qui accompagnerait également l'hypercalciurie idiopathique familiale. Les dérivés actifs de la vitamine D employés en thérapeutique, et en particulier la 1-25$(OH)_2$ vitamine D utilisée notamment dans l'insuffisance rénale, ont un index thérapeutique très

étroit, de sorte que des intoxications vitaminiques sont fréquentes lors de leur administration.

Récemment, on a mis en évidence la présence du récepteur de la vitamine D dans un grand nombre de tissus autres que les tissus cibles classiques (os, intestin, rein), et notamment au niveau de la peau, du sein, du colon, du cerveau, des glandes endocrines, du muscle, des cellules hématopoïétiques (monocytes, lymphocytes, ...). Un grand nombre de cellules cancéreuses de toutes origines possèdent des récepteurs de la vitamine D. La 1-25$(OH)_2$ vitamine D détermine au niveau de ces cellules le passage d'un état de prolifération à un état de différenciation. Elle se comporte ainsi comme une substance immunomodulatrice (par exemple stimulation de l'immunité non spécifique par les monocytes, inhibition de l'immunité spécifique lymphocytaire) et règle la croissance et la différenciation des cellules normales (embryogénèse) et cancéreuses (induction de la différenciation de cellules de mélanome, de cancer du sein, de leucémie myéloïde, de lymphome, d'ostéosarcome, etc.). Ceci pourrait expliquer les observations épidémiologiques rapportant un déficit immunitaire chez les enfants rachitiques, ou l'incidence plus élevée de cancers du colon associée à une déficience en calcium et vitamine D, ou encore la survie prolongée des patientes dont le cancer du sein exprime le récepteur à la vitamine D.

Les déficiences habituelles sont aisément corrigées par l'apport exogène de vitamine D. Une forme active, par exemple la 1-25-$(OH)_2$ vitamine D, doit être administrée si le métabolisme est anormal : c'est notamment le cas dans l'insuffisance rénale. La 1-25-$(OH)_2$ vitamine D a cependant une demie vie courte qui justifie souvent deux prises quotidiennes. De plus son index thérapeutique est étroit, conduisant facilement à des intoxications. La recherche de dérivés ayant un profil pharmacocinétique différent (par exemple par une meilleure affinité pour la DBP) et un index thérapeutique plus favorable reste donc toujours d'actualité. L'administration d'un précurseur inactif à l'avantage de limiter l'activité directe sur l'intestin lors d'une prise orale. Certains précurseurs peuvent être ensuite activés par des voies indépendantes du métabolisme normal de la vitamine D ("Prodrug"). De tels dérivés présentent une biodistribution particulière, susceptible de conférer une sélectivité d'effet biologique in vivo. Certains sont particulièrement adaptés à une autre voie d'administration, telle l'administration transcutanée qui constitue, par exemple, un traitement efficace du psoriasis. Enfin, certains agonistes permettent de dissocier les différentes activités biologiques sur les cellules cibles ; ainsi des dérivés actifs sur la différenciation des cellules leucémiques mais peu actifs sur le métabolisme ostéocalcique ont été décrits. Ces substances ont de grandes potentialités thérapeutiques (immunomodulation, cancer, athéromatose, diabète sucré,...).

Il n'y a pas encore d'antagoniste sélectif de la vitamine D. Un tel produit serait utile dans de nombreux états pathologiques, notamment les intoxications à la vitamine D, des hypercalcémies (néoplasique, hyperparathyroïdie, ...), des hypercalciuries (avec ou sans lithiases rénales), la maladie de Paget, des maladies granulomateuses (Sarcoïdose), etc. Selon leur spectre d'activité, leur biodistribution, etc. des antagonistes pourraient être associés à des agonistes pour obtenir une activité préférentielle in vivo.

Les vitamines sont hydroxylées dans le foie et les reins et produisent une série de métabolites, notamment la 25 hydroxyvitamine D, la (24,25)dihydroxyvitamine D, la ($1\alpha$ 25)dihydroxyvitamine D. Ces dérivés et des dérivés synthétiques comme la $1\alpha$-hydroxyvitamine D, la $1\alpha$, 24-dihydroxyvitamine D, sont également utilisés comme médicament dans le traitement de certaines affections comme l'hypoparathyroïdie, l'insufisance rénale et l'ostéoporose. Les quantités très faibles de ces métabolites présents ou administrés dans l'organisme requièrent des dosages présentant une grande sensibilité pour suivre leurs variations.

Les dosages de ces métabolites nécessitent la préparation de dérivés de ces derniers, en vue de leur marquage, notamment radioactif ou enzymatique pour l'obtention de traceur, d'une part, et de leur couplage à une protéine porteuse immunogénique pour l'obtention d'anticorps antimétabolite, d'autre part, en cas de dosage immunologique.

Mais d'une manière générale, les dosages de ces métabolites nécessitent le recours d'un ligand affin et spécifique du métabolite à doser. Parmi ces ligands, on peut citer :

- les anticorps comme mentionné ci-dessus lorsque le dosage est un dosage immunologique, mais aussi pour des dosages non immunologiques,
- le transporteur plasmatique de la vitamine D (on vitamine D binding protein DBP) ou encore
- le récepteur intracellulaire de la vitamine D.

Il a déjà été proposé dans l'art antérieur différents dérivés de métabolites de la vitamine D destinés à une application pour des dosages immunologiques, notamment comme haptène.

Les haptènes proposés dans les demandes de brevet FR 2 376 863 et FR 2 376 864 ne permettent pas d'obtenir des réactifs, notamment des anticorps, donnant des dosages immunologiques des métabolites de la vitamine $D_3$ suffisamment sensibles et spécifiques.

Ces haptènes ont en effet les positions 3 ou 25 de la vitamine $D_3$ occupées par un groupe hémisuccinate. Les anticorps développés par inoculation chez l'animal des antigènes résultant du couplage de ces haptènes avec une protéine porteuse immunogénique, ne présentent pas une reconnaissance de la fonction hydroxyle

en position 3 et/ou 25 pouvant exister sur les métabolites de la vitamine $D_3$.

Une dérivation en position 3 affecte également la reconnaissance du groupe hydroxyl en position 1 de la vitamine $D_3$ en raison de leur trop grande proximité et de l'encombrement stérique pouvant en résulter.

De même, une dérivation en position 25 affecte la reconnaissance des métabolites du type 24-hydroxy ou (1,24)dihydroxyvitamine $D_3$.

Pour remédier à ces problèmes, il a été proposé dans la demande de brevet européen EP 92 004 une dérivation en position 22 de la vitamine $D_3$ avec un groupe carboxyle ou son ester alkylé par une chaîne de 1 à 6 atomes de carbone substituée par un groupe carboxyle ou amine.

Toutefois, ces haptènes ne sont pas totalement satisfaisants dans la mesure où la position 22 de dérivation est encore à proximité des positions 24 et 25 où se trouvent fixés des groupes hydroxyles spécifiques de reconnaissance de plusieurs métabolites de la vitamine $D_3$.

Ces problèmes d'encombrements stériques se posent de la même façon pour l'obtention de traceurs radioactifs (I-125) ou enzymatiques qui nécessitent des dérivations chimiques identiques à celles décrites ci-avant. En conséquence, les dérivations déjà décrites n'ont pas permis l'obtention de tels traceurs.

La présente invention propose de nouveaux dérivés de la vitamine D présentant une dérivation en position 11, ces dérivés étant utiles comme médicaments mais également pour de la préparation de traceurs radio-isotopiques ou enzymatiques pour des dosages de métabolites de la vitamine D y compris ceux ayant un groupe hydroxyle en position 1,24,25 ou 26, et comme haptènes en vue de la préparation d'immunogènes et donc d'anticorps dans le cas des dosages immunologiques.

La présente invention a donc pour objet des dérivés de la vitamine D répondant à la formule I suivante

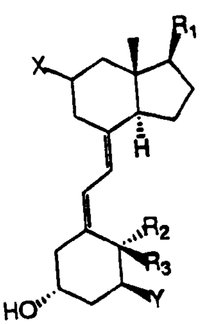

I

dans laquelle

$R_1$ représente un groupe alkylé substitué, de 1 à 15 atomes de carbone, notamment les chaînes latérales des vitamines $D_2$ (C-20 à C-28) ou $D_3$ (C-20 à C-27), ou ces mêmes chaînes partiellement modifiées, notamment

- hydroxylées en une ou plusieurs positions, par exemple les positions 24, 25 et/ou 26, et/ou
- méthylées ou éthylées en une ou plusieurs positions, par exemple les positions 24, 26 et/ou 27, et/ou
- halogénées ou poly-halogénées en une ou plusieurs positions, par exemple perfluorées (trifluorométhyl) en positions 26 et 27, ou difluorées en position 24, et/ou
- additionnées de un ou plusieurs atomes de carbone, notamment un atome C-24' entre les positions 24 et 25, avec les mêmes possibilités de substitution que mentionnées ci-dessus, et/ou
- esterifiées sur une ou plusieurs fonctions hydroxyles mentionnées ci-dessus, et/ou
- éthérifiées sur une ou plusieurs fonctions hydroxyles mentionnées ci-dessus, et/ou
- en remplaçant un ou plusieurs atomes de carbone par un atome d'oxygène, d'azote ou de souffre, par exemple un oxygène en positions 22, 23 ou 24, et/ou
- cyclisées entre les carbones C-26 et C-27 par une liaison directe (cyclopropane) ou par l'intermédiaire d'un chaîne de 1 à 3 atomes de carbone ; chaun de ces atomes pouvant porter toutes les fonctions ou modifications décrites ci-dessus, et/ou
- substituées en une ou plusieurs positions par un cycle saturé, insaturé, aromatique ou hétéroaromatique pouvant porter toutes les fonctions et modifications décrites ci-dessus, et/ou
- insaturées, avec une ou plusieurs double(s) ou triple(s) liaisons carbone-carbone ; ces groupes alkylés insaturés pouvant porter toutes les fonctions et modifications décrites ci-dessus, et
- les formes isomériques des différents groupes situés sur la chaîne.

Y représente H ou OH ou les groupes dérivés de ce dernier tels que ester et éther ;

X représente

- une chaîne alkylée, notamment de 1 à 6 atomes de carbones, éventuellement substituée à différents endroits par un ou des groupe(s) fonctionnel(s) Z qui peuvent être notamment un halogène (tel que le fluor), un groupe hydroxyle, formyle, carboxyle, amine, thiol, cyano, nitro, sulfoxyde, sulfone, phosphono ou encore des groupes dérivés de ces derniers tels que éther, ester, acétal, amide, hydrazine, phosphate, bisphosphate, ou
- une chaîne alkylée non saturée ayant une ou des double(s) ou triple(s) liaisons carbone-carbone, ces chaînes pouvant en plus porter des groupes fonctionnels Z mentionnés ci-dessus, ou
- un noyau aromatique ou hétéroaromatique éventuellement substitué de halogènes, un (ou des) groupe(s) hydroxyle, amine, formyle, carboxyle, thiol, cyano, nitro ou encore des groupes dérivés de ces derniers tels que éther, ester, acétal, amide, ou
- un halogène, un groupe cyano, sulfoxyde, sulfone, hydroxyle, thiol, amine ou encore des dérivés de ces derniers tels que éther, ester, amine et hydrazine ;

$R_2$ représente un groupe méthyl et $R_3$ un H ou, $R_2$ est H et $R_3$ est méthyl ou, $R_2$ et $R_3$ sont H, ou encore $R_2$ et $R_3$ ensemble représentent un groupe méthylène = $CH_2$.

On peut citer pour $R_1$ dans la formule I en particulier les chaînes latérales suivantes qui sont décrites dans la littérature (réf. 2).

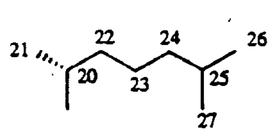
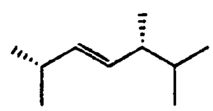
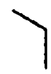

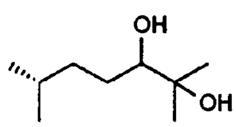

24 R ou S

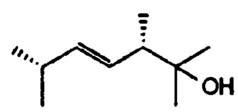
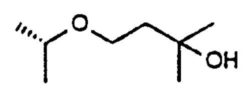

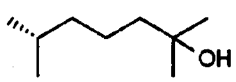
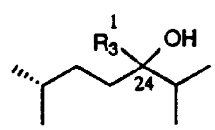

24 R ou S

$R'_3$ = H ou $CH_3$

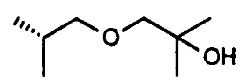

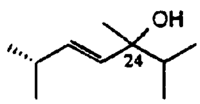

24 R ou S

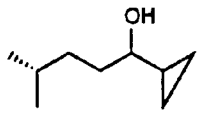

24 R ou S

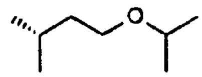

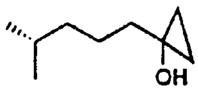

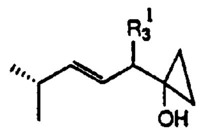

$R_3^1$ = H ou $CH_3$

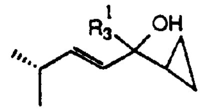

24 R ou S
$R_3^1$ = H ou $CH_3$

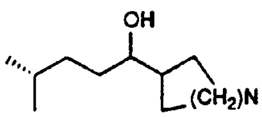

24 R ou S
n = 1,2,3

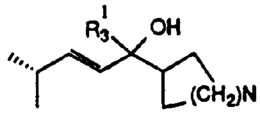

24 R ou S
n = 1,2 3
$R_3$ = H ou $CH_3$

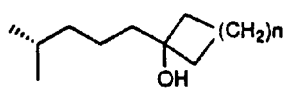

n = 1,2,3

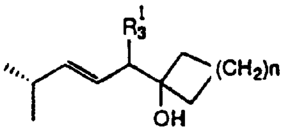

n = 1,2,3
$R_3^1$ = H ou $CH_3$ ( R ou S )

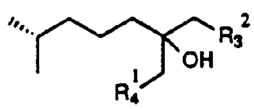

$R_3^2$ = H , $R_4^1$ = $CH_3$ ( R ou S )
$R_3^2$ = $R_4^1$ = $CH_3$

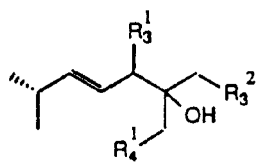

$R_3^2$ = H , $R_4^1$ = $CH_3$ ( R ou S )
$R_3^2$ = $R_4^1$ = $CH_3$
$R_3^1$ = H ou $CH_3$ ( R ou S )

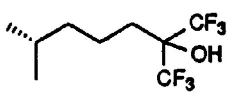

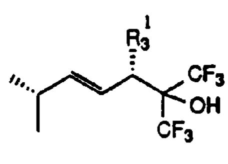

R'$_3$ = H ou $CH_3$ ( R,S )

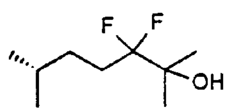

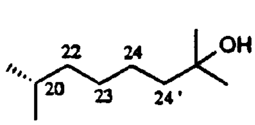

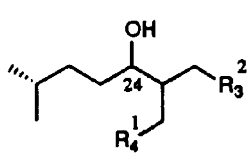

$R_3^2$ = H , $R_4^1$ = $CH_3$ ( R ou S )
24 R ou S

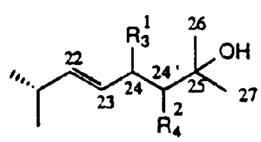

$R_3^1$ = $R_4^2$ = H
$R_3^1$ = $CH_3$ ( R ou S ) , $R_4^2$ = H
$R_3^1$ = H , $R_4^2$ = $CH_3$ (R ou S )

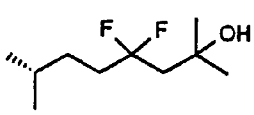

Dans un mode de réalisation particulier, la présente invention a pour objet de nouveaux dérivés de la vi-

tamine $D_3$ répondant à la formule I'

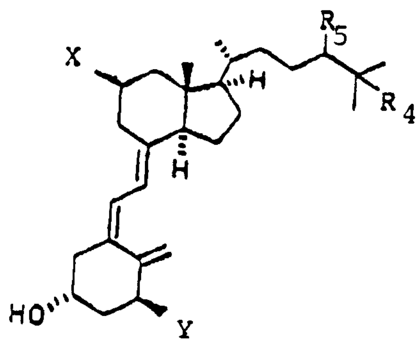

I'

dans laquelle

Y, $R_4$ et $R_5$ représentent H ou OH, notamment

(Y, $R_4$, $R_5$) = (H, OH, H), (OH, OH, H), (H, H, OH), (H, OH, OH), (OH, H, OH), (OH, H, H) et

X représente une chaîne alkylée, de 1 à 6 atomes de carbone éventuellement substituée à son extrémité terminale par un groupe fonctionnel Z qui peut être notamment un groupe hydroxyle, formyle, carboxyle, amine ou encore des groupes dérivés de ces derniers, tels que éther, ester, acétal, ou amide.

D'une manière générale, la chaîne alkylée X peut se trouver fixée en position 11 selon l'isomérie α (molécule Iα) ou β (molécule Iβ).

Dans les formules I et I', on peut citer en particulier le cas où la chaîne alkylée X consiste en un groupe méthyle, éthyle ou phényle.

On peut citer plus particulièrement les 1, 24 ou 25-hydroxy-11-(2-Z)éthyl vitamine $D_3$ ou $D_2$ et les (24, 25), (1, 24) ou (1, 25)dihydroxy-11-(2-Z)éthyl vitamine $D_3$ ou $D_2$.

Comme groupe fonctionnel Z, on peut également citer plus particulièrement un groupe -OH ou,

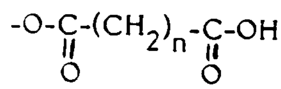

avec n compris entre 0 et 10 et de préférence n = 2.

Des dérivés de formule I ou I' peuvent être utiles pour préparer les réactifs en vue du dosage du métabolite corrrespondant, c'est-à-dire présentant les mêmes substituants $R_1$, $R_2$, $R_3$, $R_4$ et $R_5$ et Y.

La présente invention a également pour objet un procédé de préparation d'un dérivé selon l'invention, caractérisé en ce qu'on fait réagir un dérivé de formule II (IIα ou IIβ)

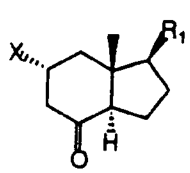

II α

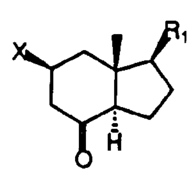

II β

dans lesquelles X et $R_1$ ont les significations données précédemment, ceux-ci étant toutefois protégés si nécessaire sur leur groupe fonctionnel, avec un dérivé de formule III

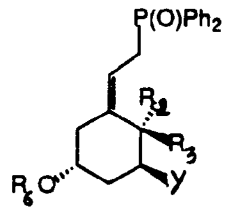

$R_2$ et $R_3$ = $CH_2$
$R_2$ = Me, $R_3$ = H
$R_2$ = H, $R_3$ = Me
Y = H ou OR'

III

dans lesquelles Y, $R_2$ et $R_3$ ont les significations données précédemment, le cas échéant étant un groupe hydroxyle protégé notamment par un groupe protecteur des groupes hydroxyle tel que diméthyl-t-butylsilyle, $R_6$ est un groupe protecteur de la fonction hydroxyle,
cette réaction du composé de formule II avec le composé de formule III se réalise en présence de n-butyl-lithium,
puis on déprotège les fonctions protégées.

En particulier, la présente invention a pour objet un procédé de préparation de dérivés de formule I' ci-dessus, caractérisé en ce qu'on effectue la réaction d'un dérivé de formule II' (II'α ou II'β)

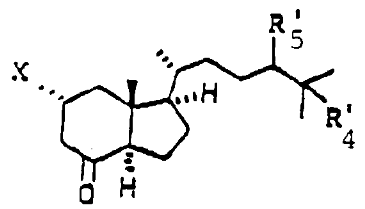

(II'α)

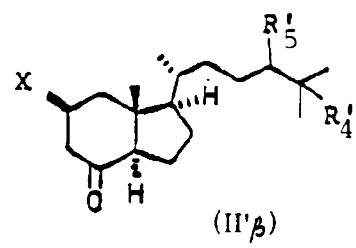

(II'β)

X étant protégée sur sa fonction terminale,
avec un dérivé de formule III'

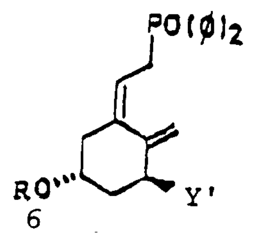

III'

Y', $R'_4$ et $R'_5$ représentent H ou un groupe hydroxyle protégé, notamment par le diméthyl-t-butylsilyle,
$R_6$ est un groupe protecteur de la fonction hydroxyle.

Cette réaction du composé de formule II' avec le composé de formule III' se réalise en présence de n-butyl-lithium.

Le couplage de la fonction cétone des dérivés de formule II (α et β) avec le dérivé phosphoryle de formule III est effectué selon une méthode décrite dans la littéraure (réf. 3).

La synthèse des dérivés de formule III a été décrite dans la littérature (réf. 3, réf. 4).

Dans un mode de réalisation particulier du procédé de préparation d'un dérivé de formule I protégé, on prépare le dérivé avec X substitué par une fonction Z hydroxyle que l'on transforme, le cas échéant, ensuite notamment en un halogène ou une fonction formyle, carboxyle, amine, ester, éther, telle que hémisuccinate selon des méthodes connues de l'homme de l'art.

On prépare tout d'abord le dérivé avec ses fonctions hydroxyles protégées.

On trouvera de nombreux exemples de groupes protecteurs appropriés au groupe hydroxyle dans la littérature, notamment dans le brevet déjà mentionné EP 92 004.

On peut citer plus particulièrement selon la présente invention, le groupe diméthyl-t-butylsilyl pour la fonction hydroxyle, et pour les fonctions hydroxy présentes dans la chaîne latérale et le groupe tétrahydropyranyl

pour les fonctions hydroxy de X.

Il est en effet possible d'introduire des changements au niveau de l'alcool libre avant d'effectuer les déprotections des fonctions alcools protégées. Ceci permet d'obtenir des dérivés de formule I où X est substitué par des fonctions autres que la fonction hydroxyle. En particulier,

. l'aldéhyde et l'acide correspondant peuvent être synthétisés en oxydant la fonction hydroxyle par un réactif à base de chrome VI ;

. l'amide correspondante est obtenue par chauffage de l'acide ou de son sel d'ammonium en présence d'urée ;

. la fonction amine peut être obtenue par réduction de l'amide correspondante avec du $LiAlH_4$ ;

. une fonction ester peut être obtenue par estérification de l'acide avec un alcool approprié ou de l'alcool avec un acide approprié ;

. une fonction acétal peut être obtenue par traitement de l'aldéhyde avec un alcool approprié.

En outre, la présente invention a pour objet les composés de formule

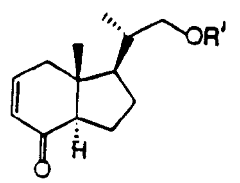

où OR′ représente OH ou les groupes dérivés de ce dernier tels que ester et éther, ainsi que leur utilisation pour la synthèse notamment d'un dérivé de la vitamine D selon l'invention.

Dans les exemples 1 à 17 ci-après est explicitée la **Synthèse des précurseurs $II'_{\alpha}$ des dérivés de la vitamine D substitués en position $11_{\alpha}$ à partir de la 11-cétoprogestérone (schéma 1)**

Le dérivé de formule **II′**α peut être avantageusement préparé selon le procédé de l'invention à partir de la 11-cétoprogestérone **1**. Le composé de formule **1** est soumis à une réaction de pyrolise à environ 330°C pendant 4 heures pour donner, après transformation de la fonction phénolique en méthyléther en présence de $CH_3I$ et $K_2CO_3$, deux dicétones isomériques **2** et **2′**.[5] Le dérivé **2′** peut être transformé en composé de formule **2** recherché qui est plus stable thermodynamiquement par traitement à la soude (NaOH) dans le méthanol. On obtient ainsi un mélange de **2** et **2′** dans un rapport molaire 3:1 à partir de **2′** pur. Après quoi les isomères sont à nouveau séparés par HPLC. Le composé est ensuite transformé en composé de formule 14.

Par exemple, selon le schéma 1, correspondant au cas où X représente un groupe 2-hydroxyléthyl, le composé **2** est traité avec l'anion triéthylphosphonoacétate dans du THF pour donner le composé **3** dans un rendement de 80 %. Puis le composé **3** est traité avec du méthyltriphénylphosphorane dans le THF pour donner le composé **4**. Ce dernier se réduit facilement par réduction métallique dans l'ammoniac liquide en présence de donneur de proton pour donner l'alcool **5** et de l'aldéhyde correspondant, lequel est converti également en alcool avec l'hydrure d'aluminium lithium. Le composé **5** est ensuite protégé de façon classique sous forme d'éther, d'éther silylé ou d'acétal, par exemple par transformation en éther de tétrahydropyranyle (THP) **6**. Le composé **6** est ensuite hydroboraté avec du 9-borabicyclononane (9-BBN) suivi par une oxydation du borane résultant avec du peroxyde d'hydrogène.[6] On obtient un seul isomère **7**, dont le centre de stéréoisomérie à $C_{20}$ formé dans cette réaction, a la configuration naturelle recherchée.[6] L'hydroxyle du composé **7** est ensuite converti en groupement partant, par exemple en tosylate **8** correspondant avec un excès de chlorure de tosyle à 0°C en présence d'une quantité catalytique de diméthylamino pyridine. Pour l'élaboration de la chaîne en $C_{20}$ l'on fait appel à l'acétylure dont la fonction hydroxyle peut être protégée sous forme d'éther, d'éther silylé ou d'acétal. Par exemple, l'acétylure résulte de l'addition de l'alcyne **9a** à une solution de dimsyl sodium.[7] La réaction de **8** avec l'acétylure de sodium dérivé de **9a** en excès dans du DMSO était complète en 1 heure avec un rendement de 90 % pour donner le composé **10a**. L'alcyne **10a** est ensuite réduite en dérivé **11a** par hydrogénation catalytique sur du Pd/C 10 % à pression de 4 atmosphère d'hydrogène en présence de triéthylamine. L'ozonolyse des composés aromatiques en acides **12** correspondants se fait sur gel de silice à -78°C.[8] Dans le cas où la fonction R′ est déprotégée durant l'ozonolyse, par exemple dans le cas de **11c**, il est avantageux de réintroduire un groupement protecteur tel que par exemple un éther triméthylsilyl par traitement avec la N-(triméthylsilyl)imidazole dans le dichlorométhane. Le traitement de l'acide **12a** avec du carbonyl diimidazole dans du THF, sous irradiation dans un tube de quartz pendant 16 heures à 250 nm a donné le composé de méthylène **13a**[9]. Enfin, l'ozonolyse de **13a** de -30°C à -60°C dans du méthanol et soumis à du $Me_2S$ donne la cétone **14a**. Comme décrit antérieurement il est avantageux ici aussi de réintroduire un groupement protecteur R′ au cas où la déprotection est observée durant l'ozonolyse, par exemple du dérivé **13b**.

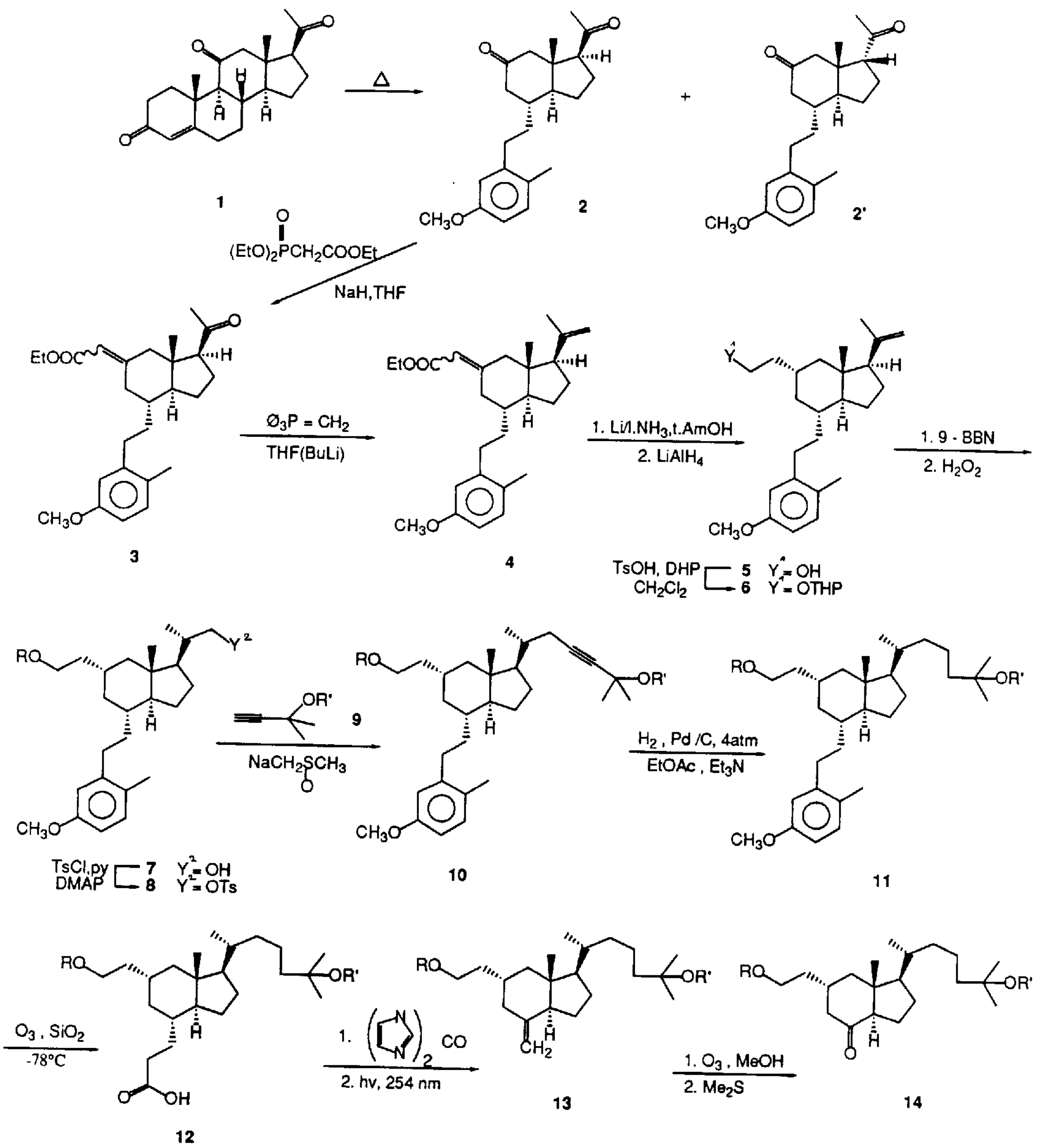


Schéma 1 (R = THP)

**a** R' = $-SiMe_2CMe_3$
**b** R' = $-SiMe_3$
**c** R' = $-CH(CH_3)OCH_2CH_3$

Dans les exemples 18 à 32 ci-après se trouve explicité la **Synthèse des précurseurs II'$_{\alpha}$ et II'$_{\alpha}$ des dérivés de la vitamine D substitués en position 11$_{\alpha}$ et 11$_{\beta}$, respectivement, à partir de la vitamine $D_3$ou de la vitamine $D_2$acétate (schémas 2 et 3)**

Les dérivés de formule II'$_{\alpha}$ et II'$_{\beta}$ peuvent être avantageusement préparés selon le procédé de l'invention à partir du composé **15**. Comme indiqué dans le schéma 2 ce dernier composé peut être préparé à partir d'alcools connus. Notamment, le traitement de l'alcool **16**, obtenu à partir de la vitamine $D_3$ par ozonolyse suivi de réduction par le borohydrure de sodium[10], absorbé sur gel de silice avec de l'ozone à température de -78°C à 20°C et l'extraction ultérieure du gel de silice avec de l'acétate d'éthyle, puis la purification chromatographique donnent la cétone **17** ainsi que la cétone 25-hydroxylée **18**. Le traitement analogue du dérivé **17** conduit également à la cétone **18**. La protection de l'alcool sous forme d'éther, d'éther silylé ou d'acétal se fait selon les méthodes établies, par exemple par transformation en éther de trimethylsilyl **15b** ou de éthoxy éthyl (EE) **15a**. Le dérivé **15** est également obtenu à partir du diol **19**, qui lui résulte de l'ozonolyse suivi de la réduction par le borohydrure de sodium de l'acétate de la vitamine $D_2$.[10] Comme pour l'élaboration de l'alcyne **10**, le dérivé **21** est obtenu par traitement du tosylate **20** avec l'acétylure dérivé de **9** dans le DMSO. Le tosylate **20** est préparé au départ de l'alcool **19** avec le chlorure de tosyle dans la pyridine. La réduction catalytique de l'alcyne **21** s'effectue dans l'acétate d'éthyle en présence de triéthylamine et de palladium sous pression de 2,8 d'atmosphère d'hydrogène. L'alcool **22** ainsi obtenu donne par oxydation le dérivé **15** désiré. Dans le cas du composé **22a** l'oxydation s'effectue avantageusement par ozonolyse du produit absorbé sur gel de silice à -78°C suivi d'extraction par l'acétate d'éthyle.

L'introduction de la chaîne en $C_{11}$ à partir du composé **15** est décrite dans le schéma 3. La cétone est d'abord transformée en une cétone α,β-insaturée **24**. Plusieurs méthodes pour réaliser ce type de transformation sont connues dans la littérature.[11] Suivant le procédé d'élimination par phénylsélénation-oxydation,[12] la cétone **15** est traitée avec du lithium diisopropylamide (LDA) dans du THF à -78°C suivi par une addition de bromure de phénylsélényle. L'oxydation du dérivé **23** ainsi obtenu avec de l'acide métachloroperbenzoique dans du dichlorométhane à -15°C conduit à la cétone insaturée **24** recherchée. Un composé similaire à **24** mais avec une double liaison 25,26 a été décrit.[13] L'addition conjuguée de nucléophiles appropriés, par exemple, des réactifs organo cuivreux,[14] cyanure[15] ou carbanions de type Michael[16] peut ensuite conduire à des intermédiaires 11-substitués. Un exemple est la réaction de l'énone **24a** avec le cuprate lithium bisméthyl dans le diéthyléther à 0°C qui conduit après purification au dérivé **25a** possédant un groupement méthyl en 11$_{\alpha}$. Un autre exemple est la réaction de l'énone **24a** avec le cuprate obtenu à partir de phényllithium et CuCN dans le THF[17] à une température de -70°C à -40°C qui conduit au dérivé **26a**. Un autre exemple encore est la réaction de l'énone **24b** avec le cuprate lithium obtenu à partir de vinyllithium et CuCN dans le diéthyléther à -78°C jusqu'à -40°C, qui mène au composé **27b**. Parmi d'autres dérivés possibles, le dernier dérivé permet l'introduction de diverses fonctions en $C_{11}$. Par exemple, la réaction de **27b** avec le 9-borabicyclononane (9-BBN) dans le THF, suivi de l'oxydation du borane obtenu par le peroxyde d'hydrogène donne l'alcool **28b**. Ce dernier est protégé, par exemple sous forme d'éther tert-butyldiméthylsilyle **29b** par traitement avec le chlorure de tert-butyldiméthylsilyl en présence d'imidazole dans le DMF. L'oxydation de l'alcool **29b** par le pyridiniumdichromate en présence de pyridinium paratoluènesulphonate dans le dichlorométhane donne le composé **30b**, analogue au dérivés **14** décrits précédemment.

La synthèse des cétones II$_{\beta}$ substitués en 11$_{\beta}$ s'effectue à partir des dérivés correspondants substitués en 11$_{\alpha}$. La méthode utilisée consiste à introduire une double liaison 9,11 qui est ensuite réduite par voie d'hydrogénation catalytique menant aux isomères 11$_{\beta}$ . La conversion en cétone α,β-insaturée s'effectue comme précédemment par phénylsélénation-oxydation. Par exemple, la cétone **25a** est traitée avec du LDA dans le THF à -78°C suivi par une addition de bromure de phénylsélényle. Le dérivé attendu **31a** peut également être obtenu directement à partir de l'énone **24a**, en traitant l'énolate intermédiaire formé in situ par addition de cuprate lithium bisméthyl avec le phénylsélénylbromure dans le diéthyléther à -78°C. La séquence phénylsélénation-oxydation appliquée par exemple à la cétone **26a** conduit au sélénure **32a**, et appliquée à la cétone **14c** au sélénure **33a**. L'oxydation des sélénures tels que **31**, **32** et **33** par l'acide métachloroperbenzoique conduit directement aux énones correspondantes telles que **34**, **35** et **36**, respectivement. L'hydrogénation catalytique de ces énones est effectuée dans l'acétate d'éthyle sous 4 atmosphère d'hydrogène. Les cétones résultantes **37**, **38** et **39**, respectivement, possèdent la stéréoisomérie β en $C_{11}$.

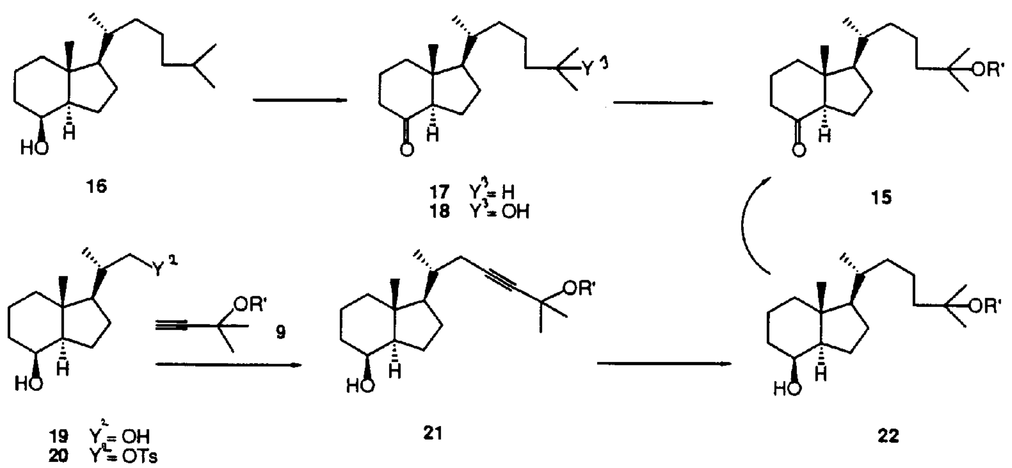

16

17 $Y^1$ = H
18 $Y^1$ = OH

15

9

19 $Y^2$ = OH
20 $Y^2$ = OTs

21

22

**a** : R' = $-CH(CH_3)OCH_2CH_3$     **b** : R' = $-SiMe_3$

Schéma 2

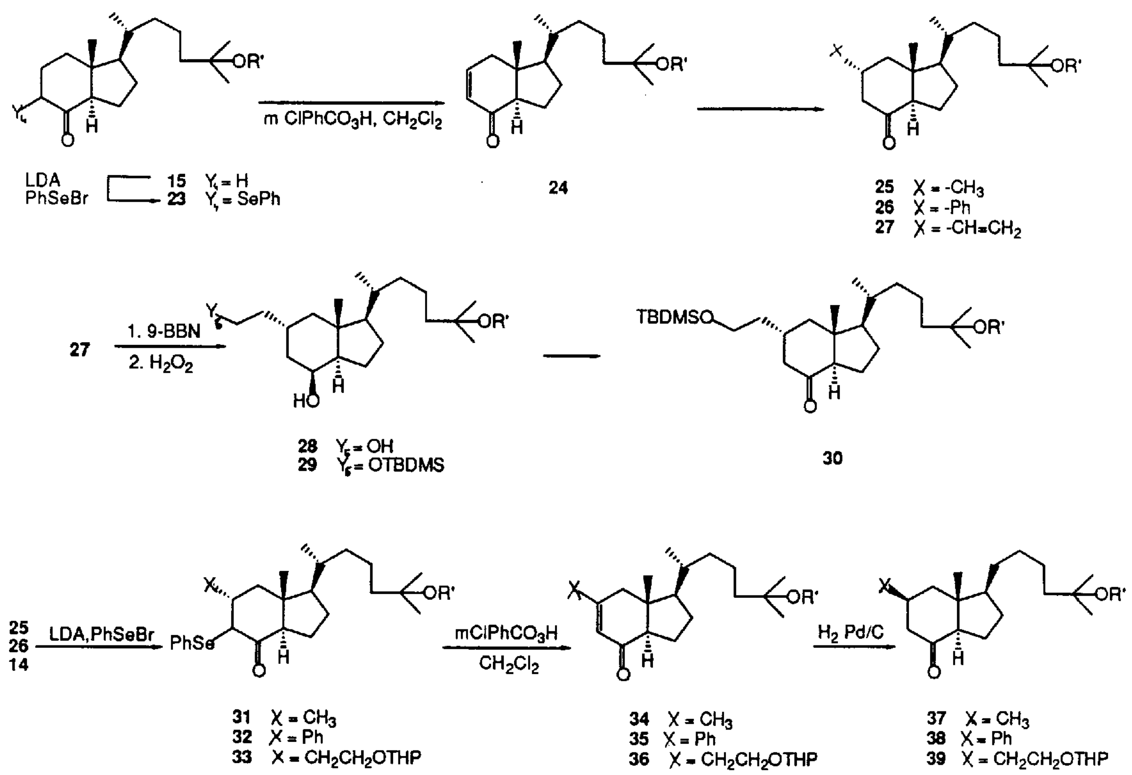


15 $Y_1$ = H
23 $Y_1$ = SePh

24

25 X = $-CH_3$
26 X = -Ph
27 X = $-CH=CH_2$

27

28 $Y_5$ = OH
29 $Y_5$ = OTBDMS

30

25
26
14

31 X = $CH_3$
32 X = Ph
33 X = $CH_2CH_2OTHP$

34 X = $CH_3$
35 X = Ph
36 X = $CH_2CH_2OTHP$

37 X = $CH_3$
38 X = Ph
39 X = $CH_2CH_2OTHP$

Schéma 3
a R' = $-CH(CH_3)OCH_2CH_3$
b R' = -SiMe3

Dans les exemples 33 à 65 ci-après se trouve détaillée la **Synthèse des dérivés I de la vitamine D substitués en $11_\alpha$et $11_\beta$ par couplage des dérivés $II'_\alpha$ et $II'_\beta$ avec les dérivés III' (schéma 4)**

Le couplage des cétones substituées en $11_\alpha$ telles que **14**, **25**, **26** et **30** avec l'anion de **40** conduit, après purification par chromatographie sur gel de silice, au dérivés protégés tels que **41**, **42**, **43** et **44**, respectivement. Un exemple est la condensation du sel de lithium, obtenir à partir du phosphonate **40** avec le n-butyllithium dans le THF à -78°C, avec la cétone **14a** qui conduit après 1 heure et demie à -78°C à la triène **43a**. Autres exemples sont les couplages analogues avec les cétones **25c** et **26c** conduisant aux triènes **41c** et **42c**, respectivement. Un autre exemple encore est la condensation analogue avec la cétone **14b** conduisant au triène **43b**, ou la cétone **30b** conduisant au triène **44b**. Le dérivé **43a** offre la possibilité d'une déprotection sélective au niveau de l'éther THP, par traitement au bromure de magnésium anhydre dans l'éther, ce qui donne l'alcool **45a**. La réaction de ce dernier dérivé avec l'anhydride succinique dans la pyridine et en présence de la 4-diméthylaminopyridine mène à l'hémi-succinate **46a** correspondant.

L'élimination des groupements protecteurs des dérivés de couplage conduit à l'obtention des triols **47** et **48** au départ de **41** et **42**, respectivement, du tétrol **49**, à partir de **43**, **44** ou **45**, ou de l'acide **50** au départ de **46**. L'hémi-succinate **50** est également avantageusement obtenu directement par traitement du tétrol **49** avec l'anhydride succinique dans la pyridine en présence de 4-diméthylaminopyridine.

Le couplage des cétones $II'_\alpha$ substituées en $11_\alpha$ avec l'anion de **51** conduit de la même façon aux triènes désirés. Un exemple est la condensation des dérivés **14a**, **14b** et **30b** menant aux triènes **52a**, **52b** et **53b**, respectivement. Ici également l'hydrolyse du groupement protecteur dans la chaîne en $C_{11}$ peut s'effectuer sélectivement, et l'on obtient par exemple au départ de **52a** l'alcool **54a** par traitement au bromure de mangésium anhydride. Comme auparavant l'hémi-succinate **55** est obtenu au départ de l'alcool **54**. Enfin, l'élimination des

groupements protecteurs conduit au triol **56**, ou à l'hémi-succinate **57** au départ du dérivé **55**. Il est également avantageux de préparer l'hémi-succinate **57** directement au départ du triol **56**.
Le couplage des dérivés $II_\beta$ avec l'anion de **40** ou de **51** s'effectue de façon identique. Des exemples pour le cas de **40** sont inclus dans le schéma 4c.
Dans les exemples 66 à 71 ci-après se trouvent détaillées les méthodes alternatives.

Une voie alternative (schéma 5) pour la synthèse des intermédiaires types II peut consister à permuter la séquence réactionelle dans le sens que le substituant X en position 11 est introduit avant d'élaborer la chaîne en position 17. Par analogie avec la transformation de **24** en **25**, **26** ou **27** (par exemple) (schéma 3, exemples 26, 27, 28) on peut effectuer cette addition-1,4 sur la molécule **66** (R′ est un groupe protecteur de la fonction hydroxyl). Le composé **66** peut être obtenu à partir de **20** ($Y^6$ groupement protecteur de la fonction hydroxyl) comme décrite pour **24** (schéma 3, exemples 25 et 25bis).
Comme décrite pour la transformation de (par exemple) **25** en **37** (exemples 30 et 31), le composé **68** peut être formé en partant de **67**. Ensuite, après réduction de la cétone (dans respectivement **67** et **68**), la chaîne latérale est construite à partir de la fonction $Y^6$ (comme par exemple **20** à **15**; schémas 2 et 3, exemples 21, 22, 23, 24) ce qui conduit aux intermédiaires $II_\alpha$ et $II_\beta$.

Un procédé alternatif de couplage des intermédiaires II avec le noyau A est possible par la méthode décrite par L. Castedo et A. Mourino[18] (voir schéma 6). Ce couplage entre **69** et **70**, qui conduit à **71** est effectué avec du $Pd(PPh_3)_2Cl_2$ (3 %) et $NEt_3$ dans du dimethylformamide à 70°C. La formation des enol triflates du type **69** consiste en l'addition 1,4 sur (par exemple) **24** (voir exemples 26, 27, 28) suivi par le piégeage de l'anion de l'énolate intermédiaire avec du N-phényltrifluorométhane sulphonimide. Une méthode alternative pour la formation des enol triflates des intermédiaires II consiste à traiter la cétone correspondante (par exemple **14**, **25**, **26**, **30**, **37**, **38**, **39**) par du lithium diisopropylamide entre -80°C et température ambiante, suivi par addition du N-phényltrifluorométhane sulphonimide. Les composés **70** (R est un groupe protecteur et $\gamma$ est H ou un groupe hydroxyl protégé), précurseurs du noyau A de la vitamine D, ont été décrits dans la littérature.[13,19] La réduction[20] sélective de la triple liaison dans **71** est connue pour conduite au système triène de la prévitamine D en équilibre avec le système triène de la vitamine D.

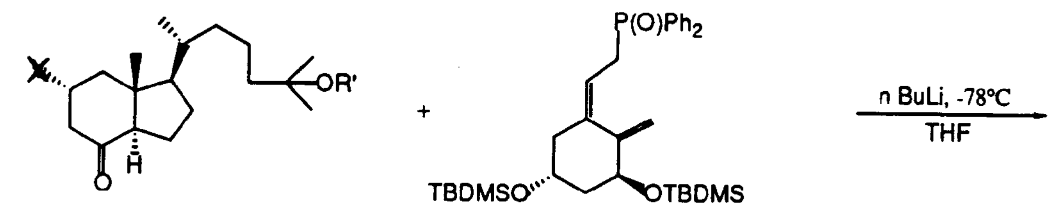


**25** X = Me
**26** X = Ph
**14** X = $CH_2CH_2OTHP$
**30** X = $CH_2CH_2OTBDMS$

**40**

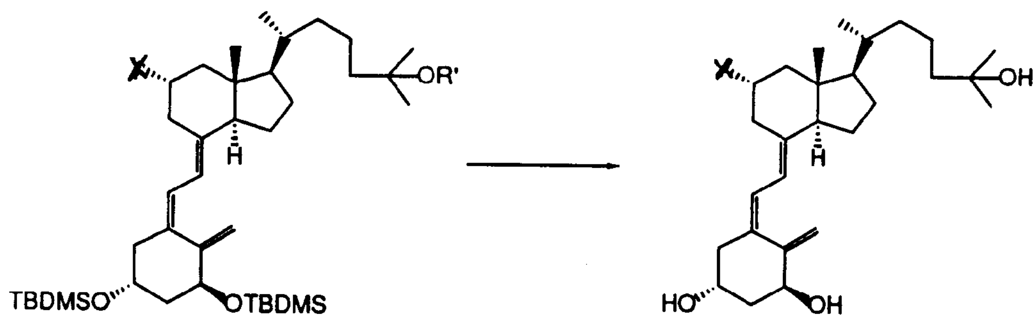

**41** X = Me
**42** X = Ph
**43** X = $CH_2CH_2OTHP$
**44** X = $CH_2CH_2OTBDMS$
**45** X = $CH_2CH_2OH$
**46** X = $CH_2CH_2OCOCH_2CH_2COOH$

**47** X = Me
**48** X = Ph
**49** X = $CH_2CH_2OH$
**50** X = $CH_2CH_2OCOCH_2CH_2COOH$

Schéma 4a

**a** R' = $SiC(Me)_3Me_2$ (TBDMS)
**b** R' = $SiMe_3$
**c** R' = $CH(CH_3)OCH_2CH_3$

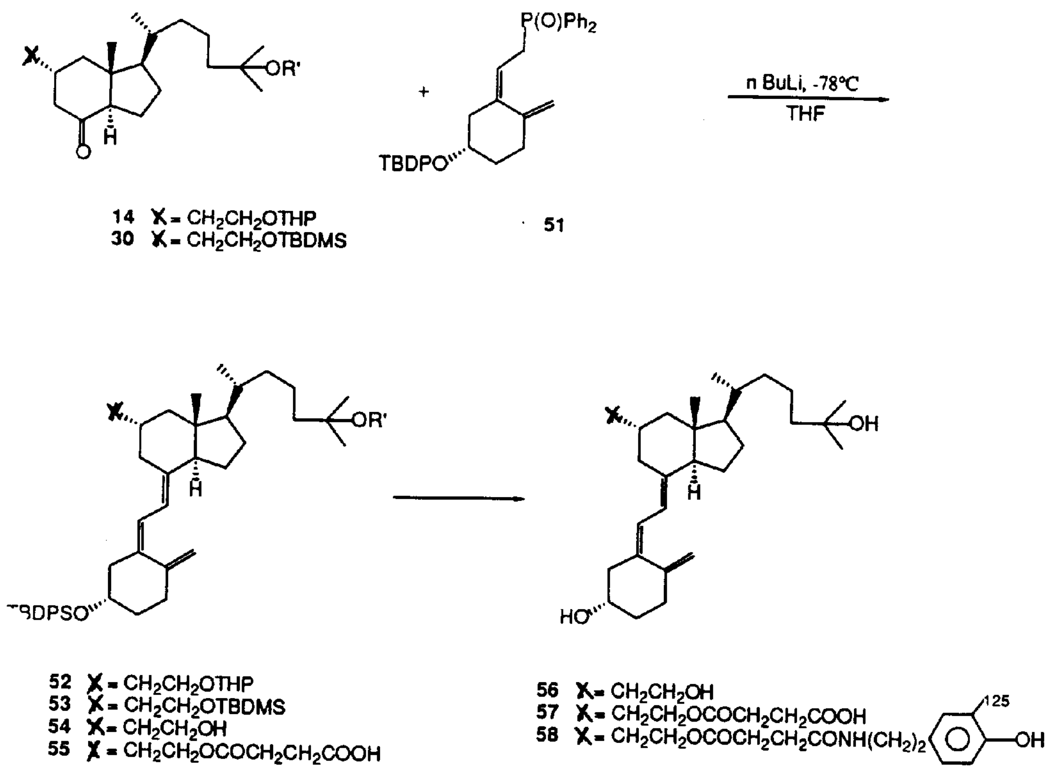


14 X = $CH_2CH_2OTHP$
30 X = $CH_2CH_2OTBDMS$

51

52 X = $CH_2CH_2OTHP$
53 X = $CH_2CH_2OTBDMS$
54 X = $CH_2CH_2OH$
55 X = $CH_2CH_2OCOCH_2CH_2COOH$

56 X = $CH_2CH_2OH$
57 X = $CH_2CH_2OCOCH_2CH_2COOH$
58 X = $CH_2CH_2OCOCH_2CH_2CONH(CH_2)_2$

Schéma 4b

a R' = $SiC(Me)_3Me_2$ (TBDMS)
b R' = $SiMe_3$
c R' = $CH(CH_3)OCH_2CH_3$

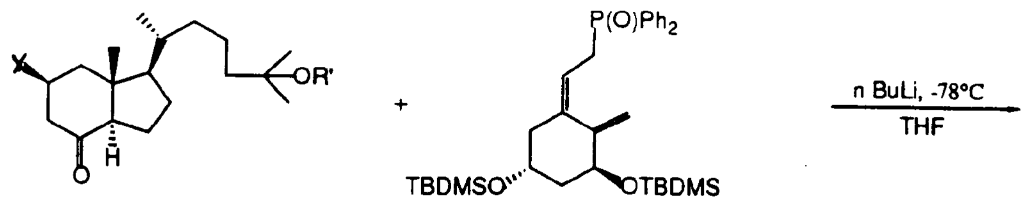


37 X = Me
38 X = Ph
39 X = $CH_2CH_2OTHP$

40

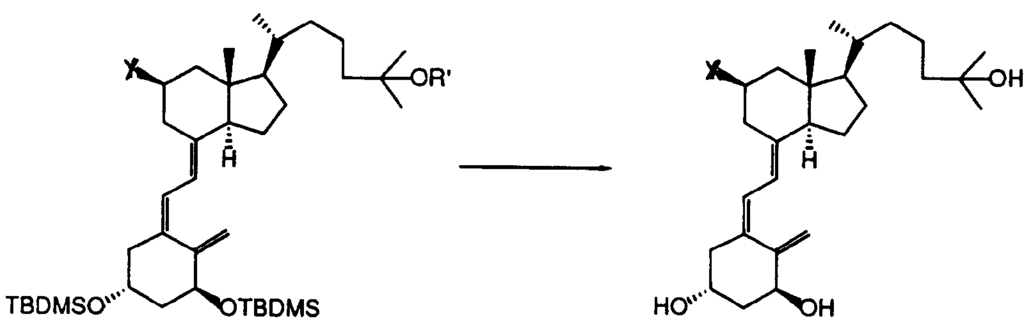

59 X = Me
60 X = Ph
61 X = $CH_2CH_2OTHP$
62 X = $CH_2CH_2OH$

63 X = Me
64 X = Ph
65 X = $CH_2CH_2OH$

Schéma 4c

a R' = $SiC(Me)_3Me_2$ (TBDMS)
b R' = $SiMe_3$
c R' = $CH(CH_3)OCH_2CH_3$

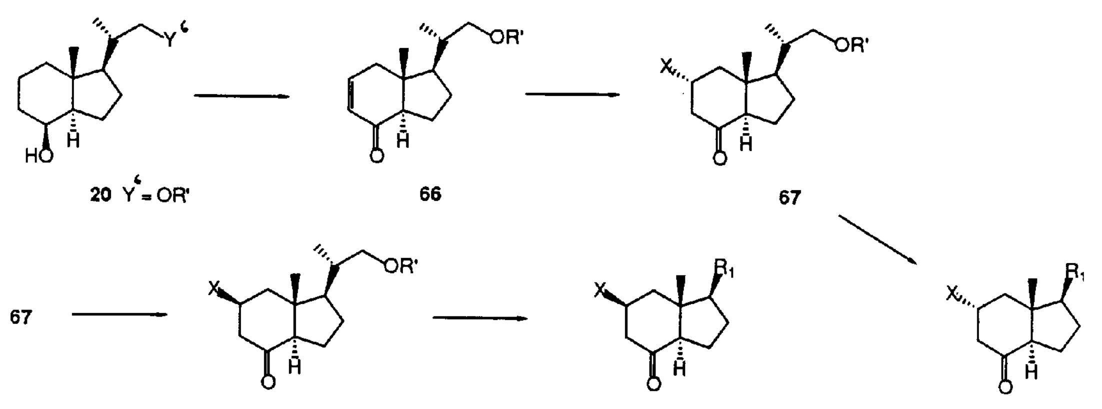

20 Y = OR'    66    67

67    68    II    II

Schéma 5
a $R' = SiMe_2CMe_3$
b $R' = CH(CH_3)OCH_2CH_3$

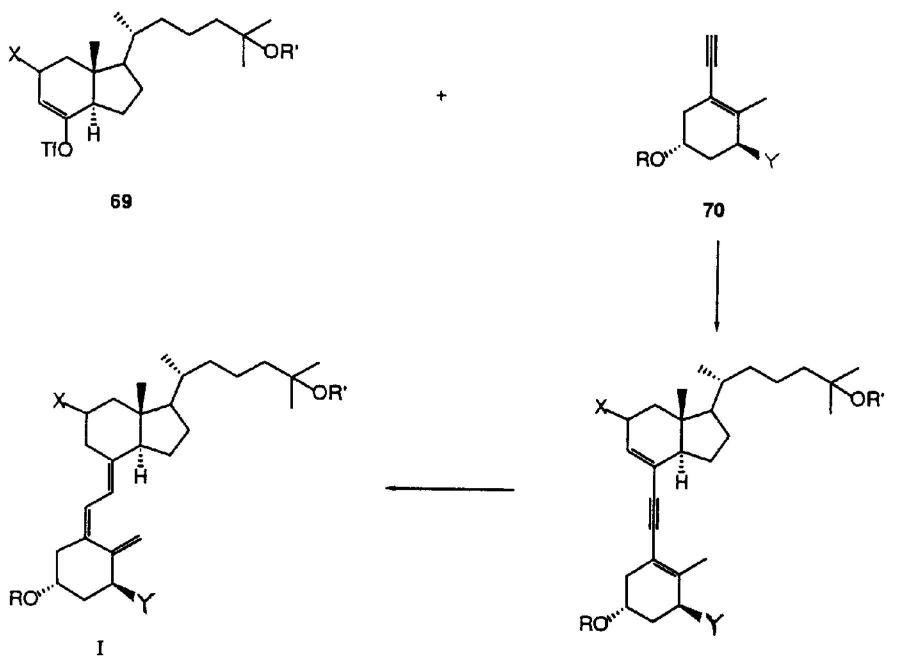

69    70

I    71

R = TBDMS ; Y = H ou TBDMSO

Schéma 6
a $R' = SiC(Me)_3Me_2$ (TBDMS)
b $R' = SiMe_3$
c $R' = CH(CH_3)OCH_2CH_3$

L'obtention des dérivés de formule (I) avec X = 11-(2-hémisuccinoyloxy)éthyl se fait à partir des dérivés avec X = 11-(2-hydroxy)éthyl, de manière classique à l'aide d'anhydre succinique (par exemple 45-46, 49-50,

54-55). Et, plus généralement, à l'aide d'un anhydre.

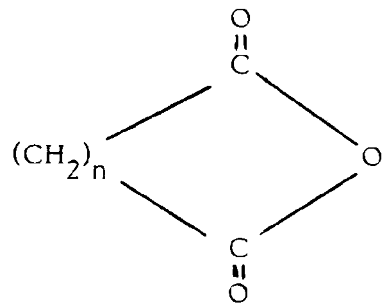

on obtiendra un dérivé où Z est

$$= -O-\underset{\underset{O}{\parallel}}{C}-(CH_2)_n-COOH$$

Il est également possible, à partir de 45, 54 ou 62 d'introduire des changements au niveau de l'alcool libre avant d'effectuer les déprotections des fonctions alcools protégées comme mentionné précédemment. Cette alternative permet d'obtenir des dérivés de formule I où X est substitué par des fonctions autres que la fonction hydroxyle. Par exemple :

. la fonction hydroxylé peut être transformée en groupe nucléofuge (par exemple un tosylate). Ceci permet la substitution nucléophilique introduisant des fonctions tels que halogènes, thiols, éthers, thioliques, amino, cyano, ester, éther ;

. l'aldéhyde ou l'acide correspondant peuvent être synthétisés en oxydant la fonction hydroxyle par un réactif à base de chrome VI ;

. l'amide correspondante est obtenue par chauffage de l'acide ou de son sel d'ammonium en présence d'urée ;

. la fonction amine peut être obtenue par réduction de l'amide correspondante avec du $LiAlH_4$ ;

. une fonction ester peut être obtenue par estérification de l'acide avec un alcool approprié ou de l'alcool avec un acide approprié ;

. une fonction acétal peut être obtenue par traitement de l'aldéhyde avec un alcool approprié.

Ces réactions bien connues de l'homme de l'art sont indiquées à titre purement illustratif et donc non limitatif.

Les dérivés de vitamine D de formule I, notamment de la vitamine $D_3$ notamment ayant une fonction hémisuccinate en terminaison d'une dérivation alkylée en position 11 sont, comme mentionné précédemment, utiles comme haptène ou pour obtenir un traceur en vue de dosages enzymo-immunologiques ou radio-immunologiques de métabolites de la vitamine D, notamment de la vitamine $D_3$ présentant des groupes hydroxyle en positions 1$\alpha$, 24 ou 25.

Lorsque les dérivés de vitamine D de formule I sont utilisés comme haptène pour des dosages immunologiques, ledit haptène est lié de façon covalente à un porteur immunogénique pour former un antigène destiné à la préparation d'anticorps antihaptène. L'anticorps obtenu à partir de cet antigène fournit, le cas échéant, un des réactifs qui constituent les kits de dosages selon l'invention. Les antigènes sont inoculés chez un animal hôte pour induire la formation d'anticorps. Comme animal hôte, on peut mentionner les animaux à sang chaud tels les lapins, les rats, les souris, les bovins, les moutons, etc. L'inoculation d'un antigène chez un animal hôte est pratiquée par administration extra buccale par exemple au moyen d'injection hypodermique ou intra-dermale. L'inoculation de l'antigène est conduite avec un adjuvent complet de Freund. L'inoculation est conduite par injection de par exemple 200 $\mu$g du même antigène de manière hypodermique ou intradermique à intervalle de quatre semaines. Pendant la période d'inoculation, la concentration d'anticorps du sang est déterminée tous les 10 jours et le sang total est retiré quand le titre le plus grand est obtenu. Le sérum séparé du sang ainsi obtenu peut être utilisé comme anticorps sans aucun traitement, ou les immunoglobulines qu'il contient peuvent être préalablement purifiées.

En tant que porteur immunogénique, on peut mentionner des protéines, polypeptides ou glycoprotéines notamment.

Comme exemple de porteurs immunogéniques, on peut citer notamment la sérum albumine bovine, la sérum albumine humaine, la sérum albumine bovine méthylée, la sérum albumine de lapin, les gammaglobulines humaines, les gammaglobulines bovines, la polylysine, le copolymère poly-L-Lysine-polyglutamate, les hémocyanines, les thyroglobuline bovine, la tuberculine et ses dérivés, etc...

La présente invention permet d'obtenir un antigène dans lequel le dérivé de formule I est lié au porteur immunogénique par exemple par une fonction amine ou carboxyle située en terminaison sur la chaîne alkylée (X).

Le couplage du dérivé de formule I au porteur immunogénique est obtenu par la formation d'un lien covalent formé directement ou indirectement entre une fonction Z (par exemple amine ou carboxyle) de la chaîne alkylée (X) du dérivé de formule I et un groupe fonctionnel du porteur immunogénique. Comme groupe fonctionnel du porteur immunogénique, on peut mentionner, par exemple, la fonction amine de la lysine, la fonction carboxyle de l'acide aspartique ou glutamique, la fonction hydroxyphénol de la tyrosine, la fonction thiol de la cystéine.

Comme méthodes de couplage par liaison covalente, on peut mentionner celles où la fonction carboxyle ou amine de X du dérivé de formule I est activée par la N-hydroxysuccinimide ou un alkylchloroformate, ceci préalablement à la formation d'un lien covalent avec le porteur immunogénique.

Lorsqu'un dérivé de formule I est utilisé comme traceur radioactif dans un radioassay, ledit dérivé est radioiodiné directement avec de l'iode 125 ou encore couplé à une molécule préalablement radioiodinée avec l'iode 125. Parmi ces molécules radioiodinées, on peut citer des molécules possédant une fonction amine comme la 125-I tyramine ou la 125-I histamine. Le couplage d'un dérivé de formule I à une molécule radioiodinée est obtenue par la formation d'un lien covalent formé entre une fonction Z (par exemple carboxyle) de X du dérivé de formule I et la fonction amine de la molécule radioiodinée. Comme méthode de couplage covalent d'une molécule radioiodinée au dérivé de formule I, on peut citer celle où la fonction carboxyle du dérivé de formule I est préalablement activée en présence de N-hydroxysuccinimide et de dicyclohexylcarboiimide. Le dérivé de formule I activé est ensuite couplé à une molécule radioiodinée portant une fonction amine. Comme méthode de préparation des molécules radioiodinées, on peut citer entre autres, la radioiodination avec le monochlorure d'iode, l'oxydation par les dérivés chorés comme par exemple la chloramine T ou l'iodo-gen en présence d'iodure radioactif, la radioiodination enzymatique par les peroxydases et la conjugaison a des dérivés radioactifs de structure aromatique, comme par exemple le réactif de Bolton et Hunter.

Lorsqu'un dérivé de formule I est utilisé comme traceur enzymatique dans un immunoassay, ledit dérivé est couplé de façon covalente à une enzyme. Comme enzyme, on peut citer par exemple les peroxydases, la β-galactosidase, la phosphatase alcaline, la glucose oxydase, la liphase, les luciférases, etc. De préférence, on utilise la peroxydase de raifort (horse radish peroxydase, HRP). Comme méthode particulière de couplage d'un dérivé de formule I à une enzyme, on peut citer celle décrite ci-avant pour la préparation des immunogènes. Le dérivé de formule I (où Z = carboxyle) est activé par la N-hydroxysuccinimide, ceci préalablement à la formation d'un lien covalent avec l'enzyme.

Les traceurs radioactifs (ou enzymatiques) obtenus au départ des dérivés de formule I peuvent être utilisés comme traceurs dans les méthodes de dosages par radioassays (ou enzymoassays) des métabolites de la vitamine D notamment de la vitamine $D_3$ possédant un groupe hydroxyle en position 1α, 24 et/ou 25. Comme radioassays (ou enzymoassays), on peut citer les méthodes dites de compétition, dans lesquelles le métabolite que l'on veut doser d'une part, et le traceur d'autre part, entrent en compétition pour un nombre limité de sites de liaison à haute affinité (ou ligand). Parmi les méthodes applicables au dosage des métabolites de la vitamine D , on peut mentionner les immunoassays (radio-immunoassays, RIA ou enzymo-immunoassays, EIA) utilisant les anticorps comme ligands; les réceptorassays, (radio-réceptorassays, RRA ou enzymo-réceptorassays, ERA) utilisant comme ligand le récepteur intracellulaire de la vitamine D ; ou encore les transporteur-assays (radio-transporteur-assays, RTA ou enzymo-transporteur-assays, ETA) avec comme ligand le transporteur plasmatique de la vitamine D .

La présente invention a donc d'une manière générale également pour objet des méthodes de dosages de métabolites de la vitamine D , utilisant des traceurs résultant de la conjugaison d'un dérivé de formule I selon l'invention avec un élément de marquage, notamment une enzyme ou une molécule iodée, ou des anticorps préparés à partir d'antigène consistant dans la liaison covalente d'un dérivé de vitamine D selon l'invention avec une protéine porteuse immunogénique.

La présente invention a également pour objet l'application thérapeutique des dérivés en position $C_{11}$ de la vitamine D selon l'invention à titre de médicament ou sous forme de composition pharmaceutique en comprenant.

L'intérêt général de la dérivation en $C_{11}$ quant à l'activité biologique des dérivés en $C_{11}$ de la vitamine D est la suivante.

Les analogues existant de la vitamine D sont essentiellement des dérivés au niveau de la chaîne latérale ($C_{21}$-$C_{27}$), ou dans une moindre mesure, du cycle A ($C_1$-$C_{10}$).

Différents aspects des relations entre structure et activité sont donc maintenant connus au niveau de la chaîne latérale. Quelques informations sont également disponibles au niveau du cycle A, où la présence du groupe hydroxyl en 1α, et dans une moindre mesure en 3β, est déterminante pour l'activité biologique de la vitamine D.

Il y a par contre très peu d'informations concernant le reste de la molécule, et notamment aucune sur le noyau C. En particulier, aucun dérivé en $C_{11}$ n'a encore été décrit. Cette position est particulièrement intéressante de par sa situation "médiane" à distance des deux régions pour lesquelles une relations structure-activité a été décrite (soit la chaîne latérale et la position 1α). Les dérivés en position 11 offrent donc la possibilité d'introduire des modifications choisies de la vitamine D sans interférer directement avec les fonctions importantes déjà connues. De tels dérivés apportent des propriétés pharmacologiques nouvelles à la molécule, tout en autorisant la combinaison avec des modifications utiles des régions déjà explorées. Les résultats présentés dans la présente demande démontrent que les dérivés $C_{11}$ peuvent modifier les propriétés pharmacologiques et biologiques de la molécule, mais également qu'il est possible d'obtenir des dérivés en $C_{11}$ qui préservent les fonctions essentielles de la vitamine D.

La position $C_{11}$ est particulièrement intéressante pour moduler l'activité biologique et pharmacologique de dérivés de la vitamine D. Parmi les dérivés décrits dans cette demande, certains sont capables de lier la DBP ou le récepteur intracellulaire de la vitamine D avec une haute affinité, d'autres pas. Certains de ces dérivés en $C_{11}$ sont capables de reproduire ou d'antagoniser, en tout ou en partie, les propriétés biologiques biologiques de la 1,25$(OH)_2$ vitamine D. La possibilité d'introduire en position 11α ou 11β des substitutions qui augmentent ou antagonisent l'activité de la 1α, 25 $(OH)_2$ vitamine D, soit globalement, soit au niveau de cibles spécifiques (cellules imunitaires, cancéreuses, cutanées, endocrines, cardiovasculaires, osseuses, etc.) ouvre de nouvelles perspectives d'applications. Il est évident que de tels dérivés ont des propriétés pharmacologiques nouvelles, différentes de la 1,25 $(OH)_2$ vitamine D (pharmacocinétique, biodistribution, métabolisme, activité biologique) et qu'ils trouvent des applications thérapeutiques dans de nombreux domaines médicaux.

D'autres caractéristiques et avantages de la présente invention apparaîtront à la lumière des exemples et des figures qui vont suivre.

**Légendes des figures**

Figure 1

Liaison à la DBP humaine purifiée de la 25 OH vitamine $D_3$, de la 1,25 $(OH)_2$ vitamine $D_3$ et de certains dérivés en $C_{11}$ de ces molécules, mesurée par déplacement de la liaison du traceur [$^3$H]-25OH vitamine $D_3$.

○——○ : 1,25 $(OH)_2$ vitamine $D_3$
●------● : 11 (2-OH) éthyl-1,25 $(OH)_2$ vitamine $D_3$
□——□ : 25OH vitamine $D_3$
■----■ : 11 (2-OH) éthyl-25OH vitamine $D_3$
△——△ : 11(2-HS) éthyl-25OH vitamine $D_3$

Figure 2

Liaison de la 1,25 $(OH)_2$ vitamine D et de deux dérivés en $C_{11}$ au récepteur intracellulaire de la vitamine D, mesurée par déplacement de la liaison du traceur [$^3$H]-1,25$(OH)_2$ vitamine $D_3$.

○——○ : 1,25$(OH)_2$ vitamine $D_3$
●----● : 11α-(2-HS) éthyl-1,25 $(OH)_2$ vitamine $D_3$
△——△ : 11α-(2-OH) éthyl-1,25 $(OH)_2$ vitamine $D_3$

Figure 3

Activité biologique, dans un test de différenciation des cellules HL 60 in vitro de la 1, 25 $(OH)_2$ vitamine

$D_3$ (active), de la 25 $(OH)_2$ vitamine $D_3$ et de dérivés en $C_{11}$ de ces molécules (peu actives).

●——● : 1,25 $(OH)_2$ vitamine $D_3$
o------o : 11 (2-OH) éthyl (1,25)-$(OH)_2$ vitamine $D_3$
▲——▲ : 25 OH vitamine $D_3$
Δ------Δ : 11 (2-OH) éthyl 25-OH vitamine $D_3$

Figure 4

Activité biologique, dans un test de différenciation des cellules HL 60 in vitro de la 1,25 $(OH)_2$ vitamine $D_3$ et de deux dérivés en $C_{11}$, le dérivé méthyl (très actif) et le dérivé phényl (modérément actif).

●——● : 1,25 $(OH)_2$ vitamine $D_3$
o------o : 1α,25-$(OH)_2$-11α-méthyl vitamine $D_3$
Δ-------Δ : 1α,25-$(OH)_2$-11α-phényl vitamine $D_3$

Exemple 1

Synthèse de 2 et 2′ à partir du 11-céto-progestérone 1

(a) On réalise une pyrolyse du 11-céto-progestérone 1 dans du phénanthrène à 333°C pendant 4 à 5 heures, ce qui donne après vérification par HPLC (45% d'éthylacétate en l'hexane) un mélange de phénols (rapport 2/1, respectivement) dans un rendement de 30 à 60%. La procédure suivie est celle de la référence 5.
(b) Un mélange des phénols ci-dessus (11 g), d'iodure de méthyle (17 ml) et de carbonate de potassium (30 g) dans l'acétone (110 ml) est chauffé au reflux pendant 16 à 20 heures dans une atmosphère d'azote. Le mélange est versé dans 300 ml d'eau et extrait à l'éther (3 x 100 ml). La phase organique est séchée ($MgSO_4$), concentrée sous vide et le résidu est purifié par HPLC (33% d'acétate d'éthyle dans l'hexane) donnant les isomères purs 2 et 2′ dans un rendement combiné de 85%.
- L'isomérisation ultérieure de 2′ en 2 est effectuée avec de l'hydroxyde de sodium dans du méthanol. Une solution de 2′ (5,6 g) et d'hydroxyde de sodium (1 g) dans du méthanol (80 ml) est agitée à température ambiante pendant 2 jours. Le méthanol est concentré sous vide et le résidu est traité à l'éther. Une purification HPLC (33% d'acétate d'éthyle dans l'hexane) donne les isomères purs 2 et 2′. Après 3 isomérisations on obtient 4,9 g de 2 pur.
- Données spectroscopiques pour 2 ;
Infrarouge: 2945 (s), 1700 (s), 1215 (s), 1005 (s), 880 (1) et 695 (s) $cm^{-1}$; $^1H$ RMN (200 MHz, $CDCl_3$) : 7.05 (1H, d, J= 9 Hz), 6.66 (1H, dd: 9, 2.5 Hz), 6.67 (1H, d: 2.5 Hz), 3.78 (3H, s), 2.21 (3H, s), 2.12 (3H, s) et 0.65 (3H, s) ppm.
Pour 2′ ; $^1H$ RMN: 7.05 (1H, d), 6.66 (1H, dd), 6.67 (1H, d), 3.78 (3H, s), 2.21 (3H, s), 2.13 (3H, s) et 0.88 (3H, s) ppm.

Exemple 2

Synthèse de 3 à partir de la dicétone 2 (schéma 1)

- A une suspension d'hydrure de sodium 60% dans de l'huile minérale (1,38 g) lavée à l'hexane dans du tétrahydrofuranne (34 ml) on a jouté lentement du triéthylphosphono acétate (6,82 ml). La solution claire a été ensuite transférée dans une solution de dicétone 2 (2,34 g) dans du tétrahydrofuranne (10 ml) et le mélange réactionnel résultant a été agité à température ambiante pendant 12 heures. Le mélange a été ensuite versé dans une solution saturée de chlorure d'ammonium. Aprés extraction à l'éther, la phase organique a été séchée ($MgSO_4$) et purifiée par colonne de chromatographie sur gel de silice (éluant: 20%

d'acétate d'éthyle dans l'hexane) pour donner 2,27 g de 3 (rendementde 80%) sous forme de mélange 1:1 d'isomères E,Z.

- Données spectroscopiques pour3 ; Infrarouge: 2935 (br, s), 1720, 1705 (s), 1640, 1610 (s), 1500 (s), 1455 (s), 1300-1200 (br, s), 1150 (br, s), 1035 (s) $cm^{-1}$; $^1H$ RMN 7.0 (1H, d: 8 Hz), 6.68 (1H, dd: 8, 2.5 Hz), 6.63 (1H, d: 2.5 Hz), 5.80 et 5.68 (1H, m), 3.77 (3H, s), 2.23 et 2.21 (3H, s), 2.13 et 2.04 (3H, s), 1.29 et 1.28 (3H, t), 0.57 et 0.55 (3H, s) ppm.

Exemple 3

Synthèse de l'oléfine 4 à partir de la cétone 3 (schéma 1)

- A une suspension de bromure de méthyltriphénylphosphonium (4,7 g) dans du tétrahydrofuranne (20 ml) a été ajoutée à-5°C une solution de n-butyllithium dans l'hexane (9,3 ml de une solution 1,4M) (goutte à goutte). Après 15 minutes la suspension jaune a été portée à température ambiante et traitée goutte à goutte avec une solution de cétone 3 (1,8 g) dans du tétrahydrofuranne (10 ml). Après 10 minutes le mélange a été coupé avec une solution saturée de chlorure d'ammonium. Après extraction avec l'hexane (150 ml) et l'éther (50 ml), la phase organique a été lavée avec de l'eau, de la saumure puis concentrée sous vide. Après addition d'hexane au résidu, la suspension résultante a été filtrée et le résidu obtenu après filtration et concentration sous vide a été purifiée par colonne de chromatographie sur gel de silice (20% d'acétate d'éthyle dans l'hexane) pour donner 1,3 g de l'oléfine 4 (rendement 72%).

- Données spectroscopiques; $^1H$ RMN (200 MHz, $CDCl_3$): 7.05 et 7.04 (1H, d: 8 Hz), 6.69 (1H, m), 6.63 (1H, m), 5.77 et 5.64 (1H, m), 4.91 (1H, m), 4.76 et 4.73 (1H, m), 3.76 (3H, s), 2.24 et 2.23 (3H,s), 1.83 et 1.76 (3H, s), 1.29 et 1.27 (3H, t), 0.53 et 0.51 (3H, s) ppm.

Exemple 4

Réduction de l'oléfine 4 en l'alcool 5 (schéma 1)

- A une solution d'oléfine 4 (1,56 g) dans de l'alcool tert-amylique (1,67 ml) du tétrahydrofuranne anhydre (24 ml) et de l'ammoniac liquide (50 ml distillé à partir de sodium), on a ajouté du lithium (175 mg sous forme de petits morceaux de fil de lithium). On ajoute de l'isoprène (0,5 ml) tant que dure la couleur bleue (15 sec). L'ammoniac est évaporé et on ajoute au résidu de l'eau et de l'éther. Après extraction à l'éther, le résidu brut obtenu après concentration sous vide est ensuite réduit selon la méthode classique avec de l'hydrure d'aluminium de lithium dans du tétrahydrofuranne pour donner après purification par HPLC avec de l'acétate d'éthyle 20% dans l'hexane, l'alcool désiré 5 (rendement 85%).

- Données spectroscopiques: RMN $^1H$ (200 MHz, $CDCl_3$): 7.04 (1H, d: 8 Hz), 6.70 (1H, d: 2.5 Hz), 6.65 (1H, dd: 8, 2.5 Hz), 4.87 (1H, m), 4.72 (1H, m), 3.77 (3H, s), 3.72 (2H, t: 7 Hz), 2.23 (3H, s), 1.77 (3H, s), 0.63 (3H, s) ppm.

Exemple 5

Protection de l'alcool 5 aux dérivé éther 6 (schéma 1)

- Une solution d'alcool (9,5 g) du dihydropyranne (9,5 ml) et de l'acide para-toluène sulfonique (100 mg) dans du benzène (150 ml) est agitée pendant 1 heure à température ambiante. La suspension est coupée avec du triéthylamine (2 ml) et filtrée sur florisile (20 g) avec de l'éther (300 ml). Après concentration sous vide, le résidu est purifié par HPLC (5% d'acétate d'éthyle dans l'hexane) pour donner 9 g d'éther pur 6 (rendement 76%).

- Données spectroscopiques; IR: 2935 (s), 1640, 1610, 1580, 1500 (s), 1255 (s), 1035 (s), 800 (s) $cm^{-1}$; MS: m/z à 454 ($M^{+\cdot}$ , 3), 136 (85), 135 (76), 85 (100) ; RMN $^1H$ (200 MHz, $CDCl_3$) : 7.03 (1H, d), 6.71 (1H, d), 6.65 (1H, dd), 4.85 (1H, m), 4.72 (1H, m), 4.60 (1H, m), 3.79 (3H, s), 2.22 (3H, s), 1.78 (3H, s), 0.62 (3H, s) ppm.

Exemple 6

Conversion de l'oléfine 6 dans l'alcool 7 (schéma 1)

- A une solution d'oléfine 6 (9 g) dans du tétrahydrofuranne anhydre (140 ml) on a ajouté une solution de

9-borobicyclo[3.3.1]nonane dans du tétrahydrofuranne (159 ml d'une solution 0.5 M). Après agitation pendant 3 heures à température ambiante sous une atmosphère d'argon, on a ajouté à 0°C une solution d'hydroxyde de sodium 15% (34,5 ml), suivie par de l'eau (29 ml). Pendant que la température est maintenue en dessous de 15°C, on a ajouté du peroxyde d'hydrogène (69 ml d'une solution à 27%) et la solution résultante a été agitée à température ambiante pendant 12 heures. Le mélange a été versé dans une solution aqueuse de sulfite de sodium (55 g). Après extraction à l'éther, la phase organique est séchée ($Na_2SO_4$), concentrée sous vide et le résidu est purifié par HPLC avec de l'acétate d'éthyle 40% dans l'hexane comme éluant pour donner 8,7 g d'alcool 7 (rendement 90%).

- Données spectroscopiques; IR: 3630-3100 (br), 2935 (s), 1255 (s), 1030 (s), 800 (s), 735 (s) $cm^{-1}$; MS: m/z à 267 (9), 207 (16), 127 (24), 109 (27), 95 (27), 85 (22), 83 (27), 81 (43), 71 (59), 41 (100); RMN $^1H$ (200 MHz, $CDCl_3$) : 7.04 (1H, d), 6.65 (1H, dd), 6.70 (1H, d), 4.60 (1H, m), 3.79 (3H, s), 2.22 (3H, s), 1.07 (3H, d), 0.72 (3H, s) ppm.

Exemple 7

Synthèse du tosylate 8 (schéma 1)

- A une solution de l'alcool 7 (6,1 g) et de diméthylaminopyridine (200 mg) dans de la pyridine (40 ml) on a ajouté à -5°C du chlorure de para-toluène sulfonyle (5 g). Après agitation pendant 3 heures, on a ajouté de la glace (5 g) et le mélange résultant a été agité pendant 30 minutes. Après extraction avec l'éther, la phase organique a été lavée avec de la saumure (2 x 15 ml), saturée avec $CuSO_4$ (3 x 100 ml), de la saumure (1 x 15 ml) et saturée avec $NaHCO_3$ (1 x 15 ml). Après séchage ($Na_2SO_4$) la phase organique a été concentrée sous vide et le résidu purifié par HPLC (éluant 17% d'acétate d'éthyle dans l'hexane) pour donner 6,5 g de tosylate 8 (rendement 80%).

- Données spectroscopiques; IR: 2930 (s), 1610, 1600, 1580, 1500, 1465, 1360 (s), 1180 (s), 1035 (s) $cm^{-1}$; RMN $^1H$ (200 MHz, $CDCl_3$) : 7.80 (2H, d), 7.35 (2H, d), 7.03 (1H, d), 6.69 (1H, d), 6.65 (1H, dd), 4.60 (1H, m), 3.79 (3H, s), 2.46 (3H, s), 2.21 (3H, s), 1.01 (3H, d), 0.67 (3H, s) ppm.

Exemple 8

Synthèse de l'acétylène 10a (schéma 1)

- Une suspension d'hydrure de sodium (60% dans de l'huile minérale; 3,3 g; lavée avec 25 ml de pentane) dans du diméthyl sulfoxyde (32 ml) et chauffée à 70°C pendant 2 heures sous azote. Après addition du 1-(tert-butyldiméthylsilyloxy)-1,1-diméthyl-2-acétylène (9a) (25,8 ml) à température ambiante, une solution de tosylate 8 (5 g) dans du diméthyl sulfoxyde (32 ml) est ajoutée au mélange de réaction pendant 30 minutes. Après agitation pendant 1 heure le mélange est versé dans de l'eau (500 ml) et extrait avec de l'étherpentane (5 x). La phase organique est lavée avec une solution saturée de $NaHCO_3$, séchée ($Na_2SO_4$) et concentrée sous vide. Après purification du résidu sur HPLC (éluant: 6% d'acétate d'éthyle dans l'hexane) on a obtenu 4,6 g d'acétylène pure 10a(rendement 87%).

- Données spectroscopiques; IR: 2940, 2240, 1615, 1504, 1470, 1255, 1163 et 940 $cm^{-1}$; RMN $^1H$ (200 MHz, $CDCl_3$) : 7.02 (1H, d; 8 Hz), 6.69 (1H, d), 6.63 (1H, dd: 7,2 1 Hz), 4.59 (1H, m), 3.85 (2H, m), 3.48 (2H, m), 3.76 (3H, s), 2.20 (3H, s), 1.42 (6H, s), 1.08 (3H, d: 6.5 Hz), 0.84 (9H, s), 0.70 (3H, s), 0.16 (6H, s) ppm.

Exemple 9

Hydrogénation catalytique de 10a (schéma 1)

- Une suspension d'acétylène 10a (2,4 g) dans de l'acétate d'éthyle (40 ml) et du triéthalamine (2,3 ml) contenant 10% de palladium sur charbon (620 mg) est hydrogénée sous une pression de 4 atmosphères pendant 3 à 4 heures. Le mélange est traité avec de la célite (3 g) puis filtré, donnant après concentration sous vide, l'éther 11a dans un rendement de 95%.

- Données spectroscopiques de 11a 2930, 1610, 1580, 1500, 1465, 1255 et 1055 $cm^{-1}$; RMN $^1H$ (360 MHz, $CDCl_3$) : 7.03 (1H, d: 8 Hz), 6.69 (1H, d: <1 Hz), 6.63 (1H, dd: 8, <1 Hz), 4.60 (1H, m), 3.85 (2H, m), 3.48 (2H, m), 3.80 (3H, s), 2.23 (3H, s), 1.23 (6H, s) 0.92 (3H, d: 6 Hz), 0.86 (9H, s), 0.70 (3H, s), 0.05 (6H, s) ppm.

Exemple 10

Synthèse de l'acide 12a par oxydation de 11a (schéma 1)

- Une solution de 11a (411 mg) dans du pentane a été traitée avec un gel de silice (20 g; 230-400 mesh) et le pentane a été éliminé sous vide. La silice anhydre ainsi obtenue a été refroidie à -78°C sous atmosphère d'azote. L'ozone a été ensuite passé à un taux de 12 l/heure pendant 15 minutes. Après avoir balayé la silice d'une façon totale avec l'azote à température ambiante le produit a été élué avec de l'acétate d'éthyle. Après concentration sous vide le résidu a été purifié par chromatographie (éluant: acétate d'éthyle 50% dans l'hexane) donnant 173 mg d'acide 12a (rendement 65 %).
- Données spectroscopiques; IR: 3100 (br), 2900, 1715 (s), 1455, 1370, 1355, 1250, 1025, 830, 765 et 680 $cm^{-1}$; NMR $^1H$ (200 MHz, $CDCl_3$) : 4.59 (1H, m), 3.82 (1H, m), 3.47 (2H, m), 1.18 (6H, s), 0.92 (3H, d: 6 Hz), 0.85 (9H, s), 0.68 (3H, s), 0.05 (6H, s) ppm.

Exemple 11

Synthèse de l'oléfine 13a (schéma 1)

- A une solution de l'acide 12a (173 mg) dans du tétrahydrofuranne (0,8 ml) a été ajoutée sous atmosphère d'argon du diimidazole carbonyle (73 mg). Après 2 heures la solution a été transférée à un tube de photolyse à quartz et diluée à un volume de 6,6 ml (0.045 M dans le substrat). Après avoir purgé avec l'argon, la solution a été irradiée à 254 nm pendant 16 heures.

Après concentration le résidu a été purifié par chromatographie sur gel de silice (éluant acétate d'éthyle 5% dans l'hexane) pour donner 51 mg d'oléfine 13a (rendement 32%).
- Données spectroscopiques; IR: 2950, 2850, 1645, 1465, 1380, 1360, 1250, 1030, 935, 735 et 670 $cm^{-1}$; MS: m/z à 520 ($M^{+\cdot}$ , 1), 379 (4), 287 (47), 175 (45), 173 (58), 75 (100); RMN $^1H$ (360 MHz, $CDCl_3$): 4.74 (1H, d: 2 Hz), 4.47 (1H, d: 2 Hz), 4.58 (1H, m), 3.82 (2H, m), 3.46 (2H, m), 1.18 (6H, s), 0.94 (3H, d: 6.2 Hz), 0.85 (9H, s), 0.56 (3H, s), 0.06 (6H, s) ppm.

Exemple 12

Synthèse de la cétone 14a (schéma 1)

- A travers une solution refroidie (-30°C) d'oléfine 13a (10 mg) dans du méthanol (5 ml) on a passé l'ozone jusqu'à ce que la solution devienne bleue. Après un refroidissement ultérieur à -60°C, l'excès d'ozone a été éliminé par balayage avec de l'azote à -60°C. Du diméthyle sulfure (200 ml) a été ajouté et le mélange a été maintenu à 0°C pendant 15 minutes. Après agitation à température ambiante, pendant une durée supplémentaire de 12 heures, la solution a été concentrée sous vide et le résidu a été purifié par chromatographie sur gel de silice (acétate d'éthyle 10% dans l'hexane) pour donner 7,8 mg de cétone 14a (rendement 79%).
- Données spectroscopiques; IR: 3020, 2920, 1720 (s), 1460, 1380, 1360, 1250, 1135, 1120, 1030, 830, 770 $cm^{-1}$; NMR $^1H$ (360 MHz, $CDCl_3$) : 4.56 (1H, m), 3.82 (2H,m), 3.50 (1H, m), 3.40 (1H, m), 1.18 (3H, s), 1.17 (3H, s), 0.96 (3H, d: 5.8 Hz), 0.85 (9H, s), 0.65 (3H, s), 0.06 (6H, s) ppm.

**Exemple 13**

Synthèse de l'alcyne **10c** (schéma 1).

- La synthèse s'effectue de la même façon que décrite dans l'exemple 8 mais en utilisant du 1-(éthoxyéthyloxy)-1,1-diméthyl-2-acétylène **9c**.

Données spectroscopiques de **10c**; RMN $^1H$ (360 MHz, $CDCl_3$): 7.03 (1H, d, 8.2 Hz), 6.69 (1H, d, 2.5 Hz), 6.64 (1H, dd, 2.5, 8.2 Hz), 5.12 (1H, q, 5.2 Hz), 4.60 (1H, q, 3.2 Hz), 3.85 (2H, m), 3.77 (3H, s), 3.68 (1H, m), 3.52 (2H, t, 7.1 Hz), 3.47 (1H, m), 2.63 (1H, ddd, 5.0, 13.1, 13.1 Hz), 2.41 (1H, m), 2.25 (1H, m), 2.17 (3H, s), 1.99 (2H, m), 1.51 (3H, s), 1.44 (3H, s,), 1.33 (3H, d, 5.2 Hz), 1.19 (3H, t, 7.1 Hz), 1.07 (3H, d, 6.5 Hz), 0.70 (3H, s) ppm.

**Exemple 14**

Synthèse de l'acide **12b** (schéma 1).

- La synthèse s'effectue par l'oxydation de **11b** suivant l'exemple **10**.

Données spectroscopiques de **12b**; RMN $^1$H (360 MHz, $CDCl_3$): 4.58 (1H, m), 3.87 (1H, m), 3.77 (1H, m), 3.51 (1H, m), 3.40 (1H, m), 2.40 (1H, m), 2.28 (1H, m), 1.99 (1H, m), 1.21 (6H, s), 0.93 (3H, d, 6.4 Hz), 0.67 (3H, s), and 0.06 (9H, s) ppm.

**Exemple 15**

Synthèse de l'acide **12b** (schéma 1, R = TBDMS).

- La synthèse s'effectue comme décrit dans l'exemple 14.

Données spectroscopiques : RMN $^1$H (360 MHz, $CDCl_3$): 3.62 (2H, m), 2.32 (2H, m), 1.95 (1H, d, 13 Hz), 1.20 (6H, s), 0.93 (3H, d, 6.5 Hz), 0.90 (9H, s), 0.67 (3H, s), 0.10 (9H, s) and 0.05 (6H, s) ppm.

**Exemple 16**

Synthèse de l'oléfine **13b** (schéma 1).

- La synthèse s'effectue au départ de l'acide **12b** comme décrit dans l'exemple **11**.

Données spectroscopiques : RMN $^1$H (360 MHz, $CDCl_3$): 4.74 (1H, s), 4.59 (1H, m), 4.47 (1H, brs), 3.87 (1H, m), 3.79 (1H, m), 3.51 (1H, m), 3.41 (1H, m), 2.35 (1H, m), 2.07 (1H, m), 1.20 (6H, s), 0.94 (3H, d, 6.1 Hz), 0.57 (3H, s), 0.10 (9H, s) ppm.

**Exemple 17**

Synthèse de la cétone **14b** (schéma 1, R = TBDMS).

- Une solution de l'oléfine **13b** (R = TBDMS) (85 mg) dans un mélange de méthanol anhydre (37 ml), de dichlorométhane anhydre (6,8 ml) et de pyridine anhydre (0,68 ml) est refroidie à -70°C et soumise à une passage d'ozone à un taux de 10 l/heure durant 3 minutes. Ensuite l'excès est balayé avec de l'azote et du diméthyl sulfure (0,08 ml) est ajouté. La solution est ramenée à température ambiante et après une durée supplémentaire de 12 heures une solution tampon de phosphate (0,5 M, pH 7.0, 50 ml) est ajoutée et le mélange est concentré sous vide. Après extraction de la solution tamponée (4 fois) avec le dichlorométhane, les extraits combinés sont filtrés sur du gel de silice (35-70 mesh) et le filtrat concentré sous vide. Le résidu est dissous dans du dichlorométhane (5 ml) et traitée avec du N-(triméthylsilyl)imidazole (0,3 g). Après agitation durant 2 heures, la solution est concentrée sous vide et le résidu purifié par HPLC (n-hexane-acétate d'éthyle 95:5) pour donner 63 mg de la cétone **14b** (R = TBDMS) (rendement 74 %).

Données spectroscopiques : RMN $^1$H (360 MHz, $CDCl_3$): 3.63 (2H, m), 2.30 (4H, m), 1.91 (1H, m), 1.70 (1H, m), 1.19 (6H, s), 0.96 (3H, d, 6.5 Hz), 0.88 (9H, s), 0;63 (3H, s), 0.09 (9H, s), 0.05 (6H, s) ppm.

**Exemple 18**

L'ozonolyse de l'alcool **16** (schéma 2).

- A une solution de l'alcool **16** (8,8 g) dans l'hexane (200 ml) est ajouté du gel de silice (53 g; 230-400 mesh) et le mélange est concentré sous vide. Une suspension du résidu dans le fréon 11 (600 ml) est traitée à l'ozone à -25°C jusqu'à obtenir une couleur bleue. On ramène la température à 4°C sur une période de 3 heures. Ce procédé est répété. L'excès d'ozone est ensuite balayé dans un courant d'azote et le mélange est filtré à -30°C. Ensuite les produits d'oxydation sont libérés par extraction de la silice avec l'acétate d'éthyle (250 ml). Après concentration sous vide le résidu est purifié par chromatographie sur gel de silice en éluant avec de 10 à 50 % d'éther-hexane. L'on obtient de cette façon 5,053 g de cétone **17** (rendement 57 %) et 658 mg d'alcool **18** (rendement 7 %).

Données spectroscopiques de **18**; RMN $^1$H (360 MHz, $CDCl_3$) : 2.45 (1H, dd : 7.3 et 11.6 Hz), 1.22 (6H, s), 0.97 (3H, d, 6.1 Hz), 0.64 (3H, s) ppm. MS m/z : 280 ($M^+$, 0.6), 279 ($M^+$-1, 1), 262 (21), 247 (9), 178 (12), 229 (2.5), 207 (2.5), 191 (3), 178 (12), 149 (25), 124 (25), 111 (41), 95 (27), 81 (40), 69 (39), 59 (80), 55 (88), 43

(100).

**Exemple 19**

Dérivatisation de l'alcool **18** en triméthylsilyle **15b** (schéma 2).

- Une solution de l'alcool **18** (200 mg) et de N-(triméthylsilyl)imidazole (0,714 ml) dans le dichlorométhane (13 ml) est agitée à température ambiante durant 16 heures. Après avoir ajouté de l'eau (4,9 ml) on laisse agiter durant 20 minutes. Le mélange est ensuite extrait avec de l'acétate d'éthyle. Les phases organiques sont lavées à la saumure et sechées. Après concentration sous vide le résidu est purifié par chromatographie sur gel de silice (230-400 mesh) (12 % diéthyléther dans l'hexane) pour donner 200 mg de dérivé **15b** (rendement 80 %).
Données spectroscopiques : MS : m/z à 480 ($M^{+\cdot}$, 1,) 465 (3), 422 (3), 396 (2), 378 (1), 351 (1), 337 (7), 321 (3), 306 (4), 293 (2), 267 (2), 261 (1), 250 (1), 241 (2), 227 (1), 193 (1), 173 (1), 155 (2), 143 (2), 131 (100), 85 (48), 73 (36).

**Exemple 20**

Dérivatisation de l'alcool **18** en éther éthoxy éthyle **15a** (schéma 2).

- A une solution de l'alcool **18** (515 mg) dans le dichlorométhane (10 ml) est ajouté de l'éthoxyéthylène (6 ml) et 5 gouttes d'acide trifluoroacétique. On agite à température ambiante durant 24 heures. Après addition d'éther on lave au bicarbonate de soude et à la saumure. Après séchage ($MgSO_4$), filtration et concentration sous vide le résidu est purifié par chromatographie sur gel de silice (10 % à 50 % diéthyléther-hexane) pour donner 428 mg d'éther **15a** (66 % de rendement).
Rf dans 50 % d'éther-hexane : 0,44.
Données spectroscopiques : $^1H$ NMR (500 MHz, $CDCl_3$) : 4.86 (1H, k, 5.27 Hz), 3.49 (2H, m), 2.44 (1H, dd, 7.47 Hz, 11.7 Hz), 2.24 (2H, m), 2.12 (1H, m), 1.26 (3H, d, 5.30 Hz), 1.20 (3H, s), 1.18 (3H, s), 1.17 (3H, t, 6.30 Hz), 0.95 (3H, d, 6.14 Hz), 0.63 (3H, s) ppm.

**Exemple 21**

Synthèse du tosylate **20** (schéma 2).

- Une solution de diol **19** (1,9 g) et de chlorure de paratoluène sulfonyle (2,56 g) dans la pyridine (38 ml) est gardée à 0°C durant 14 heures. Après avoir ajouté de la glace, la suspension est extraite avec 40 % d'acétate d'éthyle dans l'hexane. La phase organique est lavée à l'acide hydrochlorique 5 %, l'eau et du bicarbonate saturé. Après séchage, on concentre sous vide et le résidu est purifié sur gel de silice (50 % d'éthyléther dans l'hexane) pour donner 2,831 g de **20** ( (rendement 86 %).
Rf de **20** dans 50 % d'éther-hexane : 0,16.
Données spectroscopiques : $^1H$ NMR (500 MHz, $CDCl_3$) : 7.78 (2H, d, 7.76 Hz), 7.34 (2H, d, 8.04 Hz), 4.06 (1H, bs), 3.95 (1H, dd, 2.76 Hz, 9.00 Hz), 3.81 (1H, dd, 6.25 Hz, 9.17 Hz), 2.44 (3H, s), 0.96 (3H, d, 6.61 Hz), 0.89 (3H, s) ppm.

**Exemple 22**

Synthèse de l'alcyne **21a** (schéma 2).

- La synthèse s'effectue de façon analogue à celle décrite dans l'exemple 13. En utilisant 2,3 g d'hydrure de sodium (60 %; lavée avec 10 ml d'hexane), 26 ml de DMSO, 16 ml d'acétylène et 2,372 g de tosylate dans 26 ml de DMSO l'on obtient 1,723 g d'alcyne **21a** (76 % rendement).
Données spectroscopiques : $^1H$ NMR (500 MHz, $CDCl_3$) : 5.12 (1H, k, 5.14 Hz), 4.08 (1H, bs), 3.68 (1H, m), 3.49 (1H, m), 2.24 (1H, dd, 3.47 Hz, 16.6 Hz), 2.03 (1H, dd, 7.56 Hz, 16.6 Hz), 1.98 (1H, m), 1.82 (3H, m), 1.50 (3H, s), 1.43 (3H, s), 1.32 (3H, ad, 5.22 Hz), 1.18 (3H, et, 7.01 Hz), 1.04 (3H, d, 6.56 Hz), 0.94 (3H, s) ppm.
MS m/z : 335 (0.07), 306 (1.2), 291 (1.6), 277 (1.2), 259 (2.0), 243 (4.5), 227 (0.92), 217 (1.2), 201 (1.9), 187 (3.4), 173 (2.3), 161 (5.5), 149 (7.6), 135 (9.0), 121 (5.5), 107 (12), 95 (15), 81 (19), 73 (100), 67 (17), 55 (40), 45 (79).

**Exemple 23**

La synthèse de l'alcool **22a** (schéma 2).

- Une solution de l'alcool **21a** (1,723 g) dans de l'acétate d'éthyle (40 ml) contenant de la triéthylamine (2,3 ml) et du palladium/C (10 %; 620 mg) est traitée sous pression de 2,8 atmosphère d'hydrogène durant une période de 12 heures. Après l'addition de célite, on filtre la solution qui ensuite est concentrée sous vide. Le résidu ainsi obtenu est purifié sur gel de silice (15 à 20 % d'éther-hexane) pour donner 1,573 g de **22a** (rendement 90 %).
Rf de **22a** dans 40 % de diéthyléther dans l'hexane : 0,31.
Données spectroscopiques : $^1$H NMR (500 MHz, $CDCl_3$) : 4.86 (1H, k, 5.24 Hz), 4.06 (1H, bs), 3.49 (2H, m), 1.99 (1H, m), 1.81 (3H, m), 1.26 (3H, ad, 5.22 Hz), 1.19 (3H, s), 1.18 (3H, s), 1.17 (3H, t, 6.80 Hz), 0.92 (3H, s), 0.89 (3H, d, 6.40 Hz) ppm.

**Exemple 24**

L'oxydation de l'alcool **22a** (schéma 2).

- Une suspension de l'alcool **22a** (1,4 g) et de gel de silice (10 g; 230-400 mesh) dans l'hexane (150 ml) est concentré sous vide. Une suspension du résidu dans le fréon 11 (150 ml) est traitée à -78°C durant 15 minutes à l'ozone jusqu'à atteindre une couleur bleue. Ensuite on maintient le mélange à -78°C durant 2 heures. Ce procédé est répété. Après évacuation de l'excès d'ozone par passage d'azote, on filtre à -30°C. Les produits d'oxydation sont élués avec l'acétate d'éthyle (150 ml). Après concentration sous vide le résidu est purifié sur gel de silice (10 % à 20 % diéthyléther-hexane) pour donner 983 mg de **15a** (rendement 70 %).
Données spectroscopiques : $^1$H NMR (500 MHz, $CDCl_3$) : 4.86 (1H, k, 5.27 Hz), 3.49 (2H, m), 2.44 (1H, dd, 7.47 Hz, 11.7 Hz), 2.24 (2H, m), 2.12 (1H, m), 1.26 (3H, d, 5.30 Hz), 1.20 (3H, s), 1.18 (3H, s), 1.17 (3H, t, 6.30 Hz), 0.95 (3H, d, 6.14 Hz), 0.63 (3H, s) ppm.

**Exemple 25**

Synthèse de l'énone **24a** (schéma 3)

- Une solution de la cétone **15a** (1,587 g) dans le THF (9 ml) est ajoutée goutte à goutte sur une période de 10 minutes à une solution contenant du lithium diisopropylamide, préparée à partir de diisopropylamine (0,833 ml) et de n-butyllithium (2,5 ml d'une solution 2,19 M dans l'hexane) à -20°C, dans la THF (9 ml) refroidie à -78°C. Après agitation à -40°C l'on ajoute une solution de bromure de phénylsélényle (1,305 g) dans le THF (4,5 ml). Après l'addition d'une solution de chlorure d'ammonium saturée (20 ml) et d'éther (40 ml), la phase organique est lavée à l'eau, au bicarbonate saturé, à la saumure et séchée. Après concentration sous vide le résidu est purifié sur gel de silice (10 % à 12 % diéthyléther-hexane) pour donner 1,753 g de sélénure **23a** (rendement 77 %).
A une solution du sélénure **23a** (1,753 g) dans le dichlorométhane (15 ml) refroidie à -15°C est ajoutée goutte à goutte une solution d'acide méta chloroperbenzoique (0,773 g) dans le dichlorométhane (30 ml) sur une période de 10 minutes. Le mélange réactionnel est ensuite versé dans une solution saturée au bicarbonate et extraite à l'éther. La phase organique est lavée à la saumure et séchée. Après concentration sous vide le résidu est purifié sur gel de silice (10 % à 20 % diéthyléther-hexane) pour donner 0,856g d'énone **24a** (rendement 71 %).
Données spectroscopiques de **24a** : $^1$H NMR (500 MHz, $CDCl_3$) : 6.75 (1H, ddd, 2.18 Hz, 5.73 Hz, 9.93 Hz), 5.98 (1H, ddd, 0.75 Hz, 2.71 Hz, 9.93 Hz), 4.86 (1H, k, 5.26 Hz), 3.49 (2H, m), 2.62 (1H, ddd, 0.75 Hz, 5.79 Hz, 18.6 Hz), 2.56 (1H, dd, 8.88 Hz, 10.5 Hz), 2.40 (1H, dt, 2.31 Hz, 18.6 Hz), 1.26 (3H, d, 5.23 Hz), 1.20 (3H, s), 1.18 (3H, s), 1.16 (3H, t, 7.05 Hz), 0.93 (3H, d, 6.42 Hz), 0.73 (3H, s) ppm.

**Exemple 25bis**

Synthèse de l'énone **24b** (schéma 3)

- Cette synthèse s'effectue à partir de la cétone **15b** de la même façon que décrite dans l'exemple **25**.
Données spectroscopiques de **24b** : $^1$H NMR (360 MHz, $CDCl_3$) : 6.76 (1H, ddd, 2.25 Hz, 5.75 Hz, 9.95 Hz), 5.99 (1H, ddd, 0.93 Hz, 3.1 Hz, 9.93 Hz), 2.64 (1H, ddd, 1.1 Hz, 5.82 Hz, 18.4 Hz), 2.58 (1H, dd, 8.8 Hz, 10.7

Hz), 2.42 (dt, 1H, 2.67 Hz, 18.5 Hz), 1.95 (1H, m), 1.20 §6H, s), 0.95 (3H, d, 6.32 Hz), 0.76 (3H, s), 0.10 (9H, s) ppm.

**Exemple 26**

L'addition conjuguée à l'énone **24**; synthèse de **25a** (schéma 3)

- A une suspension de CuI (0,117 g) dans le diéthyléther (0,5 ml) à 0°C est ajoutée une solution de méthyllithium dans l'éther (0,74 ml d'une solution 1,4 Molaire). Après agitation à 0°C durant une période de 20 minutes une solution de l'énone **24a** (60 mg) dans l'éther (0,5 ml) est ajoutée. Après agitation supplémentaire durant 30 minutes, le mélange réactionnel est versé dans une solution saturée de chlorure d'ammonium. Après extraction à l'éther, la phase organique est lavée à la saumure et séchée. Après concentration sous vide le résidu est purifié sur gel de silice (éther-hexane,1:9) pour donner 55 mg de cétone **25a** (rendement 80 %).
Données spectroscopiques : $^1H$ NMR (500 MHz, $CDCl_3$) : 4.86 (1H, k, 5.26 Hz), 3.49 (2H, m), 2.41 (1H, dd, 7.42 Hz, 11.7 Hz), 2.30 (1H, dd, 5.34 Hz, 13.4 Hz), 2.16 (1H, m), 2.13 (1H, dd, 3.78 Hz, 12.5 Hz), 1.90 (1H, td, 1.2 Hz, 12.2 Hz), 1.89 (1H, m), 1.69 (1H, m), 1.26 (3H, d, 5.31 Hz), 1.20 (3H, s), 1.18 (3H, s), 1.17 (3H, t, 6.81 Hz), 1.03 (3H, d, 6.27 Hz), 0.95 (3H, d, 5.93 Hz), 0.63 (3H, s) ppm. MS : m/z : 308 (1), 277 (3), 259 (24), 236 (1.5), 205 (11), 203 (11), 165 (6), 125 (4), 109 (3), 95 (7), 81 (8), 73 (100), 69 (17), 55 (19), 45 (40).

**Exemple 27**

L'addition conjuguée à l'énone **24**; synthèse de **26a** (schéma 3)

- A une suspension de CuCN (81 mg) dans le THF (1 ml) à -78°C est ajoutée une solution de phényllithium (0,9 ml d'une solution 2 Molaire dans le cyclohexane-diéthyléther). Après 10 minutes l'on ramène la température à -25°C, ensuite 3 minutes à 0°C. Ensuite on ramène la température à -78°C et l'on ajoute une solution de l'énone **24a** (53 mg) dans le THF (1 ml). Après 10 minutes l'on ramène la température à -40°C et l'on agite durant 15 minutes. Après l'addition d'une solution saturée de chlorure d'ammonium et de diéthyléther, la phase organique est lavée à l'eau, au bicarbonate et à la saumure, et ensuite séchée. Après extraction à l'éther, le phase organique est lavée à la saumure et séchée. Après concentration sous vide le résidu est purifié sur gel de silice (diéthyléther-hexane,1:9) pour donner 48 mg de cétone **26a** (rendement 75 %).
Données spectroscopiques : $^1H$ NMR (500 MHz, $CDCl_3$) : 7.33 (2H, m), 7.27 (3H, m), 4.87 (1H, k, 5.25 Hz), 3.50 (2H, m), 3.31 (1H, m), 2.62 (1H, dd, 7.33 Hz, 11.7 Hz), 2.51 (1H, dd, 5.0 Hz, 13.4 Hz), 2.45 (1H, td, 0.8 Hz, 13.4 Hz), 2.34 (1H, dd, 4.1 Hz, 13.0 Hz), 1.95 (1H, m), 1.84 (1H, t, 13.0 Hz), 1.78 (1H, m), 1.27 (3H, d, 5.25 Hz), 1.21 (3H, s), 1.19 (3H, s), 1.18 (3H, t, 7.02 Hz), 0.92 (3H, d, 6.44 Hz), 0.78 (3H, s) ppm.

**Exemple 28**

L'addition conjuguée à l'énone **24**; synthèse de **27a** (schéma 3)

- Une solution de tert-butyllithium (3,342 ml 1,7 Molaire dans l'hexane) est ajoutée à une solution de bromure de vinyle (0,2 ml) dans le diéthyléther (1 ml) refroidie à -78°C sous atmosphère d'argo. Après agitation à -78°C durant 3 heures et demie le mélange est ajouté à une suspension de CuCN (127 mg) dans le diéthyléther (0,5 ml) à -78°C. Après 10 minutes l'on ramène la température à -25°C, puis après 10 minutes à 0°C est ajoutée une solution de l'énone **24b** (70 mg) dans l'éther (1 ml). Après 10 minutes on ramène la température à -40°C et l'on maintient 15 minutes à cette température. Après addition d'une solution ammoniaquée, d'une solution saturée de chlorure d'ammonium et d'éther, la phase organique est séparée et séchée. Après concentration sous vide le résidu est purifié sur gel de silice (diéthyléther-hexane,1:9) pour donner 44 mg de cétone **27a** (rendement 56 %).
Données spectroscopiques de **27a** : $^1H$ NMR (360 MHz, $CDCl_3$) : 5.79 (1H, ddd, 6.8 Hz, 10.3 Hz, 17.1 Hz), 5.02 (1H, dt, 1.3 Hz, 17.1 Hz), 4.97 (1H, dt, 1.2 Hz, 10.3 Hz), 4.86 (1H, h, 5.3 Hz), 3.49 (2H, m), 2.74 (1H, m), 2.44 (1H, dd, 7.4 Hz, 11.7 Hz), 2.34 (1H, dd, 0.9 Hz, 5.2 Hz, 13.7 Hz), 2.19 (1H, dd, 4.1 Hz, 13.2 Hz), 2.10 (1H, ddd, 1.2 Hz, 12.5 Hz, 13.7 Hz), 1.26 (3H, d, 5.3 Hz), 1.20 (3H, s), 1.18 (3H, s), 1.17 (3H, t, 7.0 Hz), 0.96 (3H, d, 6.1 Hz), 0.67 (3H, s) ppm. MS : m/z : 289 (16), 204 (2.5), 177 (7.3), 149 (12), 137 (6.6), 81 (18), 73 (100).

**Exemple 29**

Conversion de l'oléfine **27b** en cétone **30b** (schéma 3)

- A une solution de **27b** (42 mg) dans le THF (1,5 ml) est ajoutée sous atmosphère d'argon une solution de 9-borabicyclononane (0,9 ml d'une solution 0,5 Molaire dans le THF) et le mélange est agité durant 3 heures. Ensuite l'on ajoute de la soude (0,35 ml d'une solution 2 N dans l'eau) et à 0°C une solution d'hydrogène peroxyde (0,35 ml à 30 %). Après agitation à température ambiante durant 12 heures, le mélange réactionnel est versé dans l'eau et extrait à l'éther. La phase organique est lavée au bicarbonate et à la saumure, ensuite séchée. Après concentration sous vide le résidu est purifié sur gel de silice (éther-hexane,7:3) pour donner 26 mg d'alcool **28b** (rendement 59 %). Cet alcool, dissous dans la DMF (0,5 ml) est ajouté à une solution de chlorure de tert-butyldiméthylsilyle (41 mg) et d'imidazole (54 mg) dans la DMF (1,7 ml). Après agitation à température ambiante durant 4 heures, l'on ajoute de la glace, on extrait à l'éther, et on sèche. Après concentration sous vide le résidu est purifié sur gel de silice (éther-hexane,1:9) pour donner 22 mg d'alcool **29b** (rendement 68 %). L'oxydation de ce dernier s'effectue dans le dichlorométhane (2 ml) en présence de pyridinium paratoluène sulphonate (18 mg) et de pyridinium dichromate (200 mg). Après 3 heures l'on ajoute de l'éther et la suspension est filtrée sur de la célite. Le filtrat est levé avec une solution saturée de sulfate de cuivre(II) et séchée. Après concentration sous vide le résidu est purifié sur gel de silice (éther-hexane,1:9) pour donner 211 mg de cétone **30b** (rendement 55 %).
Données spectroscopiques : $^1$H NMR (500 Mhz, $CDCl_3$) : 3.64 (2H, m), 2.44 (1H, ddd, 0.9 Hz, 7.34 Hz, 11.8 Hz), 2.36 (1H, ddd, 1.31 Hz, 5.17 Hz, 13.4 Hz), 2.26 (1H, m), 2.21 (1H, ddd, 1.4 Hz, 4.44 Hz, 13.0 Hz), 1.93 (1H, ddd, 1.43 Hz, 12.0 Hz, 13.5 Hz), 1.20 (6H, s), 0.98 (3H, d, 6.2 Hz), 0.89 (9H, s), 0.65 (3H, s), 0.10 (15H, 3s) ppm.

**Exemple 30**

Synthèse de la cétone **38a** substituée en $11_\beta$ au départ de **26a** (schéma 3)

- A une solution de lithium diisopropylamide, obtenue à partir de diisopropylamine (0,024 ml) et de n-butyllithium (0,084 ml d'une solution 2 Molaire dans l'hexane) dans le THF (0,5 ml) est ajouté à -78°C une solution de la cétone **26a** (60 mg) dans le THF (0,5 ml). On ramène la température à -40°C durant 10 minutes. Une solution de bromure de phénylsélényle (40 mg) dans le THF (0,5 ml) est alors ajoutée goutte à goutte à -78°C. On ajoute de l'eau et de l'éther. La phase organique est lavée à l'eau, au bicarbonate, à la saumure, et séchée. Après concentration sous vide, le résidu est purifié sur gel de silice (éther-hexane, 1:9) pour donner 40 mg de sélénure **32a** (rendement 50 %).
A une solution de ce dernier dans le dichlorométhane (1 ml) à -15°C est ajoutée une solution d'acide métachloroperbenzoique (53 mg) dans le dichlorométhane (1 ml) durant une période de 10 minutes. L'on ajoute du bicarbonate saturé et de l'éther. La phase organique est lavée à la saumure et séchée. Après concentration sous vide le résidu est purifié sur gel de silice (diéthyléther-hexane,1:9) pour donner 5 mg d'anone **35a** (rendement 20 %).
Une solution de cette énone (13 mg) dans l'acétate d'éthyle (2 ml), contenant de la triéthylamine (0,12 ml) et du palladium/C 10 %, est réduite sous pression d'hydrogène de 3,4 atmosphère durant 20 heures. Après filtration sur célite, le filtrat est concentré sous vide et le résidu purifié par chromatographie sur gel de silice (éthyléther-hexane, 1:9) pour donner 4 mg de cétone **38a** (rendement 30 %) ainsi que 4 mg de dérivé dont la fonction cétonique a été réduite.
Données spectroscopiques : $^1$H NMR (500 MHz, $CDCl_3$) : 7.33 (2H, m), 7.23 (3H, m), 4.87 (1H, k, 5.23 Hz), 3.50 (2H, m), 3.41 (1H, m), 2.94 (1H, dd, 8.10 Hz, 10.9 Hz), 2.58 (1H, dd, 12.7 Hz, 18.6 Hz), 2.54 (1H, dd, 3.6 Hz, 18.6 Hz), 2.39 (1H, dd, 8.0 Hz, 14.1 Hz), 2.00 (1H, m), 1.77 (1H, m), 1.76 (1H, dd, 10.7 Hz, 14.1 Hz), 1.68 (1H, m), 1.27 (3H, d, 5.25 Hz), 1.20 (3H, s), 1.18 (3H, s), 1.17 (3H, t, 6.95 Hz), 0.89 (3H, d, 6.50 Hz), 0.80 (3H, s) ppm.

**Exemple 31**

Synthèse de la cétone **37a** (schéma 3)

- La synthèse peut s'effectuer au départ de la cétone **25a** en suivant l'exemple 30. Au départ de l'énone **24a** le procédé suivant donne également la cétone **37a**.

A une suspension de CuI (98 mg) dans le diéthyléther (0,4 ml) à 0°C est ajoutée une solution de méthyllithium (0,74 ml d'une solution 1,4 Molaire dans l'éther). Après agitation durant une période de 20 minutes une solution de l'énone **24a** (60 mg) dans l'éther (0,5 ml) est ajoutée. Après agitation durant 30 minutes, le mélange est refroidi à -78°C. A cette température une solution de bromure de phénylsélényle (45 mg) est ajoutée goutte à goutte. Le mélange est ensuite versé dans une solution saturée de chlorure d'ammonium et extraite avec l'éther. Après lavage à l'eau, au bicarbonate, et à la saumure, la phase organique est séchée. Après concentration sous vide, le résidu est dissous dans le dichlorométhane (1 ml) à -15°C et traité avec de l'acide métachloroperbenzoique (20 mg) dans le dichlorométhane (1 ml). Le mélange réactionnel est ensuite versé dans du bicarbonate saturé et extrait à l'éther. La phase organique est lavée à la saumure et séchée. Après concentration sous vide le résidu est purifié sur gel de silice (éther-hexane,1:9) pour donner 5 mg d'énone **34a** (rendement 48 %). L'hydrogénation s'effectue comme décrit précédemment dans l'exemple 30. La cétone **37a** est obtenue avec un rendement de 62 %.
Données spectroscopiques : $^1$H NMR (500 MHz, $CDCl_3$) : 4.87 (1H, k, 5.25 Hz), 3.50 (2H, m), 2.76 (1H, dd, 8.05 Hz, 11.0 Hz), 2.34 (1H, dd, 4.52 Hz, 18.5 Hz), 2.25 (1H, m), 2.15 (1H, dd, 7.78 Hz, 13.8 Hz), 1.98 (1H, dd, 12.2 Hz, 18.6 Hz), 1.94 (1H, m), 1.70 (1H, m), 1.60 (1H, m), 1.27 (3H, d, 5.29 Hz), 1.21 (3H, s), 1.19 (3H, s), 1.18 (3H, t, 7.04 Hz), 1.00 (3H, d, 6.51 Hz), 0.94 (3H, d, 6.49 Hz), 0.69 (3H, s) ppm.

**Exemple 32**

Synthèse de la cétone **39a** substituée en $11_\beta$ au départ de **14a** (schéma 3)

- La procédure est en tous points analogue à celle décrite dans l'exemple 30.
Données spectroscopiques de **39a** : $^1$H NMR (360 MHz, $CDCl_3$) : 4.87 (1H, k, 5.24 Hz), 4.56 (1H, k, 2.3 Hz, 4.2 Hz), 3.81 (2H, m), 3.48 (4H, m), 2.78 (1H, dd, 8.17 Hz, 10.7 Hz), 2.43 (1H, m), 2.30 (1H, m), 2.18 (1H, m), 2.00 (1H, dd, 12.2 Hz, 18.5 Hz), 1.27 (3H, 2d, 4.95 Hz), 1.20 (3H, s), 1.18 (3H, s), 1.17 (3H, t, 6.88 Hz), 0.93 (3H, d, 6.35 Hz), 0.69 (3H, s) ppm.

**Exemple 33**

Condensation de **14a** avec **40**; synthèse de **43a** (schéma 4a)

- A une solution refroidie (-78°C) de phosphonate **40** (réf. 1; 92 mg) dans du tétrahydrofuranne anhydre (0,75 ml) a été ajoutée sous argon une solution de n-butyllithium (68, 4 µl d'une solution 2,10 Molaires dans l'hexane). Après agitation pendant 30 minutes à -78°C une solution de cétone **14a** (40 mg) dans du tétrahydrofuranne anhydre (0,8 ml) a été ajoutée goutte à goutte et la solution résultante a été agitée à -78°C pendant 1 heure et demie. Le mélange a été porté à température ambiante, traité à l'eau (2 gouttes) et concentré sous vide. Le résidu a été purifié par chromatographie sur gel de silice (acétate d'éthyle 5 % dans l'hexane) pour donner le triène protégé souhaité **43a**.
Données spectroscopiques : RMN $^1$H (360 MHz, $CDCl_3$) : 6.24 (1H, d : 11.3 Hz), 6.01 (1H, d: 11.3 Hz), 5.18 (1H, s), 4.87 (1H, s), 4.62 (1H, m), 4.37 (1H, m), 4.20 (1H, m), 3.86 (2H, m), 3.49 (2H, m), 1.18 (6H, 2s), 0.91 (3H), 0.88 (17H, s), 0.86 (9H, s), 0.54 (3H, s), 0.08 (18H, br, s) ppm.

**Exemple 34**

Déprotection sélective de **43a**; synthèse de **45a** et formation de l'hémi-succinate **46a** (schéma 4a).

(1) Synthèse de **45a** :

- A une solution de triène **43a** protégée dans l'éther on a ajouté une solution de bromure de magnésium anhydre (6 équivalents) dans l'éther. Après 2 heures on a rajouté du bromure de magnésium. Après 4 heures le mélange de réaction a été versé dans une solution de $NaHCO_3$ saturé et extrait avec de l'éther. La phase organique a été lavée avec de la saumure, séchée ($Na_2SO_4$) et concentrée sous vide. Après purification du résidu par chromatographie sur gel de silice (éluant : éther-hexane) le triène silylé **45a** a été obtenu dans un rendement de 61 %.

(2) Formation de l'hémi-succinate **46a:**

- Le triène **45a** (3 mg) a été agité à température ambiante sous atmosphère d'argon dans une solution d'anhydride succinique (10 équivalents) dans la pyrridine anhydre (0,1 ml) en présence d'une quantité catalytique de 4-diméthylaminopyridine.
Quand la réaction est totale (10 jours), la pyridine est éliminée sous vide et le résidu est repris à l'éther puis filtré. On purifie par colonne de chromatographie sur gel de silice (MERCK Kieselgel 60, 230-400 mesh) avec l'hexane-éthyl-acétate (7:3) ce qui a donné l'ester **46a** (3,37 mg; 70 %).
Données spectroscopiques : MS : m/z à 803, 772, 672, 671, 624, 539, 520, 388, 379, 353, 248 et 75.

**Exemple 35**

Condensation de **30b** avec **40**; synthèse de **44b** (schéma 4a)

- La synthèse s'effectue selon le mode opératoire décrit pour la condensation de **14a** avec **40** (exemple 33). La purification s'effectue par chromatographie sur colonne 10 μm gel de silice HPLC avec l'hexane-éther (99:1). A partir de 63 mg de cétone **30b** il a été obtenue 93 mg de triène **44b** (rendement 86 %).
Données spectroscopiques : RMN $^1H$ (360 MHz, $CDCl_3$) : 6.25 (1H, d, 11.2 Hz), 6.02 (1H, d, 11.2 Hz), 5.17 (1H, d, 2.4 Hz), 4.86 (1H, d, 2.4 Hz), 4.37 (1H, dd, 3.7, 6.2 Hz), 4.20 (1H, m), 3.69 (2H, t, 6.5 Hz), 2.88 (1H, dd, 4.0, 13.9 Hz), 2.46 (1H, dd, 3.2, 13.1 Hz), 2.22 (1H, dd, 7.7, 13.1 Hz), 2.03 (1H, dd, 3.6, 12.6 Hz), 1.95 (1H, t, 10.0 Hz), 1.20 (6H, s), 0.94 (3H, d, 6.3 Hz), 0.91 (9H, s), 0.88 (9H, s), 0.87 (9H, s), 0.53 (3H, s), 0.10 (9H, s), 0.06 (18H, 3s). MS : m/z à 876 (9), 875 (13), 874 (9), 743 (78), 248 (100), 1.31 (47), 75 (76) et 73 (58).

**Exemple 36**

Condensation de **14b** avec **40**; synthèse de **43b** (schéma 4a)

- La synthèse s'effectue selon le mode opératoire décrit pour la condensation de **14a** avec **40** (exemple 33). La purification s'effectue par chromatographie sur gel de silice avec l'hexane-éther (96:4) pour donner à partir de 184 mg de cétone **14b** (243 mg de triène protégé **43b** (rendement 75 %).
Données spectroscopiques : UV(MeOH) $\lambda_{max}$ = 263 nm; RMN $^1H$ (360 MHz, $CDCl_3$) : 6.24 (1H, d, 11.2 Hz), 6.02 (1H, d, 11.2 Hz), 5.18 (1H, brs), 4.86 (1H, brs), 4.62 (1H, q, 3.4 Hz), 4.37 (1H, dd, 3.2, 6.0 Hz), 4.19 (1H, m), 3.86 (2H, m), 3.48 (2H, m), 2.90 (1H, m), 2.45 (1H, bd, 13.1 Hz), 2.22 (1H, dd, 7.7, 13.4 Hz), 2.08 (1H, m), 1.96 (1H, t, 9.7 Hz), 1.20 (6H, s), 0.93 (3H, d, 6.2 Hz), 0.87 (18H, s), 0.54 (3H, s), 0.10 (9H, s), 0.06 (12H, s) ppm. MS : m/z at 846 (17), 844 (20), 760 (30), 713 (24), 629 (28), 627 (30), 248 (84), 131 (66), 85 (74), 75 (100), 73 (69), 55 (28), 44 (19).

**Exemple 37**

Synthèse du tétraol **49** (schéma 4a)

(1) Par déprotection de **45a**

- Une solution du triène **45a** dans du THF anhydre a été traité avec 10 équivalents de fluorure de tétra-butylammonium dans du THF (solution 1 M). Le ballon de réaction a été placé sous argon, scellé et agité à 60°C pendant plusieurs jours. Après dilution avec du chlorure de méthylène, la solution a été chromatographiée et le tétraol **49** a été élué avec chlorure de méthylène méthanol (9:1).

(2) Par déprotection de **43b**

- A une solution de triène **43b** (239 mg) dans le méthanol-THF (1:1; 30 ml; anhydre et libre d'oxygène) est ajouté de l'Amberlyst-15 (9 g; lavé 3 fois au méthanol). Le ballon est placé sous argon, protégé de la lumière et agité à température ambiante durant 6 heures. La résine est filtrée et lavée 3 fois au méthanol. Aux filtrats combinés est ajouté de l'hydroxyde d'ammonium à 28 % (1 goutte). Après concentration sous vide, le tétraol est purifiée par chromatographie [gel de silice 230-400 mesh, élution au dichlorométhane-méthanol (9:1); ensuite sur HPLC colonne 10 μm avec le même éluant] pour donner 114 mg de **49** (88 % rendement).

(3) Par déprotection de **44b**

- A une solution de triène protégé **44b** (91 mg) dans le THF anhydre (0,83 ml) est ajouté du fluorure de tétra-butylammonium (1,04 ml d'une solution 1 M dans le THF anhydre). Le ballon est placé sous argon, protégé de

la lumière et agité à température ambiante durant 70 heures. Après concentration sous vide le résidu est purifié comme décrit ci-dessus (exemple 37-2) pour donner 45 mg de tétraol **49** (rendement 94 %).
Données spectroscopiques de **49** : UV(MeOH) $\lambda_{max}$ = 265 nm; IR : 3340, 2940, 2870, 1645, 1570, 1545, 1380, 1365, 1310, 1215, 1150, 1060, 960, 915, 895, 870, 800, 740 $cm^{-1}$. RMN $^1H$ (360 MHz, $CDCl_3$) : 6.37 (1H, d, 11.2 Hz), 6.02 (1H, d, 11.2 Hz), 5.33 (1H, br s), 4.99 (1H, br s), 4.43 (1H, m), 4.23 (1H, m), 3.73 (2H, t, 6.7 Hz), 2.89 (1H, dd, 3.9, 13.3 Hz), 2.60 (1H, dd, 3.5, 13.5 Hz), 2.32 (1H, dd, 6.5, 13.4 Hz), 1.21 (6H, s), 0.94 (3H, d, 6.4 Hz), 0.55 (3H, s) ppm. MS : m/z at 460 (6), 442 (62), 424 (11), 406 (16), 217 (21), 152 (23), 134 (78), 105 (25) et 59 (100).

**Exemple 38**

Synthèse de l'hémisuccinate **50** (schéma 4a)

(1) A partir du tétraol **49**

- Une solution du tétraol **49** (114 mg) et de la 4-diméthylaminopyridine (trace) dans la pyridine anhydre (1 ml) est traitée avec de l'anhydride succinique (64 mg). Le ballon est placé sous argon, protégé de la lumière et la solution agitée à température ambiante durant 4 jours. La pyridine est chassée sous un courant d'argon et le résidu purifié sur gel de silice (230-400 mesh) avec le dichlorométhane-méthanol (85:15) pour donner 83 mg de hémi-succinate **50** (rendement 60 %).

(2) A partir de l'hémisuccinate protégé **46a**

- Une solution de l'hémi-succinate protégé **46a** dans du tétrahydrofuranne anhydre a été traitée avec 10 équivalents de fluorure de tétrabutylammonium dans du tétrahydrofuranne (solution 1 M). Le ballon de réaction a été placé sous argon, scellé et agité à 60°C pendant plusieurs jours. Après dilution avec du dichlorométhane, la solution a été chromatographiée sur MERCK Kieselgel 60 avec du dichlorométhane-méthanol-acide acétique (95:5:1) pour donner l'hémisuccinate **50** (40 %) et du tétraol **49** (10 %).
Données spectroscopiques de **50** : UV(MeOH) $\lambda_{max}$ = 263 nm; IR (KBr) : 3300, 2940, 2840, 1710, 1635, 1560, 1405, 1375, 1240, 1210, 1175, 1100, 1020, 960 $cm^{-1}$. RMN $^1H$ (360 MHz, $CD_3OD$) : 6.33 (1H, d, 11.2 Hz), 6.09 (1H, d, 11.2 Hz), 5.29 (1H, br s), 4.89 (1H, br s), 4.35 (1H, t, 5.7 Hz), 4.17 (2H, t, 6.6 Hz), 4.13 (1H, m), 2.94 (1H, dd, 3.5, 13.0 Hz), 2.54 (6H, m), 2.27 (1H, dd, 6.8, 13.4 Hz), 2.10 (1H, dd, 3.2, 12.7 Hz), 2.00 (1H, dd, 7.1, 11.8 Hz), 1.89 (2H, t, 5.3 Hz), 1.16 (6H, s), 0.98 (3H, d, 6.3 Hz), 0.57 (3H, s) ppm. MS du n-butylester correspondant m/z à 616, 598, 580, 562, 442, 424, 406, 388, 152 et 134.

**Exemple 39**

Synthèse du triol **47** (schéma 4a)

- La synthèse du triol **47** s'effectue par (1) couplage de la cétone
Données spectroscopiques de **41c** : $^1H$ NMR (360 MHz, $CDCl_3$) : 6.25 (1H, d, 11.2 Hz), 6.01 (1H, d, 11.3 Hz), 5.18 (1H, d, 1.67 Hz), 4.87 (1H, k, 5.20 Hz), 4.87 (1H, s of d), 4.37 (2H, dd, 3.55 Hz, 6.51 Hz), 4.19 (1H, m), 3.50 (2H, m), 2.81 (1H, dd, 3.91 Hz, 13.5 Hz), 2.45 (1H, dd, 3.56 Hz, 13.1 Hz), 2.22 (1H, dd, 7.35 Hz, 13.1 Hz), 1.27 (3H, d, 5.26 Hz), 1.20 (3H, s), 1.19 (3H, s), 1.18 (3H, t, 7.07 Hz), 0.94 (3H, d, 6.39 Hz), 0.93 (3H, d, 6.21 Hz), 0.88 (9H, s) 0.87 (9H, s), 0.53 (3H, s), 0.06 (12H, 2s) ppm.
Données spectroscopiques de **47** : $^1H$ NMR (360 MHz, $CD_3OD$) : 6.33 (1H, d, 11.2 Hz), 6.08 (1H, d, 11.2 Hz), 5.39 (1H, dd, 1.19 Hz, 2.16 Hz), 4.89 (1H, d, 1.99 Hz), 4.35 (1H, t, 5.90 Hz), 4.12 (1H, m), 2.85 (1H, dd, 3.98 Hz, 13.4 Hz), 2.52 (1H, dd, 3.46 Hz, 13.3 Hz), 2.26 (1H, dd, 6.72 Hz, 13.4 Hz), 1.16 (6H, s), 0.97 (3H, d, 5.72 Hz), 0;96 (3H, d, 6.15 Hz), 0.57 (3H, s) ppm.

**Exemple 40**

Synthèse du triol **48** (schéma 4a)

- La synthèse du triol **48** s'effectue de la même façon que pour la synthèse du dérivé **47** (exemple 39).
Données spectroscopiques de **48** : $^1H$ NMR (360 MHz, $CD_3OD$) : 7.27 (4H, m), 7.17 (1H, m), 6.31 (1H, d, 11.0 Hz), 6.16 (1H, d, 11.2 Hz), 5.31 (1H, bs), 4.92 (1H, bs), 4.36 (1H, t, 5.82 Hz), 4.12 (1H, m), 2.98 (1H, dd, 4.40 Hz, 13.1 Hz), 2.86 (1H, m), 2.50 (1H, dd, 3.59 Hz, 13.4 Hz), 2.24 (1H, dd, 6.89 Hz, 13.5 Hz), 2.16 (2H, m), 1.16

(6H,2s), 0.93 (3H, d, 4.98 Hz), 0.71 (3H, s) ppm.

**Exemple 41**

Condensation de **14a** avec **51** (schéma 4b)

- Selon un protocole analogue tel que décrit pour le couplage de **14a** et de **40** la cétone **14a** (32 mg) a donné le triène protégé **52a** (11 mg; 70 %).
Données spectroscopiques : RMN $^1H$ (360 MHz, $CDCl_3$) : 7.68 (4H, m), 7.40 (6H, m), 6.03 (1H, d : 11.9 Hz), 5.99 (1H, d : 11.9 Hz), 4.98 (1H, d), 4.75 (1H, d), 4.61 (1H, m), 3.50 (2H, m), 3.39 (3H, m), 2.85 (1H, m), 1.18 (3H, s), 1.17 (3H, s), 1.05 (9H, s), 0.93 (3H, d : 6.2 Hz), 0.85 (9H, s), 0.52 (3H, s), 0.071 (3H, s), 0.056 (3H, s) ppm.

**Exemple 42**

Déprotection sélective de **52a** (schéma 4b)

- Dans les mêmes conditions réactionnelles que décrites pour **43a**, l'alcool primaire **54a** a été obtenu (11 mg; rendement 61 %) à partir de **52a** (27 mg).
Données spectroscopiques : RMN $^1H$ (360 MHz, $C_6D_6$) : 7.85 (4H, m), 7.25 (6H, m), 6.49 (1H, d : 11.3 Hz), 6.44 (1H, d : 11.3 Hz), 5.12 (2H, m), 4.1 (1H, m), 3.48 (2H, m), 1.22 (15H, s), 1.15 (3H, d), 1.05 (9H, s), 0.65 (3H, s), 0.20 (6H, s) ppm.

**Exemple 43**

Condensation de **14b** avec **51** (schéma 4b)

- Selon un protocole analogue tel que décrit pour le couplage de **14a** et de **51** (exemple 41) la cétone **14b** (181 mg) a donné le triène protégé **52b** (257 mg; 81 %).
Données spectroscopiques UV(MeOH) $\lambda_{max}$ = 263 nm; RMN $^1H$ (360 MHz, $CDCl_3$) : 7.68 (4H, t, 7.3 Hz), 7.39 (6H, m), 6.02 (1H, d, 11.8 Hz), 5.99 (1H, d, 11.8 Hz), 4.98 (1H, d, 2.5 Hz), 4.75 (1H, d, 2.5 Hz), 4.62 (1H, q, 3.0 Hz), 3.88 (3H, m), 3.48 (2H, m), 2.85 (1H, m), 2.34 (3H, m), 1.20 (6H, s), 1.05 (9H, s), 0.93 (3H, d, 6.2 Hz), 0.52 (3H, s), 0.10 (9H, s) ppm. MS m/z à 839, 755, 754, 753, 317, 239, 199, 131, 85, 75, 73, 69, 57, 55, 45 et 44.

**Exemple 44**

Condensation de **30b** avec **51** (schéma 4b)

- Selon un protocole analogue tel que décrit pour le couplage de **14b** avec **51**(exemple 43) la cétone **30b** (26 mg) a donné le triène **53b**(39 mg; 88 %).
Rf (hexane-diéthyléther, 99:1) = 0.24.

**Exemple 45**

Synthèse du triol **56** (schéma 4b)

(1) Au départ du triène protégé **53b**

-Selon un protocole analogue tel que décrit pour la déprotection de **44b** (exemple 37-3), l'on obtient après 17 heures 9 mg de triol **56** (98 % rendement) à partir de 18 mg de triène **53b**.

(2) Au départ du triène protégé **52b**

- Une solution contenant le triène **52b** (255 mg) et de l'Amberlyst-15 (20 g, lavé 3 fois au méthanol) dans du méthanol-THF anhydre et déoxygéné (30 ml, 1:1), est placée sous argon, protégée de la lumière et agitée à température ambiante durant 2,5 heures. Après filtration et lavage de la résine au méthanol, les filtrats combinés sont traités avec de l'hydroxide d'ammonium à 28 % (1 goutte). Après concentration sous vide le résidu

est dissous dans le THF anhydride (1 ml) et traité avec du fluorure de tétrabutylammonium (0,9 ml d'une solution 1 M dans le THF). Le ballon est placé sous argon, protégé de la lumière et la solution agitée à température ambiante durant 45 heures. On chasse le THF sous un flow d'argon et purifie le résidu par chromatographie sur gel de silice suivi d'HPLC pour obtenir 117 mg de triol **56** (rendement 87 %).
Données spectroscopiques : UV(MeOH) $\lambda_{max}$ = 263 nm; IR : 3350, 3080, 3040, 2940, 2870, 1680, 1465, 1440, 1380, 1360, 1265, 1215, 1160, 1050, 960, 940, 910, 890, 860, 820 et 740 $cm^{-1}$. RMN $^1H$ (360 MHz, $CDCl_3$) : 6.22 (1H, d, 11.2 Hz), 6.03 (1H, d, 11.2 Hz), 5.05 (1H, d, 2.3 Hz), 4.81 (1H, d, 2.3 Hz), 3.95 (1H, m), 3.72 (2H, t, 6.7 Hz), 2.88 (1H, dd, 3.9, 13.3 Hz), 2.58 (2H, dd, 3.4, 13.2 Hz), 2.40 (1H, ddd, 4.9, 7.7, 12.9 Hz), 2.29 (1H, dd, 7.4, 13.0 Hz), 2.17 (1H, ddd, 5.1, 8.5, 13.2 Hz), 1.21 (6H, s), 0.94 (3H, d, 6.4 Hz), 0.55 (3H, s) ppm. MS m/z à 445, 426, 411, 136, 118, 105, 91, 81, 69, 59, 55, 43.

**Exemple 46**

Synthèse de l'hémi-succinate **57** (schéma 4b)

- Une solution de triol **56** (91 mg) et de 4-diméthylaminopyridine (trace) dans la pyridine anhydre (1 ml) est traitée avec de l'anhydride succinique (31 mg). Le ballon est placé sous argon protégé de la lumière et la solution agitée à température ambiante durant 45 heures. On chasse la pyridine sous argon et le résidu est purifié par chromatographie sur gel de silice (230-400 mesh) avec du dichlorométhane-méthanol (9:1), ensuite par HPLC sur colonne 10 μm phase inversée C18-gel de silice éluée au méthanol-eau (3:1) pour donner 65 mg de **57** (rendement 58 %).
Données spectroscopiques UV(MeOH) $\lambda_{max}$ = 263 nm; RMN $^1H$ (360 MHz, $CDCl_3$) : 6.23 (1H, d, 11.2 Hz), 6.04 (1H, d, 11.2 Hz), 5.04 (1H, d, 2.3 Hz), 4.81 (1H, d, 2.3 Hz), 4.20 (2H, t, 6.4 Hz), 3.98 (1H, m), 2.91 (1H, dd, 3.9, 13.4 Hz), 2.65 (4H, m), 2.59 (1H, m), 2.40 (1H, m), 2.31 (1H, dd, 7.4, 12.9 Hz), 2.18 (1H, ddd, 5.1, 8.6, 13.3 Hz), 2.03 (1H, dd, 3.1, 12.4 Hz), 1.21 (6H, s), 0.94 (3H, d, 6.4 Hz), 0.54 (3H, s) ppm. MS m/z à 545, 527, 508, 444, 426, 408, 136, 118, 81, 69, 59, 55 et 44.

**Exemple 47**

Synthèse du triol **63** (schéma 4c)

(1) Couplage de la cétone **37c** avec **40** pour donner **59c**.
La synthèse s'effectue selon le même mode opératoire que pour la synthèse de **41c** à partir de **25c** et donne au départ de 14 mg de cétone **37c** le triène protégé **59c** (6 mg; 27 % rendement).
(2) L'hydrolyse de **59c** s'effectue avec de la résine Amberlyst suivant le mode opératoire décrit pour la conversion de **43b** et **49** (exemple 37-2).
Données spectroscopiques : RMN $^1H$ (360 MHz, $CD_3OD$) : 6.23 (1H, d, 11.2 Hz), 6.11 (1H, dd, 1.9, 11.2 Hz), 5.28 (1H, dd, 1.3, 2.4 Hz), 4.35 (1H, t, 5.9 Hz), 4.12 (1H, m), 2.51 (1H, dd, 3.6, 13.4 Hz), 2.43 (2H, m), 2.26 (1H, dd, 7.0, 13.4 Hz), 1.89 (2H, t, 5.6 Hz), 1.16 (6H, s), 0.96 (3H, d, 7.5 Hz), 0.61 (3H, s) ppm.

**Exemple 48**

Synthèse du tétraol **65** (schéma 4c)

(1) Couplage de la cétone **39c** avec **40** pour donner **61c**.
La synthèse s'effectue de la même façon que décrit pour **59c**. On obtient au départ de 10 mg de cétone 3 mg de triène protégé **61c** (20 % rendement).
Données spectroscopiques : RMN $^1H$ (360 MHz, $CDCl_3$) : 6.14 (1H, d, 11.3 Hz), 6.05 (1H, d, 11.3 Hz), 5.18 (1H, br, s), 4.88 (1H, q, 5.7 Hz), 4.87 (1H, br s), 4.59 (1H, m), 4.37 (1H, dd, 3.6, 6.4 Hz), 4.19 (1H, m), 3.87 (1H, m), 3.79 (1H, m), 3.48 (4H, m), 2.43 (3H, m), 2.17 (2H, m), 1.27 (3H, d, 5.2 Hz), 1.21 (SH, s), 1.19 (3H, s), 1.18 (3H, t, 7.O Hz), 0.91 (3H, d, 6.2 Hz), 0.88 (9H, s), 0.87 (9H, s), 0.58 (3H, s), 0.06 (12H, 3s) ppm.
(2) L'hydrolyse de **61c** s'effectue avec de la résine Amberlyst suivant le mode opératoire décrit pour la conversion de **43b** et **49** (exemple 37-2).
Données spectroscopiques de **65** : UV (MeOH) : λmax = 265 nm = 265 nm; $^1H$ NMR (360 MHz, $CD_3OD$) : 6.24 (1H, d, 11.3 Hz), 6.12 (1H, d, 11.3 Hz), 5.29 (1H, bbs), 4.35 (1H, t, 5.7 Hz), 4.12 (1H, m), 3.59 (2H, m), 2.52 (1H, dd, 3.4, 13.4 Hz), 2.47 (1H, m), 2.44 (1H, m), 2.26 (1H, dd, 6.6, 13.3 Hz), 1.89 (2H, t, 5.6 Hz), 1.16 (6H, s), 0.96 (3H, d, 5.2 Hz) and 0.62 (3H, s) ppm; MS : m/z : 460 $M^{+\cdot}$, 1.4), 442 (23), 424 (5.4),

406 (3.4), 368 (3.0), 313 (2.9), 256 (2.6), 239 (3.0), 185 (3.0), 157 (3.7), 105 (9.6).

**Exemple 49**

Synthèse de l'énone **66** (R'=TBDPS) à partir du diol **20 (R'=H)** (schéma 5)

- A une solution du diol **20** (R'=H) (0,138 g) dans le DMF anhydre (5 ml) est ajoutée goutte à goutte une solution de chlorure de tert.butyldiphénylsilane (0,785 g) et d'imidazole (0.56 g) dans le DMF (15 ml). Après agitation à température ambiante pour 2 heures, de la glace et de la saumure sont ajoutées, suivi d'une extraction à l'éther. L'extrait est seché et concentré sous vide et le résidu est purifié sur gel de silice (éther-hexane 1:9) pour donner 0,19 g de l'alcool **20** (R'=TBDPS) (rendement 65 %).
Données spectroscopiques de **20** : RMN $^1$H (360 MHz, $CDCl_3$) : 7.67 (4H, m), 7.40 (6H, m), 4.07 (1H, br, s), 3.62 (1H, dd, 3.3, 9.7 Hz), 3.37 (1H, dd, 6.9, 9.7 Hz), 2.01 (1H, m), 1.08 (3H, d, 6.6 Hz), 1.06 (9H, s), 0.91 (3H, s) ppm. MS : m/z : 393 (58), 375 (29), 315 (9), 297 (9), 239 (9), 199 (100), 183 (22), 177 (53), 151 (21), 135 (54), 121 (22), 95 (67), 55 (29).
- Une solution de l'alcool **20** (R'-TBDPS)(0,19 g) dans la dichlorométhane (5 ml) contentant du paratoluènesulfonate de pyridine (0,02 g) et du dichromate de pyridine (0,25 g) est agitée à température ambiante pour 1 heure. Le mélange réactionnel est ensuite dilué avec de l'éther et filtré sur célite. Le filtrate est lavé au sulfate de cuivre saturé, seché et concentré sous vide. Purification du résidu sur gel de silice (5 % à 10 % qdiéthyléther-hexane) donne 0,15 g de la cétone correspondante (rendement 80 %).
Données spectroscopiques : RMN $^1$H (360 MHz, $CDCL_3$) : 7.66 (4H, m), 7.40 (6H, m), 3.61 (1H, dd, 2.7, 9.8 Hz), 3.41 (1H, dd, 6.1, 9.8 Hz), 2.42 (1H, dd, 7.6, 11.3 Hz), 2.225 (2H, m), 2.12 (1H, m), 2.01 (1H, m), 1.12 (3H, d, 6.2 Hz), 1.06 (9H, s), 0.61 (3H, s) ppm. MS : m/z : 448 ($M^{+\cdot}$), 391 (100), 313 (3), 295 (4), 283 (2), 239 (7), 199 (74), 175 (27), 135 (27).
- Une solution de la cétone (0,14 g) dans le THF (1 ml) est ajoutée goutte à goutte sur une période de 10 minutes à une solution contenant du lithium diisopropylamide, préparée à partir de diisorpopylamine (0,053 ml) et de n-butyllithium (0,152 ml d'une solution 2,45 M dans l'hexane) à -20°C, dans le THF (1 ml) refroidie à -78°C. AAprès agitation à -40°C l'on ajoute une solution de bromure de phénylsélényle (0.088 g) dans le THF (0,5 ml). Après l'addition d'une solution de chlorure d'ammonium saturée et d'éther, la phase organique est lavée à l'eau, au bicarbonate saturé, à la saumure et sechée. Après concentration sous vide le résidu est purifié sur gel de silice (3 % à 15 % diéthyléther-hexane) pour donner 0,142 g du sélénure (rendement 75 %).
- A une solution de sélénure (0,14 g) dans la dichlorométhane (2 ml) refroidie à -15°C est ajoutée goutte à goutte une solution d'acide méta chloroperbenzoique (0,067 g) dans le dichlorométhane (2 ml) sur une période de 10 minutes. Le mélange réactionnel est ensuite versé dans une solution saturée au bicarbonate et extraite à l'éther. La phase organique est lavée à la saumure et sechée. Après concentration sous vide le résidu est purifié sur gel de silice (3 % à 20 % diéthyléther-hexane) pour donner 0,08 g d'énone **66** (R'-=TBDPS) (rendement 77 %).
Données spectroscopiques de **66** (R'=TBDPS) : RMN $^1$H (360 MHz, $CDCl_3$) : 7.66 (4H, m), 7.41 (6H, m), 6.77 (1H, ddd, 2.2, 5.7, 9.9 Hz), 5.99 (1H, ddd, 0.8, 3.1, 9.9 Hz), 3.62 (1H, dd, 3.1, 9.8 Hz), 3.42 (1H, dd, 6.3, 9.8 Hz), 2.63 (1H, ddd, 0.7, 5.8, 18.5 Hz), 2.55 (1H, t, 9.4 Hz), 2.42 (1H, dt, 2.4, 18.5 Hz), 1.11 (3H, d, 6.4 Hz), 1.07 (9H, s), 0.72 (3H, s) ppm. MS : m/z : 446 ($M^{+\cdot}$), 389 (100), 311 (5), 295 (3), 199 (62), 173 (12), 147 (7), 121 (10).

La réaction est effectuée en tampon borate de sodium 0.1 M à pH 8. A 40 x $10^{-9}$ mol de tyramine (20 µl) on ajoute 4 millicuries de $Na^{125}I$ (environ 1.8 x $10^{-9}$mol, 40 µl) puis 20 x $10^{-6}$ g de chloramine T (10 µl). Exactement 60 secondes plus tard, le mélange réactionnel est additionné de 60 x $10^{-6}$ g de métabisulfite de sodium (30 µl).

(3) Couplage de tyramine radioiodinée

34 x $10^{-9}$mol,de produit de formule **57** préalablement activé avec la N-hydroxysuccinimide (DMF, 25 µl) sont additionnés de 8 x $10^{-9}$mol (tampon phosphate, 20 µl). Le couplage s'effectue pendant 20 heures à 25°C

**Exemple 50**

Synthèse du dérivé **58** à partir du dérivé **57**

(1) Activation du dérivé de formule **57** par la N-hydroxysuccinimide

Un mélange de produit de formule **57** (1.38 x $10^{-7}$mol), de N-hydroxysuccinimide (1.5 x $10^{-7}$mol) et de dicyclohexylcarbodiimide (1.5 x $10^{-7}$ mol) dans du diméthylformamide anhydre (DMF, 100 µl) est placé pendant 16 heures à 25°C dans l'obscurité.

(2) Radioiodination de la tyramine

La réaction est effectuée en tampon borate de sodium 0.1 M à pH 8. A 40 x $10^{-9}$mol de tyramine (20 µl) on ajoute 4 millicuries de $Na^{125}I$ (environ 1.8 x $10^{-9}$mol, 40 µl) puis 20 x $10^{-6}$ g de chloramine T (10 µl). Exactement 60 secondes plus tard, le mélange réactionnel est additionné de 60 x $10^{-6}$ g de métabisulfite de sodium (30 µl).

(3) Couplage de tyramine radioiodinée

34 x $10^{-9}$mol ,de produit de formule **57** préalablement activé avec la N-hydroxysuccinimide (DMF, 25 µl) sont additionnés de 8 x $10^{-9}$mol (tampon phosphate, 20 µl). Le couplage s'effectue pendant 20 heures à 25°C dans l'obscurité. Le produit du couplage, 11α[2-0-($^{125}$I-tyramido succinoyl)éthyl]25 hydroxy vitamine $D_3$ (**58**), est purifié par HPLC (colonne $C_{18}$, gradient d'élution acétonitrile/eau de 0 à 100 %).

**Exemple 51**

Préparation d'un immunogène : couplage du dérivé de formule **50** à la serum albumine bovine

(1) Activation du dérivé de formule **50** par la N-hydroxysuccinimide

Un mélange de dérivé de formule **50** (1.3 x $10^{-6}$mole), de N-hydroxysuccinimide (1.47 x $10^{-6}$mol) et de dicyclohexylcarboddimide (1.47 x $10^{-6}$mol) dans du diméthylformamide anhydre (DMF, 100 µl) est agité pendant 16 heures à 4°C dans l'obscurité.

(2) Couplage du produit activé à la serum albumine bovine.

A 100 µl de produit activé (1.3 x $10^{-6}$mol, DMF) on ajoute la serum albumine bovine (BSA, 22.5 x $10^{-9}$mol) en tampon phosphate 0.1 M pH 7.5 (1.5 ml). Le mélange réactionnel est agité pendant 24 heures à 4°C dans l'obscurité puis dialysé contre une solution de chlorure de sodium (9 g/l) à 4°C. Le conjugué est utilisé tel quel comme immunogène.

**Exemple 52**

Méthode de dosage de la 25-hydroxy-vitamine $D_3$ sérique

(1) Réactifs

Traceur : dérivé de formule **58** obtenu comme décrit dans la présente invention (exemple 50). En solution dans l'éthanol (environ 5 x $10^5$cpm/ml).

Ligand : transporteur plasmatique de la vitamine D (DBP préparé à partir du sérum de rats carrencés en vitamine D (réf. 20). Pour les besoins de cette expérience, le serum a été dilué 9000 fois dans le tampon veronal composition, voir ci-dessous).

Suspension de charbon-dextran : 2 mg de Norit A (120 mesh) et 0.2 mg de dextran T70 par ml.

Solvant pour extraction : cyclohexane/acétate d'éhtyle (l/l, vol/vol).

Tampon : Veronal 0.1 M pH 8.6, merthiolate de sodium 0.2 g/l, ovalbumine 0.1 g/l.

(2) Matériel

Tubes d'extraction (10 ml) en verre avec bouchon à visser.
Tubes à essai en verre siliconé.
Compteur à scintillation gamma.
Centrifugeuse réfrigérée (4°C, 1500 G).
Système d'évaporation sous pression réduite.

(3) Mode opératoire du dosage

Une courbe d'étalonnage est obtenue avec des échantillons sériques précalibrés pour la concentration en 25-hydroxy-vitamine $D_3$ (2.5 à 80 ng/ml).

- 100 $\mu$l de serum additionné de 200 $\mu$l d'eau et de 1 ml de solvant pour extraction sont placés dans un tube d'extraction et agités vigoureusement pendant 30 minutes à 20-25°C.
- un volume 100 $\mu$l de la phase organique est transféré dans un tube à essai en vere siliconé et évaporé sous pression réduite à 40°C.
- dans le tube contenant le résidu de l'extraction de la 25-hydroxy-vitamine $D_3$, ajouter successivement 50 $\mu$l de la solution de traceur (± 25000 cpm) et 0.5 ml ligand. Agiter et incuber pendant 1 Heure à 4°C.
- Ajouter 1 ml de la suspension de charbon-dextran à 4°C, agiter et incuber pendant 30 minute à 4°C.
- Centrifuger (4°C, 1300 xg, 10 min), et mesurer la radioactivité de 1 ml de surangeant dans un compteur à scintillation gamma.

La courbe d'étalonnage est dessinée à l'aide des échantillons précalibrés de concentration connue (cpm = f[concentration]). Les concentrations de 25-hydroxy-vitamine $D_3$ dans les autres sera sont estimées graphiquement sur la courbe d'étallonage.

**Exemple 53**

Résultats d'un dosage utilisant un nouveau dérivé en $C_{11}$ de la vitamine D comme traceur iodé

On a montré selon la présente invention qu'il serait possible d'obtenir des dérivés en $C_{11}$ de la vitamine D qui présentent une affinité pour la DBP comparable au métabolite naturel 25 OH vitamine $D_3$. Les dosages actuels de la 25 OH vitamine $D_3$ dans le sérum sont basés sur la compétition pour la DBP (de poissons, d'oiseaux ou de mammifères) du métabolite naturel 25 OH vitamine $D_3$ et d'un traceur tritié [$^3$H]-25 OH $D_3$, aucun traceur iodé n'étant décrit. La présente invention permet notamment d'utiliser un analogue iodé de la 25 OH vitamine $D_2$ ou 25 OH vitamine $D_3$ comme traceur. A titre d'exemple, la molécule 11$\alpha$[2-0-(m$^{125}$I-tyramidosuccinoyl)ethyl]-25 OH vitamine $D_3$ (58) a été utilisée comme traceur et les résultats du dosage obtenu ont été comparés avec ceux d'un dosage conventionnel utilisant la [$^3$H]-25 OH $D_3$. Il apparaît que pour une large échelle de valeurs (0 - 260 ng/ml) reprenant des valeurs normales et pathologiques extrêmes, la corrélation entre les deux dosages est remarquable (> 0,97 ; n = 204) démontrant la qualité du dosage utilisant un traceur iodé de la présente invention. En particulier cette corrélation est remarquable pour les valeurs basses et paraphysiologiques, où la précision du dosage est particulièrement importante (coef. corrélation > 0,97 ; n = 154). De plus ces résultats montrent un "bruit de fond" ("Background") inférieur avec le traceur iodé ce qui constitue un avantage supplémentaire par rapport à la meilleure technique de référence disponible.

**Exemple 54**

Application thérapeutique des dérivés en $C_{11}$ de la vitamine $D_3$

(1) Certains dérivés en $C_{11}$ lient la DBP avec une haute affinité

La dérivation en $C_{11}$ n'inhibe pas la liaison de l'analogue de la 25OH vitamine $D_3$ à la DBP. A titre d'exemple, la figure (1) montre que la liaison à la DBP humaine purifiée du dérivé 11(2-hydroxy) éthyl 25 OH vitamine $D_3$ (56) est semblable à celle de la 25 OH vitamine $D_3$, et est donc de haute affinité. De plus, l'addition d'un groupe hémisuccinate 11(2-HS)éthyl-25OH vitamine $D_3$ (57) ne modifie pas nécessairement sensiblement cette affinité. Il est connu que la 1,25 $(OH)_2$ vitamine $D_3$ a une affinité plus faible pour la DBP. Cette affinité n'est pas modifiée avec un dérivé 11 hydroxy éthyl. L'expérience est comparable avec de la DBP d'autres origines (poissons, oiseaux, mammifères) et notamment avec la DBP de rat. En conclusion, la dérivation en $C_{11}$ n'altère pas la liaison à la DBP et il est possible de concevoir différents dérivés ayant une plus ou moins grande affinité

pour la DBP. Cette propriété a été mise à profit pour obtenir des analogues iodables par leur dérivation en $C_{11}$, et ces dérivés ont été utilisés avec succès, par exemple, pour la mise au point du premier dosage spécifique de la 25 OH vitamine $D_3$ utilisant un traceur iodé.

(2) Des dérivés en $C_{11}$ lient le récepteur de la vitamine $D_3$ avec une haute affinité

La liaison de la 1, 25 $(OH)_2$ vitamine $D_3$ à son récepteur intracellulaire paraît indispensable à la plupart de ses propriétés biologiques.

La figure (2) montre que les dérivés 11α hydroxy éthyl et 11α éthyl (2-hémisuccinate) de la 1, 25 $(OH)_2$ vitamine $D_3$ lient le récepteur de la vitamine $D_3$. L'affinité cependant est d'environ 100 à 200 fois moindre que celle de la 1, 25 $(OH)_2$ vitamine $D_3$. Cette expérience est reproductible avec des récepteurs d'origines différentes (duodénum de rat dans l'exemple illustré, mais aussi thymus de veau, lymphocytes humains, etc.). Il est possible d'obtenir des dérivés qui ont une meilleure affinité pour le récepteur : par exemple le dérivé 11α phényl de la 1-25 $(OH)_2$ vitamine $D_3$ lie mieux le récepteur que les dérivés 11α hydroxyéthyl, et le dérivé 11α méthyl lie le récepteur avec une affinité comparable à la 1,25 $(OH)_2$ vitamine $D_3$ non substituée.

En conclusion, la dérivation en position $C_{11}$ permet d'obtenir des analogues de la vitamine $D_3$ présentant des affinités variables pour le récepteur spécifique. Certains dérivés ont une haute affinité comparable à la 1-25 $(OH)_2$ vitamine $D_3$, d'autres ont des affinités moyennes ou faibles. Ceci démontre l'intérêt pharmacologique de cette position pour dériver des analogues de la vitamine $D_3$. (Sur le plan diagnostique cela implique notamment la possibilité d'un dosage à l'aie du récepteur spécifique, ou partie de ce récepteur, et d'un traceur iodé au niveau de la dérivation en $C_{11}$).

(3) Des dérivés en $C_{11}$ sont des analogues biologiquement actifs de la vitamine $D_3$.

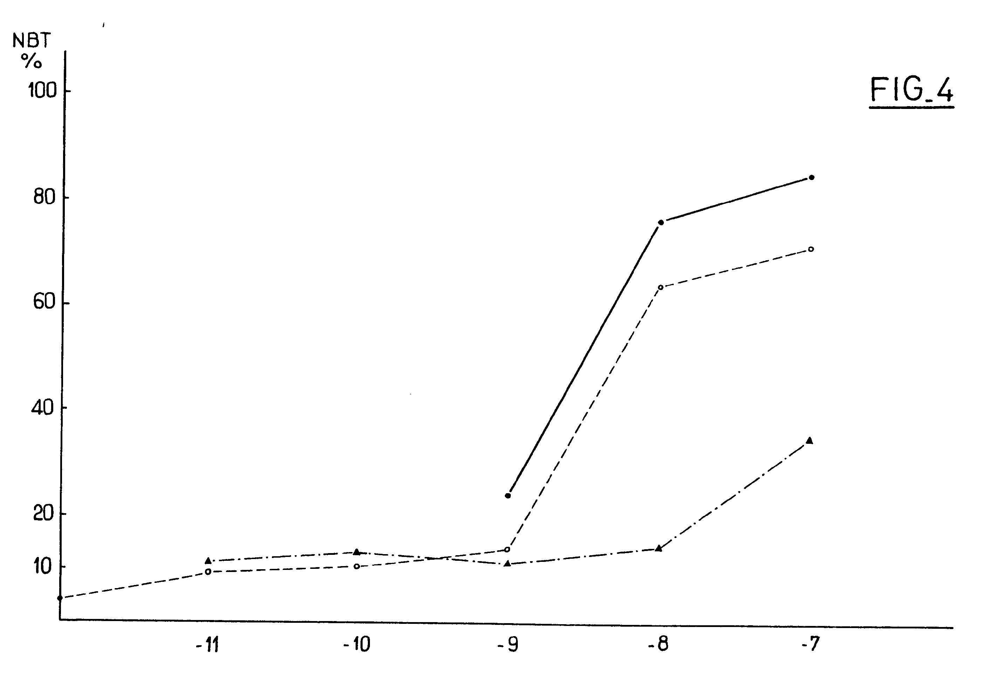

La liaison au récepteur laisse présumer la possibilité d'une activité biologique de certains dérivés en $C_{11}$ de la vitamine $D_3$. Ceci est en effet directement confirmé dans différents tests biologiques (induction de la différenciation de cellules leucémiques HL 60, inhibition de la prolifération lymphocytaire, modification de la prolifération de cellules cancéreuses, effet calciotropique, etc.). Les figures (3-4) illustrent l'effet de différents analogues sur la différenciation des cellules leucémiques HL 60. On voit que les dérivés 11 hydroxy éthyl 1,25 $(OH)_2$ vitamine $D_3$ et 11 hydroxy éthyl 25 OH vitamine $D_3$ sont peu actifs, comme la 25 OH vitamine $D_3$ (figure 3). Par contre, le dérivé 11α phényl 1, 25 $(OH)_2$ vitamine $D_3$ est actif, et le dérivé 11α méthyl 1,25 $(OH)_2$ vitamine $D_3$ présente une forte activité qui approche celle de la 1, 25 $(OH)_2$ vitamine $D_3$ (figure 4).

En conclusion, la dérivation en $C_{11}$ permet d'obtenir des analogues biologiquement actifs de la vitamine $D_3$, et l'intensité de l'effet biologique est modulable en fonction de la substitution en $C_{11}$.

(4)Des dérivés en $C_{11}$ permettent d'observer une dissociation entre différentes propriétés de la vitamine $D_3$.

La synthèse de différents dérivés permet de rechercher des analogues qui ne présentent qu'en partie les différentes propriétés de la vitamine $D_3$, ou dans une proportion distincte. Nous avons montré précédemment, à titre d'exemple, que les liaisons au récepteur de la vitamine $D_3$ des dérivés 11α hydroxyéthyl 1, 25 $(OH)_2$ vitamine $D_3$ et 11α éthyl (2-hémisuccinate) 1,25 $(OH)_2$ vitamine $D_3$ étaient comparables (figure 2). L'affinité pour le récepteur est environ 100 à 200 fois moindre que celle de la 1,25 $(OH)_2$ vitamine $D_3$. Dans un test biologique (inhibition de la prolifération lymphocytaire), le dérivé 11α hydroxy éthyl est également environ 100 fois moins actif que la 1, 25 $(OH)_2$ vitamine $D_3$. Pour le même test, le dérivé 11α éthyl (2-hémissuccinate) est environ 1000 fois moins actif.

En conclusion, différents dérivés en $C_{11}$ peuvent présenter des dissociations entre leurs propriétés, par exemple des activités biologiques distinctes et une affinité pour le récepteur semblable.

(5) Des dérivés en $C_{11}$ ont un effet antagoniste partiel vis-à-vis des prorpriétés de la vitamine $D_3$.

Non seulement certains dérivés en $C_{11}$ lient le récepteur et exercent des activités agonistes comparables à la 1, 25 $(OH)_2$ vitamine $D_3$, mais ils sont également susceptibles d'avoir un effet antagoniste. Ainsi dans un test de prolifération lymphocytaire, l'addition de 1,25 $(OH)_2$ vitamine $D_3$ provoque une nette inhibition de prolifération. Par exemple si la réponse proliférative de lymphocytes humains stimulés par la PHA est de 100 en l'absence de vitamine $D_3$, elle est ramenée à 66,5 en présence de $10^{-11}$ M de 1, 25 $(OH)_2$ vitamine $D_3$ (tableau 1). Dans le même test, l'addition de 11 (2-hydroxy)éthyl, 1, 25 $(OH)_2$ $D_3$ n'induit aucune inhibition à $10^{-10}$ M (102,6). Pourtant cette concentration ($10^{-10}$ M) du dérivé 11(2-hydroxy)éthyl 1, 25 $(OH)_2$ $D_3$, ajoutée à la concentration inhibitrice ($10^{-11}$ M) de 1, 25 $(OH)_2$ vitamine $D_3$, antagonise son effet inhibiteur : la prolifération est ramenée de 66,5 % à 91,8 % (tableau 1).

Tableau 1 : Effet de la 1,25 $(OH)_2$ vitamine $D_3$, du dérivé 11 OH éthyl, et d'un mélange de deux produits sur la prolifération lymphocytaire : le dérivé se comporte comme un antagoniste partiel de la vitamine $D_3$.

| | | 11 (2-OH) Ethyl 1,25 $(OH)_2$ vitamine $D_3$ | |
|---|---|---|---|
| | | 0 | $10^{-10}$ M |
| 1,25 $(OH)_2$ vitamine $D_3$ | 0 | 100 % | 102,6 % |
| | $10^{-12}$ M | 93,8 % | 98,5 % |
| | $10^{-11}$ M | 66,5 % | 91,8 % |

En conclusion, différents dérivés en $C_{11}$ de la vitamine $D_3$ présentent un effet antagoniste sur la forme biologiquement active de la vitamine $D_3$.

Les références bibliographiques 21, 22 et 23 ont servi de références pour la méthodologie des test de liaison à la DBP, au récepteur et du test d'inhibition de prolifération lymphocytaire.

**Références**


1. (Bibliographie générale ou autres brevets
2. V.K. Ostrem et H.F. DeLuca, Steroids (1987) **49**, 73; M.S. Calverley, Tetrahedron (1987) **43**, 80.
3. E.G. Baggiolini, J.A. Jacobelli, B.M. Hennessy et Uskokovic, J. Am. Chem. Soc., (1982) **104**, 2945.
4.
   (a) H.T. Tok et W.H. Okamura, J. Org. Chem. (1983) **48**, 1414;
   (b) J.L. Mascarenas, A. Mourino et L. Castedo, J. Org. Chem. (1986) **51**, 1269.
5. B.J. Magerlin et J.H. Hogg, Tetrahedron (1958) **2** 80.
6. M.M. Midland et Y.C. Kwon, Tetrahedron Lett. (1985) **26**, 5017.
7. J. Kriz. M.J. Benet et J. Peska, Tetrahedron Lett. (1965) 2881.
8. H. Klein et A. Steinmetz, Tetrahedron Lett. (1975) 4249.
9. G. Hilgers et N.D. Scharf, Tetrahedron Lett. (1984) 1765.
10. F.J. Sardina, A. Mourino et L. Castedo, J. Org. Chem. (1986) **51**, 1264.
11. (a) B.M. Trost, T.N. Salzmann et K. Hiroi, J. Am. Chem. Soc. (1976) **98**, 4887; (b) H.J. Reich, I.L. Reich et J.M. Renga, J. Am. Chem. Soc. (1973) **95**, 5813.
12. H.J. Reich, J.M. Renga et I.L. Reich, J. Org. Chem. (1976) **39**, 2133.
13. R.A. Gibbs et W.H. Okamura, Tetrahedron Lett. (1987) **28**, 6021.
14. (a) G.H. Posner, Org. React. (1972) **19**, 1; (b) B.H. Lipshutz, R.S. Wilhelm et J.A. Kozlowski, Tetrahedron (1984) **40**, 5005; B.H. Lipshutz, Synthesis (1987) 325.

15. (a) W. Nagata, M. Yoshioka et S. Hirai, J. Am. Chem. Soc. (1972) **94**, 4635; (b) M. Samson et M. Vandewalle, Synthetic Communications (1978) **8**, 231.
16. E. Hatzigrigoriou, M.C. Roux-Schmitt, L. Wartski et J. Seyden-Penne, Tetrahedron (1988) **44**, 4457.
17. B.H. Lipshutz, R.S. Wilhelm, J.A. Kozlowski, J. Org. Chem. (1984) **49**, 3938.
18. L. Castedo, A. Mourino et L.A. Sanandeses, Tetrahedron Lett. (1986) **27**, 1523 et L. Castedo, J.L. Mascarenas, A. Mourino et L.A. Sanandeses, Tetrahedron Lett. (1988) **29**, 1203
19. L. Castedo, J.L. Mascarenas et A. Mourino, Tetrahedron Lett. (1987) **28**, 2099.
20. R. Bouillon, E. Van Herck, I. Jans, B. Keng Tan, H. Van Baelen and P. de Moor, Clin. Chem., 30, 1731, 1984.
21 J. Steroid Biochem Vol. 29, N°4, pp. 381-386, 1988.
22. Archives of Biochemistry and Biophysics Vol. 217, N°1, August, pp. 257-263, 1982.
23. The Journal of Biological Chemistry Vol. 253, N°12 Issue of June, pp. 4426-4431, 1978

## Revendications

### Revendications pour les Etats contractants suivants: AT, BE, CH, DE, FR, GB, GR, IT, LI, LU, NL, SE

**1.** Dérivés de la vitamine D répondant à la formule I suivante

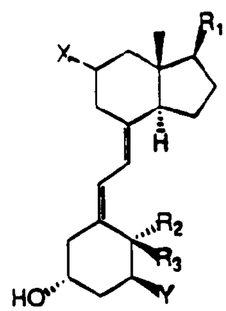

I

dans laquelle

$R_1$ représente un groupe alkylé substitué, de 1 à 15 atomes de carbone, notamment les chaînes latérales des vitamines $D_2$ (C-20 à C-28) ou $D_3$ (C-20 à C-27), ou ces mêmes chaînes partiellement modifiées, notamment

- hydroxylées en une ou plusieurs positions, par exemple les positions 24, 25 et/ou 26, et/ou
- méthylées ou éthylées en une ou plusieurs positions, par exemple les positions 24, 26 et/ou 27, et/ou
- halogènées ou poly-halogènées en une ou plusieurs positions, par exemple perfluorées (trifluorométhyl) en positions 26 et 27, ou difluorées en position 24, et/ou
- additionnées de un ou plusieurs atomes de carbone, notamment un atome C-24′ entre les positions 24 et 25, avec les mêmes possibilités de substitution que mentionnées ci-dessus, et/ou
- esterifiées sur une ou plusieurs fonctions hydroxyles mentionnées ci-dessus, et/ou
- éthérifiées sur une ou plusieurs fonctions hydroxyles mentionnées ci-dessus, et/ou
- en remplaçant un ou plusieurs atomes de carbone par un atome d'oxygène, d'azote ou de souffre, par exemple un oxygène en positions 22, 23 ou 24, et/ou
- cyclisées entre les carbones C-26 et C-27 par une liaison directe (cyclopropane) ou par l'intermédiaire d'un chaîne de 1 à 3 atomes de carbone ; chaun de ces atomes pouvant porter toutes les fonctions ou modifications décrites ci-dessus, et/ou
- substituées en une ou plusieurs positions par un cycle saturé, insaturé, aromatique ou hétéroaromatique pouvant porter toutes les fonctions et modifications décrites ci-dessus, et/ou
- insaturées, avec une ou plusieurs double(s) ou triple(s) liaisons carbone-carbone ; ces groupes alkylés insaturés pouvant porter toutes les fonctions et modifications décrites ci-dessus, et
- les formes isomériques des différents groupes situés sur la chaîne.

Y représente H ou OH ou les groupes dérivés de ce dernier tels que ester et éther ;

X représente

- une chaîne alkylée, notamment de 1 à 6 atomes de carbones, éventuellement substituée à différents endroits par un ou des groupe(s) fonctionnel(s) Z qui peuvent être notamment un halogène (tel que le fluor), un groupe hydroxyle, formyle, carboxyle, amine, thiol, cyano, nitro, sulfoxyde, sulfone, phosphono ou encore des groupes dérivés de ces derniers tels que éther, ester, acétal, amide, hydrazine, phosphate, bisphosphate, ou
- une chaîne alkylée non saturée ayant une ou des double(s) ou triple(s) liaisons carbone-carbone, ces chaînes pouvant en plus porter des groupes fonctionnels Z mentionnés ci-dessus, ou
- un noyau aromatique ou hétéroaromatique éventuellement substitué de halogènes, un (ou des) groupe hydroxyle, amine, formyle, carboxyle, thiol, cyano, nitro ou encore des groupes dérivés de ces derniers tels que éther, ester, acétal, amide, ou
- un halogène, un groupe cyano, sulfoxyde, sulfone, hydroxylz, thiol, amine ou encore des dérivés de ces derniers tels que éther, ester, amine et hydrazine ;

$R_2$ représente un groupe méthyl et $R_3$ un H ou, $R_2$ est H et $R_3$ est méthyl ou, $R_2$ et $R_3$ sont H, ou encore $R_2$ et $R_3$ ensemble représentent un groupe méthylène = $CH_2$.

**2.** Dérivés selon la revendication 1, caractérisés en ce que $R_1$ représente l'une des chaînes suivantes

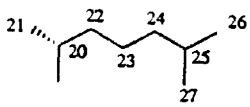

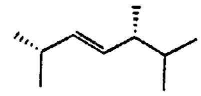

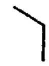

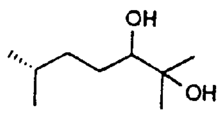

24 R ou S

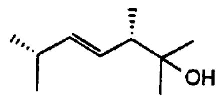

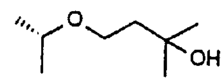

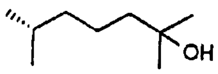

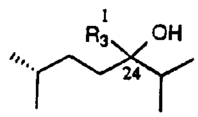

24 R ou S
$R_3^1$ = H ou $CH_3$

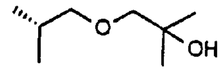

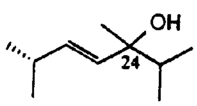

24 R ou S

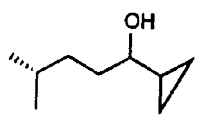

24 R ou S

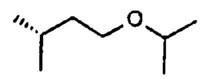

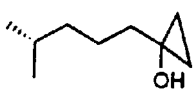

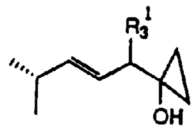

$R_3^1$ = H ou $CH_3$

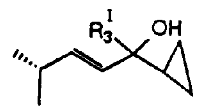

24 R ou S
$R_3^1$ = H ou $CH_3$

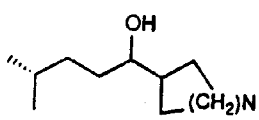

24 R ou S
n = 1,2,3

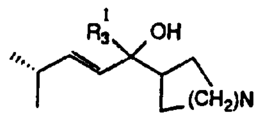

24 R ou S
n = 1,2 3
$R_3$ = H ou $CH_3$

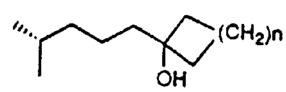

n = 1,2,3

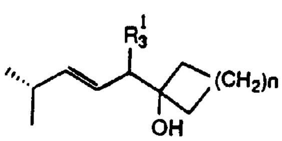

n = 1,2,3
$R_3^1$ = H ou $CH_3$ ( R ou S )

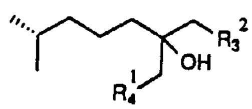

$R_3^2$ = H , $R_4^1$ = $CH_3$ ( R ou S )
$R_3^2$ = $R_4^1$ = $CH_3$

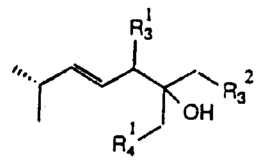

$R_3^2$ = H , $R_4^1$ = $CH_3$ ( R ou S )
$R_3^2$ = $R_4^1$ = $CH_3$
$R_3^1$ = H ou $CH_3$ ( R ou S )

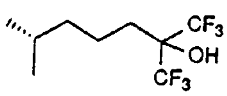

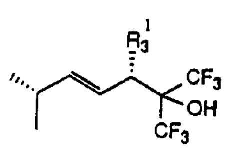

R'3 = H ou $CH_3$ ( R,S )

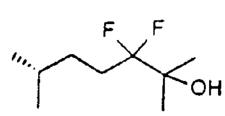

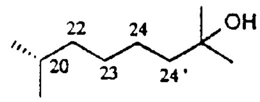

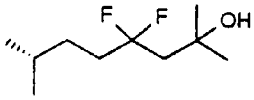

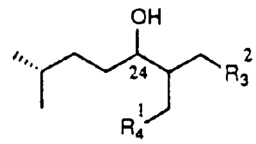

$R_3^2 = H$, $R_4^1 = CH_3$ (R ou S)
24 R ou S

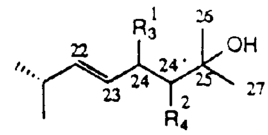

$R_3^1 = R_4^2 = H$

$R_3^1 = CH_3$ (R ou S), $R_4^2 = H$

$R_3^1 = H$, $R_4^2 = CH_3$ (R ou S)

3. Dérivés de la vitamine $D_3$ répondant à la formule I'

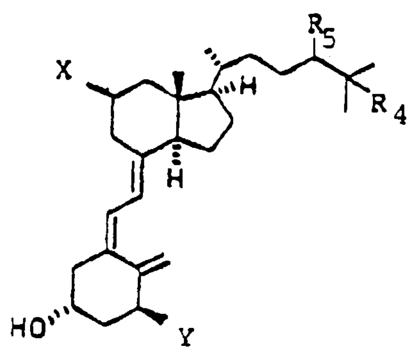

I'

dans laquelle

Y, $R_4$ et $R_5$ représentent H ou OH et

X représente une chaîne alkylée, notamment de 1 à 6 atomes de carbone éventuellement substituée notamment à son extrémité terminale par un groupe fonctionnel Z qui peut être choisi notamment parmi les groupes hydroxyle, formyle, carboxyle, amine, amide, et les fonctions dérivées comme les fonctions ester, éther, acétal, X étant en isomérie $\alpha$ ou $\beta$.

4. Dérivés selon la revendication 3, caractérisés en ce que Y, $R_4$ et $R_5$) est choisi parmi (H, OH, H), (OH, OH, H), (H, H, OH), (H, OH, OH), (OH, H, OH), (OH, H, H).

5. Dérivés selon l'une des revendications précédentes, caractérisés en ce que X représente le (2-Z) éthyl, un méthyl ou un phényl.

6. Dérivés selon l'une des revendications précédentes caractérisés en ce que Z représente le groupe

$$-O-\underset{\underset{O}{\|}}{C}-(CH_2)_n-COOH$$

avec n compris entre 0 et 10 ou le groupe OH.

7. Procédé de préparation d'un dérivé selon l'une des revendications précédentes, caractérisé en ce qu'on fait réagir un dérivé de formule II (II$\alpha$ ou II$\beta$)

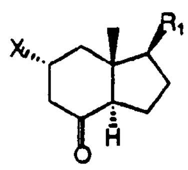

II α

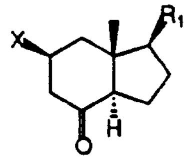

II β

dans lesquelles X et $R_1$ ont les significations données précédemment, ceux-ci étant toutefois protégés si nécessaire sur leur groupe fonctionnel, avec un dérivé de formule III

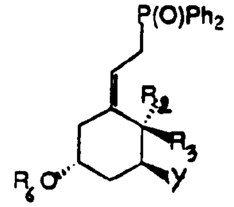

III

$R_2$ et $R_3$ = $CH_2$
$R_2$ = Me, $R_3$ = H
$R_2$ = H, $R_3$ = Me
Y = H ouOR'

dans lesquelles Y, $R_2$ et $R_3$ ont les significations données précédemment, le cas échéant étant un groupe hydroxyl protégé notamment par un groupe protecteur des groupes hydroxyl tel que diméthyl-t-butylsilyl ; $R_6$ est un groupe protecteur de la fonction hydroxyle.
Cette réaction du composé de formule II avec le composé de formule III se réalise en présence de n-butyllithium,
puis on déprotège les fonctions protégées.

**8.** Procédé de préparation d'un dérivé selon la revendication précédente, caractérisé en ce qu'on fait réagir un dérivé de formule II' (II'α ou II'β)

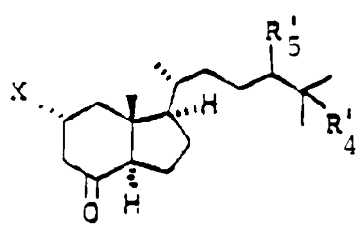

(II'α)

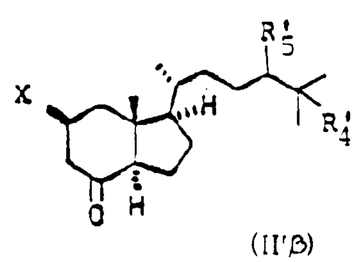

(II'β)

la fonction terminale de X étant protégée par un groupe protecteur avec un dérivé de formule III'

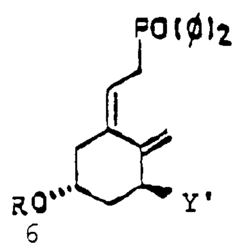

III'

dans lesquelles Y', $R'_4$ et $R'_5$ représentent H ou un groupe hydroxyle protégé, notamment par diméthyl-t-butylsilyl, et $R_6$ représente un groupe protecteur d'hydroxyle.

**9.** Procédé de préparation d'un dérivé de formule I selon la revendication 8, caractérisé en ce que l'on prépare le dérivé avec Z qui représente une fonction hydroxyle éventuellement protégée, que l'on transforme

ensuite éventuellement en un autre groupe fonctionnel.

**10.** A titre de médicaments des dérivés selon l'une des revendications 1 à 6.

**11.** Antigène, caractérisé en ce qu'il est préparé par liaison covalente d'un dérivé de vitamine D de formule I selon l'une des revendications précédentes, avec une protéine porteuse immunogénique, via une dérivation en $C_{11}$ dudit dérivé.

**12.** Anticorps qui est induit par inoculation chez un animal hôte de l'antigène selon l'une des revendications 1 à 11.

**13.** Traceur utile pour des dosages, caractérisé en ce qu'il consiste dans la conjugaison d'un dérivé de formule I selon l'une des revendications 1 à 12, avec un élément de marquage, notamment une enzyme ou une molécule iodée.

**14.** Traceur selon la revendication 13, caractérisé en ce qu'il consiste dans la 11a [2-O-($m^{125}I$-tyramidosuccinoyl)-éthyl]25 hydroxy vitamine $D_3$ (58).

**15.** Méthode de dosages de métabolites de la vitamine D, caractérisée en ce qu'elle comporte l'utilisation comme réactif des traceurs selon les revendications 13 et 14 ou des anticorps selon la revendication 12.

**16.** Composé utile notamment pour la synthèse d'un dérivé de la vitamine D selon l'une des revendications 1 à 6, caractérisé en ce qu'il répond à la formule générale

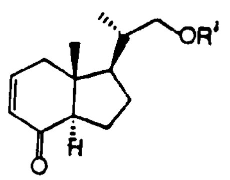

où OR′ représente OH ou les groupes dérivés de ce dernier tels que ester et éther,

**Revendications pour l'Etat contractant suivant: ES**

**1.** Procédé de préparation d'un dérivé de la vitamine D répondant à la formule I suivante

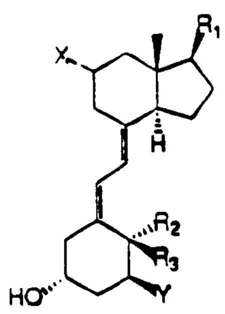

I

dans laquelle

$R_1$ représente un groupe alkylé substitué, de 1 à 15 atomes de carbone, notamment les chaînes latérales des vitamines $D_2$ (C-20 à C-28) ou $D_3$ (C-20 à C-27), ou ces mêmes chaînes partiellement modifiées, notamment

- hydroxylées en une ou plusieurs positions, par exemple les positions 24, 25 et/ou 26, et/ou
- méthylées ou éthylées en une ou plusieurs positions, par exemple les positions 24, 26 et/ou 27, et/ou
- halogènées ou poly-halogènées en une ou plusieurs positions, par exemple perfluorées (trifluorométhyl) en positions 26 et 27, ou difluorées en position 24, et/ou
- additionnées de un ou plusieurs atomes de carbone, notamment un atome C-24′ entre les positions

24 et 25, avec les mêmes possibilités de substitution que mentionnées ci-dessus, et/ou

- esterifiées sur une ou plusieurs fonctions hydroxyles mentionnées ci-dessus, et/ou
- éthérifiées sur une ou plusieurs fonctions hydroxyles mentionnées ci-dessus, et/ou
- en remplaçant un ou plusieurs atomes de carbone par un atome d'oxygène, d'azote ou de souffre, par exemple un oxygène en positions 22, 23 ou 24, et/ou
- cyclisées entre les carbones C-26 et C-27 par une liaison directe (cyclopropane) ou par l'intermédiaire d'un chaîne de 1 à 3 atomes de carbone ; chaun de ces atomes pouvant porter toutes les fonctions ou modifications décrites ci-dessus, et/ou
- substituées en une ou plusieurs positions par un cycle saturé, insaturé, aromatique ou hétéroaromatique pouvant porter toutes les fonctions et modifications décrites ci-dessus, et/ou
- insaturées, avec une ou plusieurs double(s) ou triple(s) liaisons carbone-carbone ; ces groupes alkylés insaturés pouvant porter toutes les fonctions et modifications décrites ci-dessus, et
- les formes isomériques des différents groupes situés sur la chaîne.

Y représente H ou OH ou les groupes dérivés de ce dernier tels que ester et éther ;

X représente

- une chaîne alkylée, notamment de 1 à 6 atomes de carbones, éventuellement substituée à différents endroits par un ou des groupe(s) fonctionnel(s) Z qui peuvent être notamment un halogène (tel que le fluor), un groupe hydroxyle, formyle, carboxyle, amine, thiol, cyano, nitro, sulfoxyde, sulfone, phosphono ou encore des groupes dérivés de ces derniers tels que éther, ester, acétal, amide, hydrazine, phosphate, bisphosphate, ou
- une chaîne alkylée non saturée ayant une ou des double(s) ou triple(s) liaisons carbone-carbone, ces chaînes pouvant en plus porter des groupes fonctionnels Z mentionnés ci-dessus, ou
- un noyau aromatique ou hétéroaromatique éventuellement substitué de halogènes, un (ou des) groupe hydroxyle, amine, formyle, carboxyle, thiol, cyano, nitro ou encore des groupes dérivés de ces derniers tels que éther, ester, acétal, amide, ou
- un halogène, un groupe cyano, sulfoxyde, sulfone, hydroxylz, thiol, amine ou encore des dérivés de ces derniers tels que éther, ester, amine et hydrazine ;

$R_2$ représente un groupe méthyl et $R_3$ un H ou, $R_2$ est H et $R_3$ est méthyl ou, $R_2$ et $R_3$ sont H, ou encore $R_2$ et $R_3$ ensemble représentent un groupe méthylène : $CH_2$,

caractérisé en ce qu'on fait réagir un dérivé de formule II (IIalpha ou IIbêta)

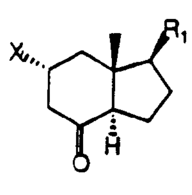

II α

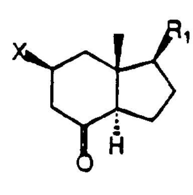

II β

dans lesquelles X et $R_1$ ont les significations données précédemment, ceux-ci étant toutefois protégés si nécessaire sur leur groupe fonctionnel, avec un dérivé de formule III

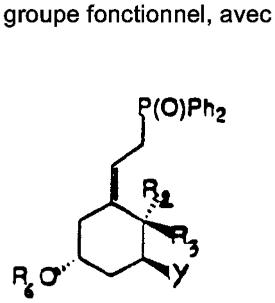

III

$R_2$ et $R_3$ = $CH_2$
$R_2$ = Me, $R_3$ = H
$R_2$ = H, $R_3$ = Me
Y = H ou OR'

dans lesquelles Y, $R_2$ et $R_3$ ont les significations données précédemment, le cas échéant étant un groupe hydroxyl protégé notamment par un groupe protecteur des groupes hydroxyl tel que diméthyl-t-butylsilyl ; $R_6$ est un groupe protecteur de la fonction hydroxyle.

Cette réaction du composé de formule II avec le composé de formule III se réalise en présence de n-butyllithium,

puis on déprotège les fonctions protégées.

2. Procédé de préparation d'un dérivé selon la revendication précédente, caractérisé en ce qu'on fait réagir un dérivé de formule II' (II'alpha ou II'bêta)

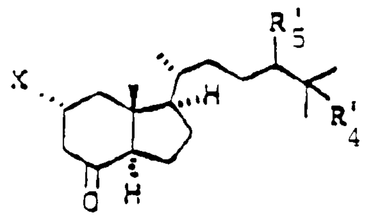

(II'α)

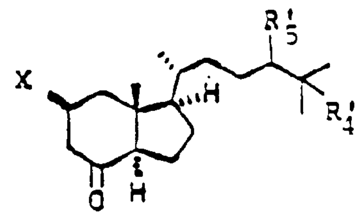

(II'β)

la fonction terminale de X étant protégée par un groupe protecteur avec un dérivé de formule III'

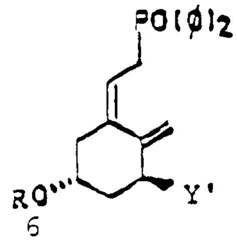

III'

dans lesquelles Y', $R'_4$ et $R'_5$ représentent H ou un groupe hydroxyle protégé, notamment par diméthyl-t-butylsilyl, et $R_6$ représente un groupe protecteur d'hydroxyle.

3. Procédé de préparation d'un dérivé de formule I selon la revendication 2, caractérisé en ce que l'on prépare le dérivé avec Z qui représente une fonction hydroxyle éventuellement protégée, que l'on transforme ensuite éventuellement en un autre groupe fonctionnel.

4. Procédé selon l'une des revendications 1 à 3, caractérisé en ce que $R_1$ représente l'une des chaînes suivantes

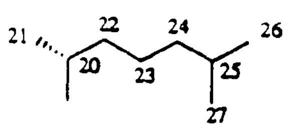

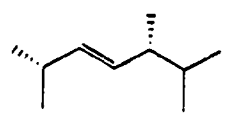

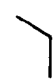

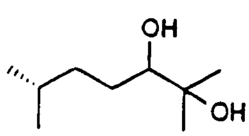

24 R ou S

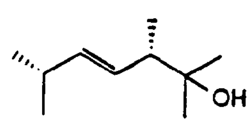

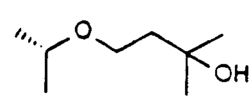

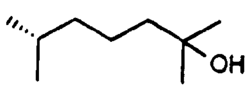

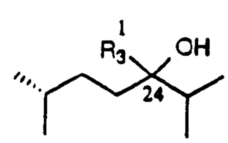

24 R ou S
$R'_3$ = H ou $CH_3$

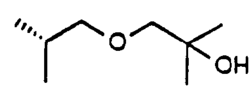

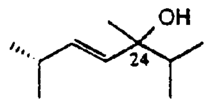

24 R ou S

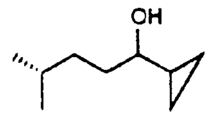

24 R ou S

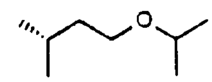

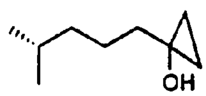

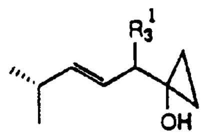

$R_3^1$ = H ou $CH_3$

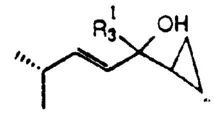

24 R ou S
$R_3^1$ = H ou $CH_3$

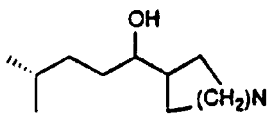

24 R ou S
n = 1,2,3

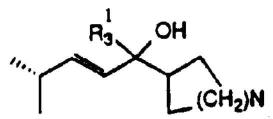

24 R ou S
n = 1,2 3
$R_3$ = H ou $CH_3$

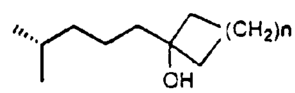

n = 1,2,3

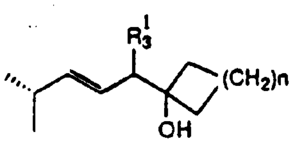

n = 1,2,3
$R_3^1$ = H ou $CH_3$ ( R ou S )

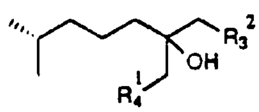

$R_3^2$ = H , $R_4^1$ = $CH_3$ ( R ou S )
$R_3^2$ = $R_4^1$ = $CH_3$

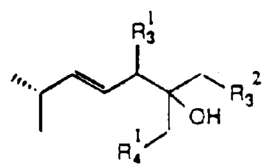

$R_3^2$ = H , $R_4^1$ = $CH_3$ ( R ou S )
$R_3^2$ = $R_4^1$ = $CH_3$
$R_3^1$ = H ou $CH_3$ ( R ou S )

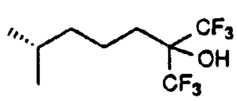

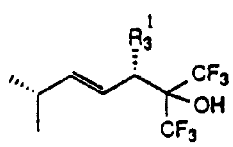

$R'_3$ = H ou $CH_3$ ( R,S )

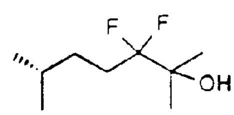

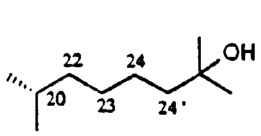

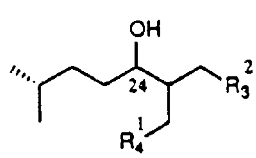

$R_3^2$ = H , $R_4^1$ = $CH_3$ ( R ou S )
24 R ou S

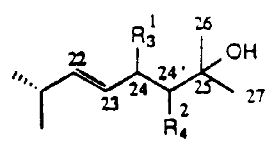

$R_3^1$ = $R_4^2$ = H
$R_3^1$ = $CH_3$ ( R ou S ) , $R_4^2$ = H
$R_3^1$ = H , $R_4^2$ = $CH_3$ (R ou S )

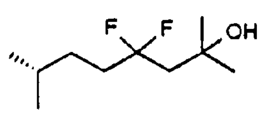

5. Procédé selon l'une des revendications 1 à 4 caractérisé en ce que le dérivé de la vitamine $D_3$ répond à la formule

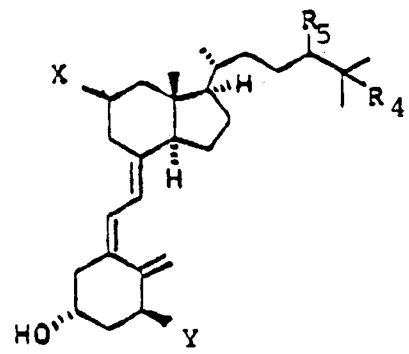

I'

dans laquelle

Y, $R_4$ et $R_5$ représentent H ou OH et

X représente une chaîne alkylée, notamment de 1 à 6 atomes de carbone éventuellement substituée notamment à son extrémité terminale par un groupe fonctionnel Z qui peut être choisi notamment parmi les groupes hydroxyle, formyle, carboxyle, amine, amide, et les fonctions dérivées comme les fonctions ester, éther, acétal, X étant en isomérie alpha ou bêta.

**6.** Procédé selon la revendication 5, caractérisé en ce que Y, $R_4$ et $R_5$) est choisi parmi (H, OH, H), (OH, OH, H), (H, H, OH), (H, OH, OH), (OH, H, OH), (OH, H, H).

**7.** Procédé selon l'une des revendications précédentes, caractérisé en ce que X représente le (2-Z) éthyl, un méthyl ou un phényl.

**8.** Procédé selon l'une des revendications précédentes, caractérisé en ce que Z représente le groupe

$$-O-\underset{\overset{\|}{O}}{C}-(CH_2)_n-COOH$$

avec n compris entre 0 et 10 ou le groupe OH.

**9.** Méthode de dosages de métabolites de la vitamine D,caractérisée en ce qu'elle comporte l'utilisation comme réactif d'un traceurqui consiste dans la conjugaison d'un dérivé de formule I des revendications 1 à 12, avec un élément de marquage, notamment une enzyme ou une molécule iodée ou d'anticorps induits par inoculation chez un animal hôte d'un antigène préparé par liaison covalente d'un dérivé de vitamine D de formule I selon l'une des revendications précédentes, avec une protéine porteuse immunogénique, via une dérivation en $C_{11}$ dudit dérivé.

**10.** Méthode selon la revendication 9 caractérisée en ce que le traceur consiste dans la 11a [2-O-(m$^{125}$I-tyramidosuccinoyl)-éthyl]25 hydroxy vitamine $D_3$ (58).

## Patentansprüche

### Patentansprüche für folgende Vertragsstaaten: AT, BE, CH, DE, FR, GB, GR, IT, LI, LU, NL, SE

**1.** Derivate von Vitamin D, die der folgenden Formel I entsprechen

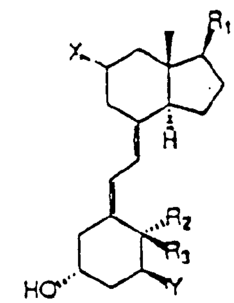

(I)

in der

$R_1$ eine substituierte Alkyl-Gruppe mit 1 bis 15 Kohlenstoffatomen, insbesondere die Seitenketten des Vitamins $D_2$ (C-20 bis C-28) oder des Vitamins $D_3$ (C-20 bis C-27), oder diese gleichen Ketten teilweise modifiziert darstellt, insbesondere

- hydroxyliert in einer oder in mehreren Positionen, beispielsweise in den Positionen 24, 25 und/oder 26, und/oder
- methyliert oder ethyliert in einer oder in mehreren Positionen, beispielsweise in den Positionen 24, 26 und/oder 27 und/oder
- halogeniert oder polyhalogeniert in einer oder in mehreren Positionen, beispielsweise perfluoriert (Trifluormethyl) in den Positionen 26 und 27 oder difluoriert in Position 24, und/oder
- Einfügung von einem oder mehreren Kohlenstoffatomen, insbesondere ein Atom C24′ zwischen den Positionen 24 und 25, mit den gleichen Möglichkeiten der Substitution,, wie zuvor erwähnt, und/oder
- Veresterung von einer oder mehreren der zuvor erwähnten Hydroxyl-Funktionen, und/oder
- Veretherung von einer oder mehreren der zuvor erwähnten Hydroxyl-Funktionen, und/oder
- Ersetzen von einem oder mehreren Kohlenstoffatomen durch ein Sauerstoffatom, Stickstoffatom oder Schwefelatom, beispielsweise ein Sauerstoff in Position 22, 23 oder 24, und/oder
- Cyclisierung zwischen den Kohlenstoffen C-26 und C-27 durch eine direkte Bindung (Cyclopropan) oder durch Zwischensetzung einer Kette mit 1 bis 3 Kohlenstoffatomen, wobei jedes dieser Atome alle zuvor beschriebenen Funktionen oder Modifikationen tragen kann, und/oder
- substituiert in einer oder in mehreren Positionen durch einen gesättigten, ungesättigten, aromatischen oder heteroaromatischen Ring, der alle zuvor beschriebenen Funktionen oder Modifikationen tragen kann, und/oder
- ungesättigt, mit einer oder mehreren Kohlenstoff-Kohlenstoff-Doppel- oder -Dreifachbindungen, wobei diese ungesättigten Alkyl-Gruppen alle zuvor beschriebenen Funktionen oder Modifikationen tragen können, und
- die isomeren Formen der verschiedenen in der Kette befindlichen Gruppen;

Y H oder OH oder die von diesen abgeleiteten Gruppen darstellt, wie Ester oder Ether,

X - eine Alkyl-Kette, insbesondere mit 1 bis 6 Kohlenstoffatomen darstellt, gegebenenfalls substituiert an verschiedenen Stellen durch eine oder mehrere Funktions-Gruppen Z, die insbesondere ein Halogen (wie Fluor), eine Hydroxyl-, Formyl-, Carboxyl-, Amin-, Thiol-, Cyano-, Nitro-, Sulfoxid-, Sulfon-, Phosphono- oder auch eine von diesen abgeleitete Gruppe sein können, wie Ether, Ester, Acetal, Amid, Hydrazin, Phosphat, Biphosphat, oder

- eine ungesättigte Alkyl-Kette mit einer oder mehreren Kohlenstoff-Kohlenstoff-Doppel- oder -Dreifachbindungen bedeutet, wobei diese Ketten außerdem die zuvor erwähnten Funktions-Gruppen Z tragen können,
- einen aromatischen oder heteroaromatischen Kern darstellt, gegebenenfalls substituiert durch Halogene, ein oder mehrere Hydroxyl-, Amin-, Formyl-, Carboxyl-, Thiol-, Cyano-, Nitro- oder von diesen abgeleitete Gruppen, wie Ether, Ester, Acetal, Amid, oder
- ein Halogen, eine Cyano-, Sulfoxid-, Sulfon-, Hydroxyl-, Thiol- oder Amin-Gruppe oder von diesen abgeleitete Gruppen bedeutet, wie Ether, Ester, Amin und Hydrazin;

$R_2$ eine Methyl-Gruppe ist und $R_3$ Wasserstoff bedeutet, oder $R_2$ Wasserstoff ist und $R_3$ eine Methyl-Gruppe bedeutet, oder $R_2$ und $R_3$ Wasserstoff sind, oder $R_2$ und $R_3$ zusammen eine Methylen-Gruppe $=CH_2$ sind.

**2.** Derivate nach Anspruch 1, dadurch gekennzeichnet, daß $R_1$ eine der folgenden Ketten darstellt:

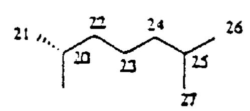

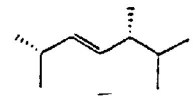

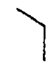

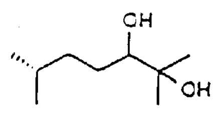

24 R o. S

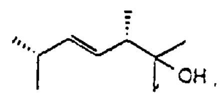

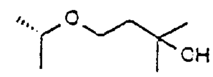

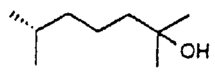

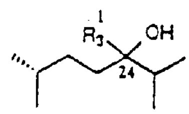

24 R o. S
$R_3^1$ = H o. $CH_3$

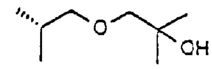

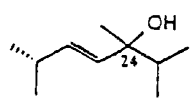

24 R o. S

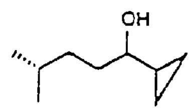

24 R o. S

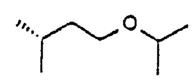

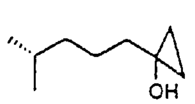

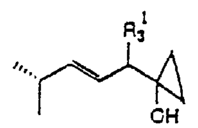

$R_3^1$ = H o. $CH_3$

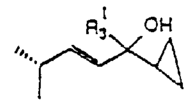

24 R o. S
$R_3^1$ = H o. $CH_3$

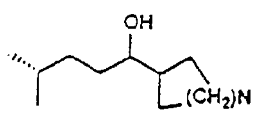

24 R o. S
n = 1,2,3

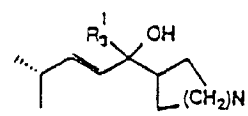

24 R o. S
n = 1,2 3
$R_3$ = H o. $CH_3$

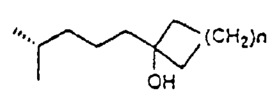

n = 1,2,3

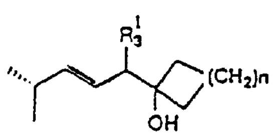

n = 1,2,3
$R_3^1$ = H o. $CH_3$ ( R o. S )

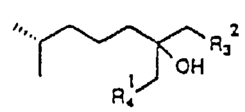

$R_3^2$ = H, $R_4^1$ = $CH_3$ ( R o. S )
$R_3^2$ = $R_4^1$ = $CH_3$

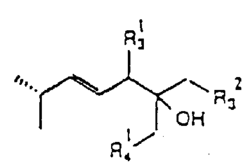

$R_3^2$ = H, $R_4^1$ = $CH_3$ ( R o. S )
$R_3^2$ = $R_4^1$ = $CH_3$
$R_3^1$ = H o. $CH_3$ ( R o. S )

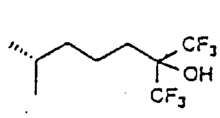

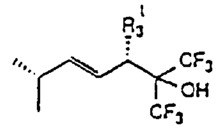

$R'_3$ = H o. $CH_3$ ( R,S )

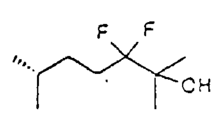

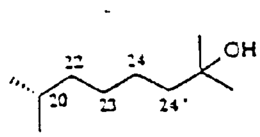

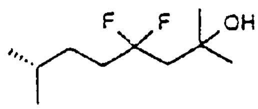

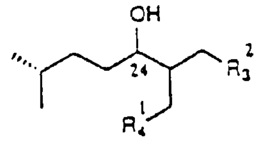

$R_3^2 = H$, $R_4^1 = CH_3$ (R o. S)
24 R o. S

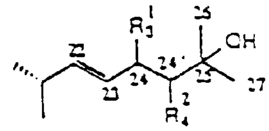

$R_3^1 = R_4^2 = H$
$R_3^1 = CH_3$ (R o. S), $R_4^2 = H$
$R_3^1 = H$, $R_4^2 = CH_3$ (R o. S)

3. Derivate von Vitamin $D_3$, die der Formel I′ entsprechen

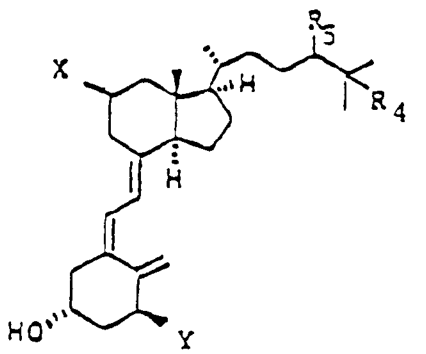

I′

in der
Y, $R_4$ und $R_5$ H oder OH darstellen, und
X eine Alkyl-Kette, insbesondere mit 1 bis 6 Kohlenstoffatomen darstellt, gegebenenfalls substituiert, insbesondere an ihrem Ende, durch eine Funktions-Gruppe Z, insbesondere ausgewählt unter den Hydroxyl-, Formyl-, Carboxyl-, Amin- oder Amid-Gruppen und den Funktions-Derivaten, wie den Ester-, Ether- und Acetal-Funktionen, wobei X in α- oder β-Isomerie vorliegen kann.

4. Derivate nach Anspruch 3, dadurch gekennzeichnet, daß Y, $R_4$ und $R_5$ ausgewählt werden unter (H, OH, H), (OH, OH, H), (H, H, OH), (H, OH, OH), (OH, H, OH) und (OH, H, H).

5. Derivate nach einem der vorstehenden Ansprüche, dadurch gekennzeichnet, daß X das (2-Z)-ethyl, ein Methyl oder ein Phenyl darstellt.

6. Derivate nach einem der vorstehenden Ansprüche, dadurch gekennzeichnet, daß Z die Gruppe

$$-O-\underset{\underset{O}{\|}}{C}-(CH_2)_n-COOH$$

mit n zwischen 0 und 10 oder die Gruppe OH ist.

7. Verfahren zur Herstellung eines Derivates nach einem der vorstehenden Ansprüche, dadurch gekennzeichnet, daß man ein Derivat der Formel II (IIα oder IIβ)

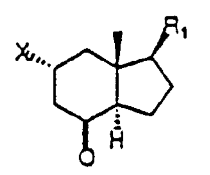

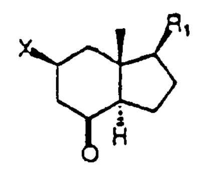

II α　　　　II β

in denen X und $R_1$ die vorstehend angegebenen Bedeutungen besitzen, und die auch, wenn erforderlich, an diesen Funktions-Gruppen geschützt sein können, mit einem Derivat der Formel III

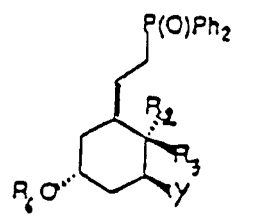

III

$R_2$ und $R_3$ = $CH_2$
$R_2$ = Me, $R_3$ = H
$R_2$ = H, $R_3$ = Me
Y = H oder OR

zur Reaktion bringt, in der Y, $R_2$ und $R_3$ die vorstehend angegebenen Bedeutungen besitzen, wobei gegebenenfalls eine Hydroxyl-Gruppe geschützt sein kann, insbesondere durch eine Schutz-Gruppe für Hydroxyl-Gruppen, wie Dimethyl-t-butylsilyl und $R_6$ eine Schutz-Gruppe für die Hydroxyl-Funktion darstellt, und man die Reaktion der Verbindung der Formel II mit der Verbindung der Formel III in Anwesenheit von Butyl-Lithium durchführt sowie anschließend die Schutz-Funktionen wieder entfernt.

8. Verfahren zur Herstellung eines Derivates nach dem vorstehenden Anspruch, dadurch gekennzeichnet, daß man ein Derivat der Formel II' (II'α oder II'β)

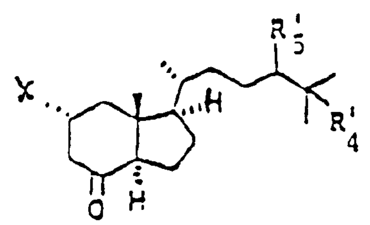

(II'α)

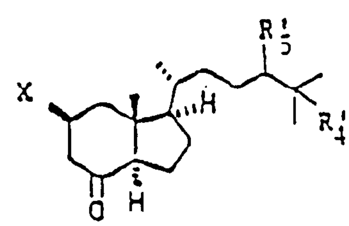

(II'β)

worin die Endfunktion von X durch eine Schutz-Gruppe geschützt ist, mit einem Derivat der Formel III'

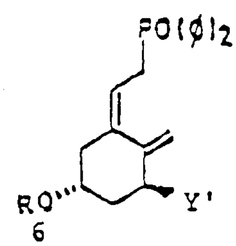

III'

zur Reaktion bringt, in der Y', $R'_4$, und $R'_5$ Wasserstoff oder eine geschützte Hydroxyl-Gruppe, insbesondere durch Dimethyl-t-butylsilyl, darstellen und $R_6$ eine Schutz-Gruppe für die Hydroxyl-Funktion bedeutet.

9. Verfahren zur Herstellung eines Derivates der Formel I nach Anspruch 8, dadurch gekennzeichnet, daß man das Derivat mit Z als gegebenenfalls geschützter Hydroxyl-Funktion herstellt und anschließend diese

gegebenenfalls in eine andere Funktions-Gruppe umwandelt.

**10.** Als Medikamente die Derivate nach einem der Ansprüche 1 bis 6.

**11.** Antigene, dadurch gekennzeichnet, daß sie durch kovalente Bindung eines Derivates von Vitamin D der Formel I nach einem der vorstehenden Ansprüche mit einem Immunträger-Protein über eine Ableitung in $C_{11}$ des genannten Derivates hergestellt werden.

**12.** Antikörper, der durch Inokulation des Antigens nach einem der Ansprüche 1 bis 11 in einen Tierwirt induziert wird.

**13.** Tracer für Bestimmungen, dadurch gekennzeichnet, daß er aus der Verbindung eines Derivates der Formel I nach einem der Ansprüche 1 bis 12 mit einem Markierungs-Element, insbesondere einem Enzym oder einem Iod-Molekül, besteht.

**14.** Tracer nach Anspruch 13, dadurch gekennzeichnet, daß er aus 11a-[2-0-(m$^{125}$I-Tyramidosuccinoyl)-ethyl-]-25-hydroxy-Vitamin $D_3$ (58) besteht.

**15.** Methode zur Bestimmung von Metaboliten des Vitamins D, dadurch gekennzeichnet, daß sie die Verwendung der Tracer nach Anspruch 13 und 14 und der Antikörper nach Anspruch 12 als Reagens umfaßt.

**16.** Verbindung, insbesondere nützlich für die Synthese eines Derivates von Vitamin D nach einem der Ansprüche 1 bis 6, dadurch gekennzeichnet, daß sie der allgemeinen Formel

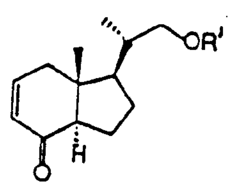

entspricht, in der OR' eine OH-Gruppe darstellt oder die von dieser abgeleiteten Gruppen, wie Ester und Ether.

**Patentansprüche für folgenden Vertragsstaat: ES**

**1.** Verfahren zur Herstellung eines Derivates von Vitamin D, das der folgenden Formel I entspricht:

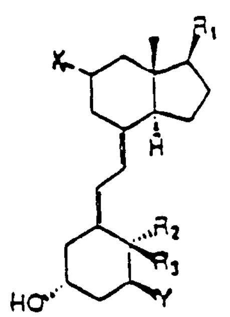

I

in der
$R_1$ eine substituierte Alkyl-Gruppe mit 1 bis 15 Kohlenstoffatomen, insbesondere die Seitenketten des Vitamins $D_2$ (C-20 bis C-28) oder des Vitamins $D_3$ (C-20 bis C-27), oder diese gleichen Ketten teilweise modifiziert darstellt, insbesondere
- hydroxyliert in einer oder in mehreren Positionen, beispielsweise in den Positionen 24, 25 und/oder 26, und/oder
- methyliert oder ethyliert in einer oder in mehreren Positionen, beispielsweise in den Positionen 24, 26 und/oder 27 und/oder
- halogeniert oder polyhalogeniert in einer oder in mehreren Positionen, beispielsweise perfluoriert (Trifluormethyl) in den Positionen 26 und 27 oder difluoriert in Position 24, und/oder

- Einfügung von einem oder mehreren Kohlenstoffatomen, insbesondere ein AtomC24′ zwischen den Positionen 24 und 25, mit den gleichen Möglichkeiten der Substitution, wie zuvor erwähnt, und/oder
- Veresterung von einer oder mehreren der zuvor erwähnten Hydroxyl-Funktionen, und/oder
- Veretherung von einer oder mehreren der zuvor erwähnten Hydroxyl-Funktionen, und/oder
- Ersetzen von einem oder mehreren Kohlenstoffatomen durch ein Sauerstoffatom, Stickstoffatom oder Schwefelatom, beispielsweise ein Sauerstoff in Position 22, 23 oder 24, und/oder
- Cyclisierung zwischen den Kohlenstoffen C-26 und C-27 durch eine direkte Bindung (Cyclopropan) oder durch Zwischensetzung einer Kette mit 1 bis 3 Kohlenstoffatomen, wobei jedes dieser Atome alle zuvor beschriebenen Funktionen oder Modifikationen tragen kann, und/oder
- substituiert in einer oder in mehreren Positionen durch einen gesättigten, ungesättigten, aromatischen oder heteroaromatischen Ring, der alle zuvor beschriebenen Funktionen oder Modifikationen tragen kann, und/oder
- ungesättigt, mit einer oder mehreren Kohlenstoff-Kohlenstoff-Doppel- oder -Dreifachbindungen, wobei diese ungesättigten Alkyl-Gruppen alle zuvor beschriebenen Funktionen oder Modifikationen tragen können, und
- die isomeren Formen der verschiedenen in der Kette befindlichen Gruppen; Y H oder OH oder die von diesen abgeleiteten Gruppen darstellt, wie Ester oder Ether,

X - eine Alkyl-Kette, insbesondere mit 1 bis 6 Kohlenstoffatomen darstellt, gegebenenfalls substituiert an verschiedenen Stellen durch eine oder mehrere Funktions-Gruppen Z, die insbesondere ein Halogen (wie Fluor), eine Hydroxyl-, Formyl-, Carboxyl-, Amin-, Thiol-, Cyano-, Nitro-, Sulfoxid-, Sulfon-, Phosphono- oder auch eine von diesen abgeleitete Gruppe sein können, wie Ether, Ester, Acetal, Amid, Hydrazin, Phosphat, Biphosphat, oder
- eine ungesättigte Alkyl-Kette mit einer oder mehreren Kohlenstoff-Kohlenstoff-Doppel oder -Dreifachbindungen bedeutet, wobei diese Ketten außerdem die zuvor erwähnten Funktions-Gruppen Z tragen können,
- einen aromatischen oder heteroaromatischen Kern darstellt, gegebenenfalls substituiert durch Halogene, ein oder mehrere Hydroxyl-, Amin-, Formyl-, Carboxyl-, Thiol-, Cyano-, Nitro- oder von diesen abgeleitete Gruppen, wie Ether, Ester, Acetal, Amid, oder
- ein Halogen, eine Cyano-, Sulfoxid-, Sulfon-, Hydroxyl-, Thiol- oder Amin-Gruppe oder von diesen abgeleitete Gruppen bedeutet, wie Ether, Ester, Amin und Hydrazin;

$R_2$ eine Methyl-Gruppe ist und $R_3$ Wasserstoff bedeutet, oder $R_2$ Wasserstoff ist und $R_3$ eine Methyl-Gruppe bedeutet, oder $R_2$ und $R_3$ Wasserstoff sind, oder $R_2$ und $R_3$ zusammen eine Methylen-Gruppe $=CH_2$ sind, dadurch gekennzeichnet, daß man ein Derivat der Formel II (IIα oder IIβ)

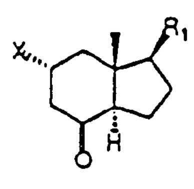

II α

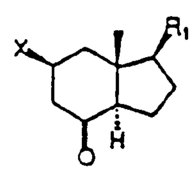

II β

in denen X und $R_1$ die vorstehend angegebenen Bedeutungen besitzen, und die auch, wenn erforderlich, an diesen Funktions-Gruppen geschützt sein können, mit einem Derivat der Formel III

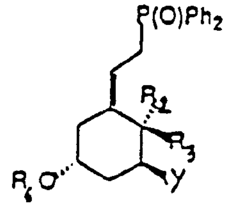

III

$R_2$ und $R_3$ = $CH_2$
$R_2$ = Me, $R_3$ = H
$R_2$ = H, $R_3$ = Me
Y = H oder OR

zur Reaktion bringt, in der Y, $R_2$ und $R_3$ die vorstehend angegebenen Bedeutungen besitzen, wobei gegebenenfalls eine Hydroxyl-Gruppe geschützt sein kann, insbesondere durch eine Schutz-Gruppe für Hydroxyl-Gruppen, wie Dimethyl-t-butylsilyl und $R_6$ eine Schutz-Gruppe für die Hydroxyl-Funktion darstellt, und man die Reaktion der Verbindung der Formel II mit der Verbindung der Formel III in Anwesenheit von Butyl-Lithium durchführt sowie anschließend die Schutz-Funktionen wieder entfernt.

**2.** Verfahren zur Herstellung eines Derivates nach dem vorstehenden Anspruch, dadurch gekennzeichnet, daß man ein Derivat der Formel II' (II'α oder II'β)

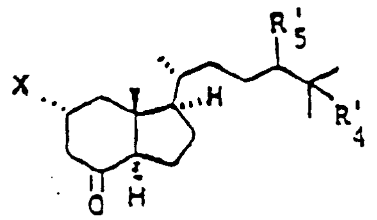

(II'α)

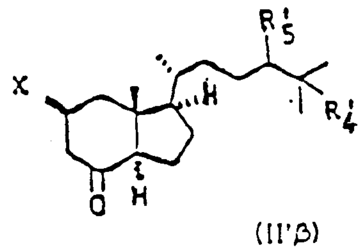

(II'β)

worin die Endfunktion von X durch eine Schutz-Gruppe geschützt ist, mit einem Derivat der Formel III'

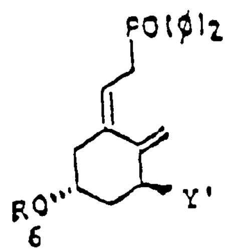

III'

zur Reaktion bringt, in der Y', $R'_4$ und $R'_5$ Wasserstoff oder eine geschützte Hydroxyl-Gruppe, insbesondere durch Dimethyl-t-butylsilyl, darstellen und $R_6$ eine Schutz-Gruppe für die Hydroxyl-Funktion bedeutet.

**3.** Verfahren zur Herstellung eines Derivates der Formel I nach Anspruch 2, dadurch gekennzeichnet, daß man das Derivat mit Z als gegebenenfalls geschützter Hydroxyl-Funktion herstellt und anschließend diese gegebenenfalls in eine andere Funktions-Gruppe umwandelt.

**4.** Verfahren nach einem der Ansprüche 1 bis 3, dadurch gekennzeichnet, daß $R_1$ eine der folgenden Ketten darstellt:

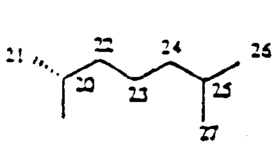

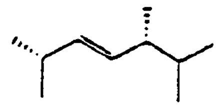

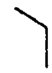

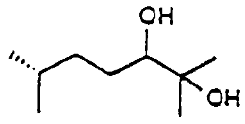

24 R o, S

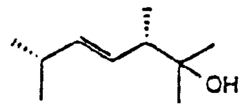

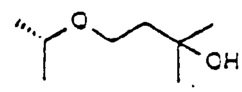

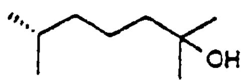

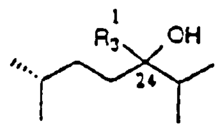

24 R o, S
$R^{1}_{3}$ = H o, $CH_3$

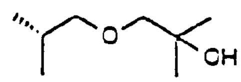

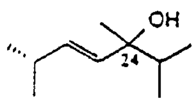

24 R o. S

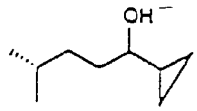

24 R o. S

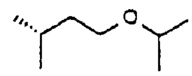

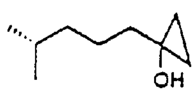

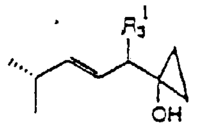

$R_3^1$ = H o. $CH_3$

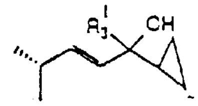

24 R o. S
$R_3^1$ = H o. $CH_3$

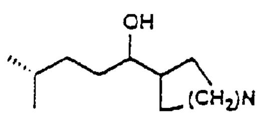

24 R o. S
n = 1,2,3

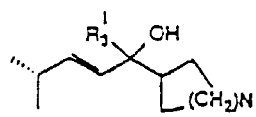

24 R o. S
n = 1,2 3
$R_3$ = H o. $CH_3$

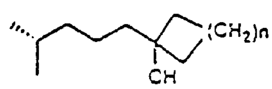

n = 1,2,3

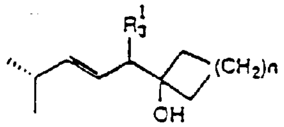

n = 1,2,3
$R_3^1$ = H o. $CH_3$ ( R o. S)

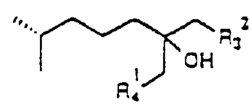

$R_3^2$ = H, $R_4^1$ = $CH_3$ (R o. S)
$R_3^2$ = $R_4^1$ = $CH_3$

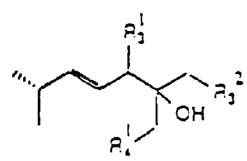

$R_3^2$ = H, $R_4^1$ = $CH_3$ (R o. S)
$R_3^2$ = $R_4^1$ = $CH_3$
$R_3^1$ = H o. $CH_3$ (R o. S)

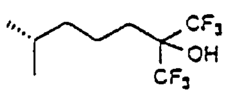

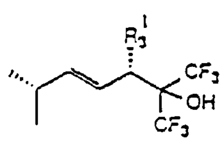

$R'_3$ = H o. $CH_3$ ( R,S)

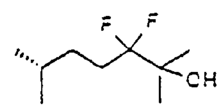

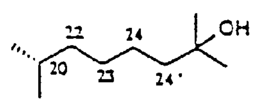

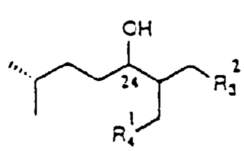

$R_3^2$ = H, $R_4^1$ = $CH_3$ (R o. S)
24 R o. S

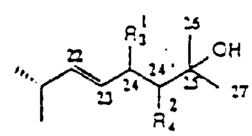

$R_3^1$ = $R_4^2$ = H
$R_3^1$ = $CH_3$ (R o. S), $R_4^2$ = H
$R_3^1$ = H, $R_4^2$ = $CH_3$ (R o. S)

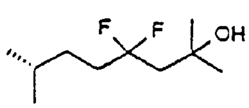

5. Verfahren nach einem der Ansprüche 1 bis 4, dadurch gekennzeichnet, daß das Derivat von Vitamin D,

der Formel I' entspricht

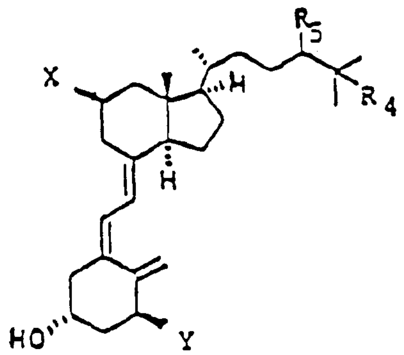

I'

in der
Y, $R_4$ und $R_5$ H oder OH darstellen, und
X eine Alkyl-Kette, insbesondere mit 1 bis 6 Kohlenstoffatomen darstellt, gegebenenfalls substituiert, insbesondere an ihrem Ende, durch eine Funktions-Gruppe Z, insbesondere ausgewählt unter den Hydroxyl-, Formyl-, Carboxyl-, Amin- oder Amid-Gruppen und den Funktions-Derivaten, wie den Ester-, Ether- und Acetal-Funktionen, wobei X in α- oder β-Isomerie vorliegen kann.

6. Verfahren nach Anspruch 5, dadurch gekennzeichnet, daß Y, $R_4$ und $R_5$ ausgewählt werden unter (H, OH, H), (OH, OH, H), (H, H, OH), (H, OH, OH), (OH, H, OH) und (OH, H, H).

7. Verfahren nach einem der vorstehenden Ansprüche, dadurch gekennzeichnet, daß X das (2-Z)-ethyl, ein Methyl oder ein Phenyl darstellt.

8. Verfahren nach einem der vorstehenden Ansprüche, dadurch gekennzeichnet, daß Z die Gruppe

$$-O-\underset{\underset{O}{\|}}{C}-(CH_2)_n-COOH$$

mit n zwischen 0 und 10 oder die Gruppe OH ist.

9. Methode zur Bestimmung von Metaboliten des Vitamins D, dadurch gekennzeichnet, daß sie die Verwendung eines Tracers als Reagens umfaßt, der aus der Verbindung eines Derivates der Formel I mit einem Markierungs-Element, insbesondere einem Enzym oder einem Iod-Molekül, besteht, oder eines Antikörpers, der durch Inokulation eines Antigens, hergestellt durch kovalente Bindung eines Derivates von Vitamin D der Formel I nach einem der vorstehenden Ansprüche mit einem Immunträger-Protein über eine Ableitung in $C_{11}$ des genannten Derivates, in einen Tierwirt induziert wird.

10. Methode nach Anspruch 9, dadurch gekennzeichnet, daß der Tracer aus 11a-[2-0-(m$^{125}$I-Tyramidosuccinoyl)-ethyl]-25-hydroxy-Vitamin $D_3$ (58) besteht.

**Claims**

**Claims for the following Contracting States: AT, BE, CH, DE, FR, GB, GR, IT, LI, LU, NL, SE**

1. Vitamin D derivatives corresponding to the following formula I

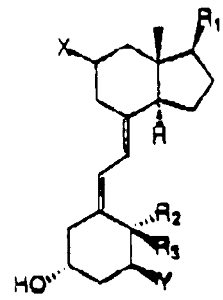

I

in which

$R_1$ denotes a substituted alkyl group having 1 to 15 carbon atoms, in particular the side chains of vitamin $D_2$ ($C^{20}$ to $C^{28}$) or $D_3$ ($C^{20}$ to $C^{27}$), or these same chains partially modified, in particular

- hydroxylated at one or mote positions, for example the 24-, 25- and/or 26-positions, and/or
- methylated or ethylated at one or mote positions, for example the 24-, 26- and/or 27-positions, and/or
- halogenated or polyhalogenated at one or more positions, for example perfluorinated (trifluoromethyl) at the 26- and 27-positions, or difluorinated at the 24-position, and/or
- by the addition of one or mote carbon atoms, in particular an atom $C^{24'}$ between the 24- and 25-positions, with the same possibilities of substitution as mentioned above, and/or
- esterified on one or mote hydroxyl groups mentioned above, and/or
- etherified on one or mote hydroxyl groups mentioned above, and/or
- by replacing one or more carbon atoms by an oxygen, nitrogen or sulfur atom, for example an oxygen at the 22-, 23- or 24-positions, and/or
- cyclized between carbons $C^{26}$ and $C^{27}$ by a direct bond (cyclopropane) or via a chain of 1 to 3 carbon atoms; it being possible for each of these atoms to beat all the groups or modifications described above, and/or
- substituted at one or more positions with a saturated, unsaturated, aromatic or heteroaromatic ring, capable of bearing all the groups and modifications described above, and/or
- unsaturated, with one or mote carbon-carbon double or triple bond(s); it being possible for these unsaturated alkyl groups to bear all the groups and modifications described above, and
- the isomeric forms of the different groups situated on the chain;

Y denotes H or OH or groups detived from the latter such as eater and ether;

X denotes

- an alkyl chain, in particular of 1 to 6 carbon atoms, optionally substituted at different points by one or more functional group(s) Z, which can be, in particular, a halogen (such as fluorine), a hydroxyl, formyl, carboxyl, amine, thiol, cyano, nitro, sulfoxide, sulfone or phosphono group, or alternatively groups derived from these latter, such as ether, ester, acetal, amide, hydrazine, phosphate or bis(phosphate), or
- an unsaturated alkyl chain having one or more carbon-carbon double or triple bond (s), it being possible for these chains, in addition, to bear functihain having oneentioned above, or
- an aromatic or heteroaromatic ring, optionally substituted with halogens, one (or more) hydroxyl, amine, formyl, carboxyl, thiol, cyano or nitro group(s), or alternatively groups derived from these latter, such as ether, ester, acetal or amide, or
- a halogen, a cyano, sulfoxide, sulfone, hydroxyl, thiol or amine group, or alternatively derivatives of these latter, such as ether, ester, amine amd hydrazine;

$R_2$ denotes a methyl group and $R_3$ an H, or $R_2$ is H and $R_3$ is methyl, or $R_2$ and $R_3$ are H, or alternatively $R_2$ and $R_3$ together denote a methylene group = $CH_2$.

2. The derivatives as claimed in claim 1, in which $R_1$ denotes one of the following chains

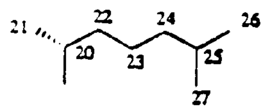

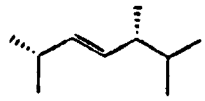

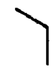

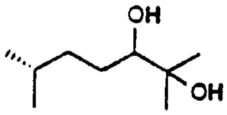

24 R ou S

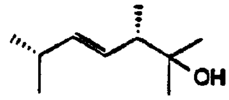

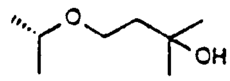

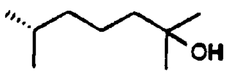

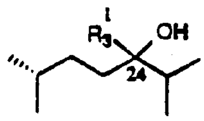

24 R ou S
$R_3^1$ = H ou $CH_3$

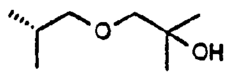

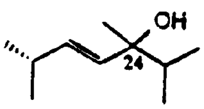

24 R ou S

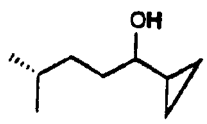

24 R ou S

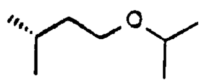

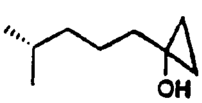

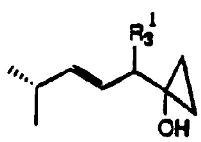

$R_3^1$ = H ou $CH_3$

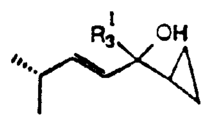

24 R ou S
$R_3^1$ = H ou $CH_3$

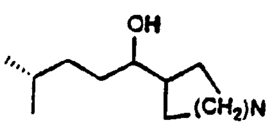

24 R ou S
n = 1,2,3

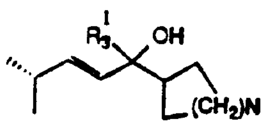

24 R ou S
n = 1,2 3
$R_3$ = H ou $CH_3$

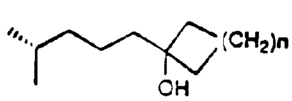

n = 1,2,3

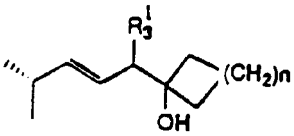

n = 1,2,3
$R_3^1$ = H ou $CH_3$ ( R ou S )

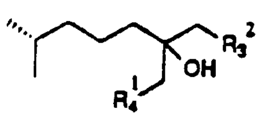

$R_3^2$ = H, $R_4^1$ = $CH_3$ ( R ou S )
$R_3^2$ = $R_4^1$ = $CH_3$

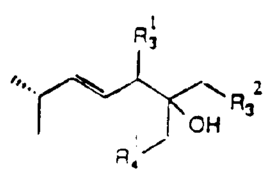

$R_3^2$ = H, $R_4^1$ = $CH_3$ ( R ou S )
$R_3^2$ = $R_4^1$ = $CH_3$
$R_3^1$ = H ou $CH_3$ ( R ou S )

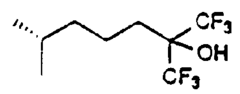

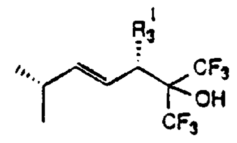

$R'_3$ = H ou $CH_3$ ( R,S )

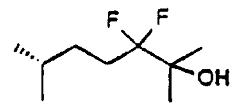

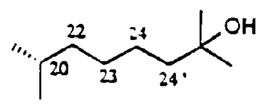

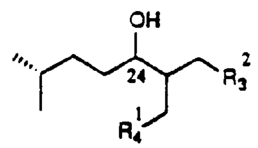

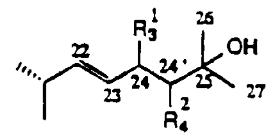

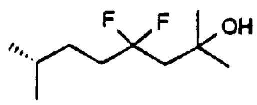

$R_3^2$ = H, $R_4^1$ = $CH_3$ ( R ou S )
24 R ou S

$R_3^1$ = $R_4^2$ = H

$R_3^1$ = $CH_3$ ( R ou S ), $R_4^2$ = H

$R_3^1$ = H, $R_4^2$ = $CH_3$ (R ou S )

3. Vitamin $D_3$ derivatives corresponding to the formula I′

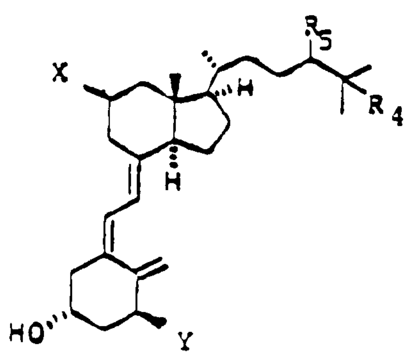

I′

in which

Y, $R_4$ and $R_5$ denote H or OH, and

X denotes an alkyl chain, in particular of 1 to 6 carbon atoms, optionally substituted, in particular at its terminal end, with a functional group Z which can be chosen, in particular, from hydroxy, formyl, carboxyl, amine and amine groups, and derived groups such as ester, ether and acetal groups, X being in the α or β isomeric form.

4. The derivatives as claimed in claim 3, in which (Y, $R_4$, $R_5$) is chosen from (H, OH, H), (OH, OH, H), (H, H, OH), (H, OH, OH), (OH, H, OH), (OH, H, H).

5. The derivatives as claimed in one of the preceding claims, in which X denotes (2-Z-ethyl), a methyl or a phenyl.

6. The derivatives as claimed in one of the preceding claims, in which Z denotes a

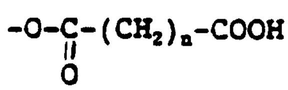

group with n between 2 and 10, or an OH group.

7. A method for preparing a derivative as claimed in one of the preceding claims, wherein a derivative of formula II (IIα or IIβ)

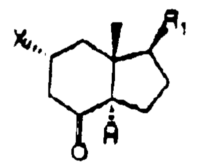

IIα

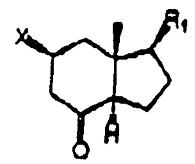

IIβ

in which X and $R_1$ have the meanings given above, these derivatives being, however, protected, if necessary, on their functional group, is reacted with a derivative of formula III

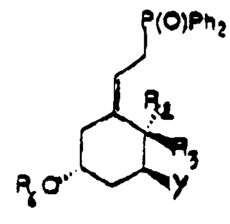

III

$R_2$ and $R_3$ = $CH_2$
$R_2$ = Me, $R_3$ = H
$R_2$ = H, $R_3$ = Me
Y = H or OR′

in which Y, $R_2$ and $R_3$ have the meanings given above, where appropriate being a hydroxyl group protected, in particular, by a group which protects hydroxyl groups, such as dimethyl-t-butylsilyl; and
$R_6$ is a group which protects the hydroxyl group.
This reaction of the compound of formula II with the compound of formula III is carried out in the presence of n-butyllithium,
and the protected groups are then deprotected.

8. The method for preparing a derivative as claimed in the preceding claim, wherein a derivative of formula II′ (II′α or II′β)

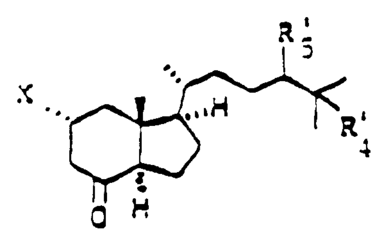

(II'α)

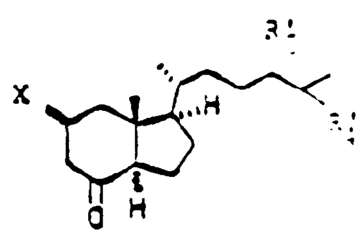

(II'β)

the terminal group of X being protected by a protective group, is reacted with a derivative of formula III′

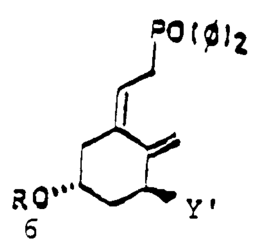

III'

in which Y′, $R'_4$ and $R'_5$ denote H or a hydroxyl group protected, in particular, by dimethyl-t-butylsilyl, and $R_6$ denotes a group which protects hydroxyl.

9. The method for preparing a derivative of formula I as claimed in claim 8, wherein the derivative is prepared with Z denoting an optionally protected hydroxyl group, which is then optionally converted to another functional group.

10. By way of drugs, derivatives as claimed in one of claims 1 to 6.

11. An antigen, which is prepared by the covalent binding of a vitamin D derivative of formula I as claimed in one of the preceding claims, with an immunogenic carrier protein, via a branch at $C^{11}$ of the said derivative.

12. An antibody which is induced by the inoculation in a host animal of the antigen as claimed in one of claims 1 to 11.

13. A tracer which is useful for assays, which consists of the conjugation of a derivative of formula I as claimed in one of claims 1 to 12, with a labeling component, in particular an enzyme or an iodinated molecule.

14. The tracer as claimed in claim 13, which consists of 11α-[2-(m-$^{125}$I-tyramidosuccinoyloxy)ethyl]-25-hydroxy-vitamin $D_3$ (58).

15. An assay method for metabolites of vitamin D, which comprises the use as a reagent of the tracers as claimed in claims 13 and 14 or the antibodies as claimed in claim 12.

16. Compound useful especially for the preparation of vitamin derivatives of claim 1 corresponding to the formula

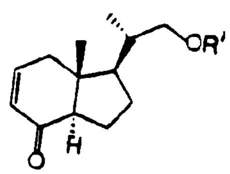

in which OR′= OH or a derivative group thereof such as ester and ether.

**Claims for the following Contracting State: ES**

1. A method for preparing a vitamin D corresponding to the following formula I

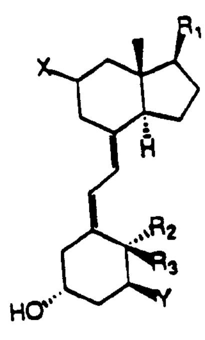

I

in which

$R_1$ denotes a substituted alkyl group having 1 to 15 carbon atoms, in particular the side chains of vitamin $D_2$ ($C^{20}$ to $C^{28}$) or $D_3$ ($C^{20}$ to $C^{27}$), or these same chains partially modified, in particular

- hydroxylated at one or more positions, for example the 24-, 25-and/or 26- positions, and/or
- methylated or ethylated at one or more positions, for example the 24-, 26- and/or 27 positions, and/or
- halogenated or polyhalogenated at one or more positions, for example perfluorinated (trifluoromethyl) at the 26- and 27- positions, or difluorinated at the 24- position, and/or
- by the addition of one or more carbon atoms, in particular an atom $C^{24}$ between the 24- and 25- positions, with the same possibilities of substitutionas mentioned above, and/or
- esterified on one or more hydroxyl groups mentioned above, and/or
- etherified on one or more hydroxyl groups mentioned above, and/or
- by replacing one or more carbon atoms by an oxygen, nitrogen or sulfur atom, for example an oxygen at the 22-, 23- or 24- positions, and/or
- cyclized between carbons $C^{26}$ and $C^{27}$ by a direct bond (cyclopropane) or via a chain of 1 to 3 carbon atoms; it being possible for each of these atoms to bear all the groups or modifications described above, and/or
- substituted at one or more positions with a saturated, unsaturated, aromatic or heteroaromatic ring, capable of bearing all the groups and modifications described above, and/or
- unsaturated, with one or more carbon-carbon double or triple bond(s); it being possible for these unsaturated alkyl groups to bear all the groups and modifications described above, and
- the isomeric forms of the different groups situated on the chain;

Y denotes H or OH or groups derived from the latter such as ester and ether;

X denotes
- an alkyl chain, in particular of 1 to 6 carbon atoms, optionally substituted at different points by one or more functional group(s) Z, which can be, in particular, a halogen (such as fluorine), a hydroxyl, formyl, carboxyl, amine, thiol, cyano, nitro, sulfoxide, sulfone or phosphono group, or alternatively groups derived from these latter, such as ether, ester, acetal, amide, hydrazine, phosphate or bis(phosphate), or
- an unsaturated alkyl chain having one or more carbon-carbon double or triple bond(s), it being possible for these chains, in addition, to bear functional groups mentioned above, or
- an aromatic or heteroaromatic ring, optionally substituted with halogens, one (or more) hydroxyl, amine, formyl, carboxyl, thiol, cyano or nitro group(s), or alternatively groups derived from these latter, such as ether, ester, acetal or amide, or
- a halogen, a cyano, sulfoxide, sulfone, hydroxyl, thiol or amine group, or alternatively derivatives of these latter, such as ether, ester, amine and hydrazine;

$R_2$ denotes a methyl group and $R_3$ an H, or $R_2$ is H and $R_3$ is methyl, or $R_2$ and $R_3$ are H, or alternatively $R_2$ and $R_3$ together denote a methylene group = $CH_2$,
wherein a derivative of formula II (IIα or IIβ)

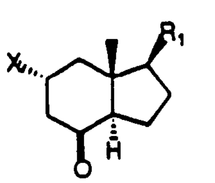

II α

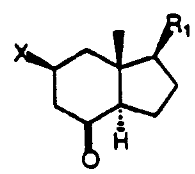

II β

in which X and $R_1$ have the meanings given above, these derivatives being, however, protected, if necessary, on their functional group, is reacted with a derivative of formula III

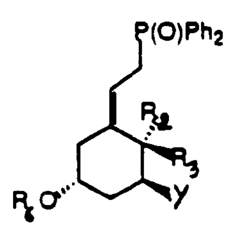

III

$R_2$ and $R_3$ = $CH_2$
$R_2$ = Me, $R_3$ = $CH_2$
$R_2$ = H, $R_3$ = Me
Y = H or OR′

in which Y, $R_2$ and $R_3$ have the meanings given above, where appropriate being a hydroxyl group protected, in particular, by a group which protects hydroxyl groups, such as dimethyl-t-butylsilyl; and
$R_6$ is a group which protects the hydroxyl group.
This reaction of the compound of formula II with the compound of formula III is carried out in the presence of n-butyllithium,
and the protected groups are then deprotected.

2. The method for preparing a derivative as claimed in the preceding claim, wherein a derivative of formula II′ (II′α or II′β)

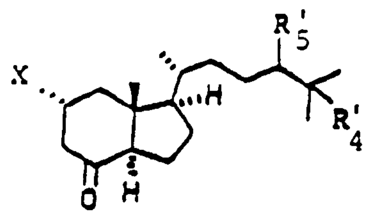

(II'α)

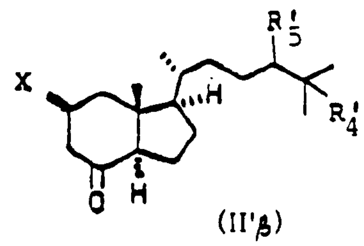

(II'β)

the terminal group of X being protected by a protective group, is reacted with a derivative of formula III′

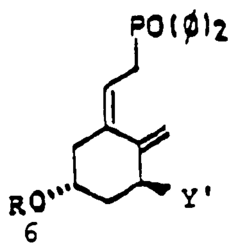

III'

in which Y′, $R'_4$ and $R'_5$ denote H or a hydroxyl group protected, in particular, by dimethyl-t-butylsilyl, and $R_6$ denotes a group which protects hydroxyl.

3. The method for preparing a derivative of formula I as claimed in claim 2, wherein the derivative is prepared with Z denoting an optionally protected hydroxyl group, which is then optionally converted to another functional group.

4. The Process as claimed in any of claims 1 to 3, in which $R_1$ denotes one of the following chains

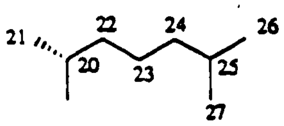

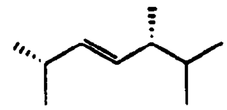

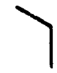

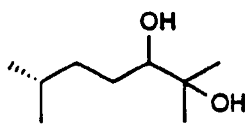

24 R ou S

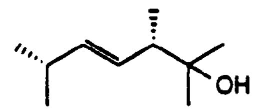

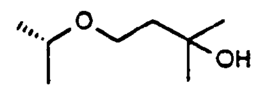

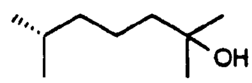

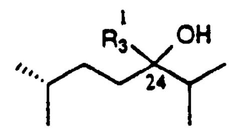

24 R ou S
$R'_3$ = H ou $CH_3$

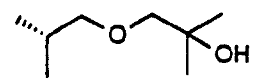

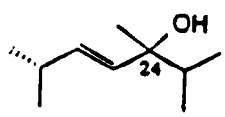

24 R ou S

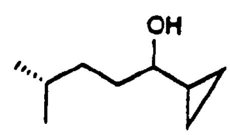

24 R ou S

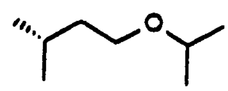

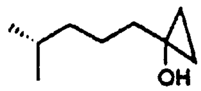

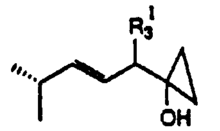

$R_3^1$ = H ou $CH_3$

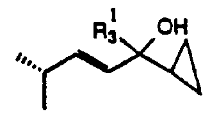

24 R ou S
$R_3^1$ = H ou $CH_3$

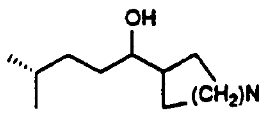

24 R ou S
n = 1,2,3

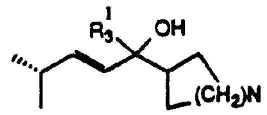

24 R ou S
n = 1,2 3
$R_3$ = H ou $CH_3$

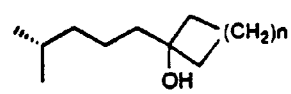

n = 1,2,3

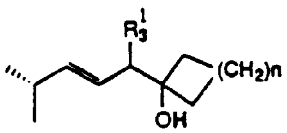

n = 1,2,3
$R_3^1$ = H ou $CH_3$ ( R ou S )

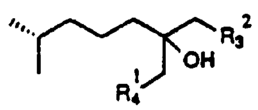

$R_3^2$ = H , $R_4^1$ = $CH_3$ ( R ou S )
$R_3^2$ = $R_4^1$ = $CH_3$

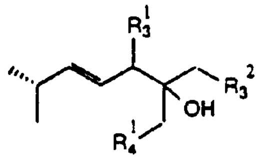

$R_3^2$ = H , $R_4^1$ = $CH_3$ ( R ou S )
$R_3^2$ = $R_4^1$ = $CH_3$
$R_3^1$ = H ou $CH_3$ ( R ou S )

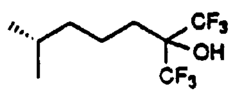

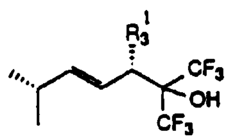

$R'_3$ = H ou $CH_3$ ( R,S )

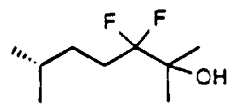

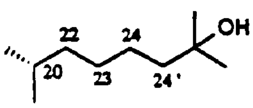

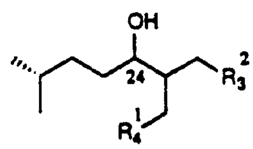

$R_3^2$ = H , $R_4^1$ = $CH_3$ ( R ou S )
24 R ou S

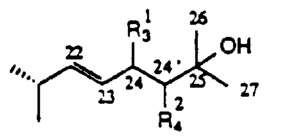

$R_3^1$ = $R_4^2$ = H
$R_3^1$ = $CH_3$ ( R ou S ) , $R_4^2$ = I
$R_3^1$ = H , $R_4^2$ = $CH_3$ (R ou S

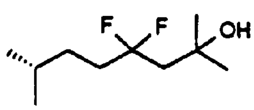

**5.** The process as claimed in any of claims 1 to 4 wherein the formula I′

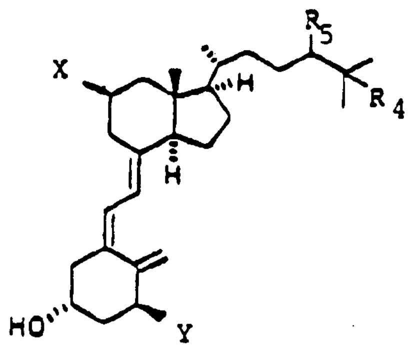

I'

in which Y, $R_4$ and $R_5$ denote H or OH, and

X denotes an alkyl chain, in particular of 1 to 6 carbon atoms, optionally substituted, in particular at its terminal end, with a functional group Z which can be chosen, in particular, from hydroxyl, formyl, carboxyl, amine and amide groups, and derived groups such as ester, ether and acetal groups, X being in the α or β isomeric form.

**6.** The process as claimed in claim 5, in which (Y, $R_4$, $R_5$) is chosen from (H, OH, H), (OH, OH, H), (H, H, OH), (H, OH, OH), (OH, H, OH), (OH, H, H).

**7.** The process as claimed in one of the preceding claims, in which X denotes (2-Z-ethyl), a methyl or a phenyl.

**8.** The process as claimed in one of the preceding claims, in which Z denotes a

$$-O-\underset{\underset{O}{\|}}{C}-(CH_2)_n-COOH$$

group with n between 2 and 10, or an OH group.

**9.** An assay method for metabolites of vitamin D, which comprises the use as a reagent of a tracer which is useful for assays, which consists of the conjugation of a derivative of formula I as claimed in one of the claims 1 to 8, with a labeling component, in particular an enzyme or an iodinated molecule or the antibodies induced by the inoculation in a host animal of an antigen prepared by the covalent binding of a vitamin D derivative of formula I as claimed in one of the preceding claims, with an immunogenic carrier protein, via a branch at $C^{11}$ of the said derivative.

**10.** The method as claimed in claim 9 wherein the tracer consists of 11 -[2-(m-$^{125}$I-tyramidosuccinoyloxy) ethyl]-25-hydroxy-vitamin $D_3$ (58).

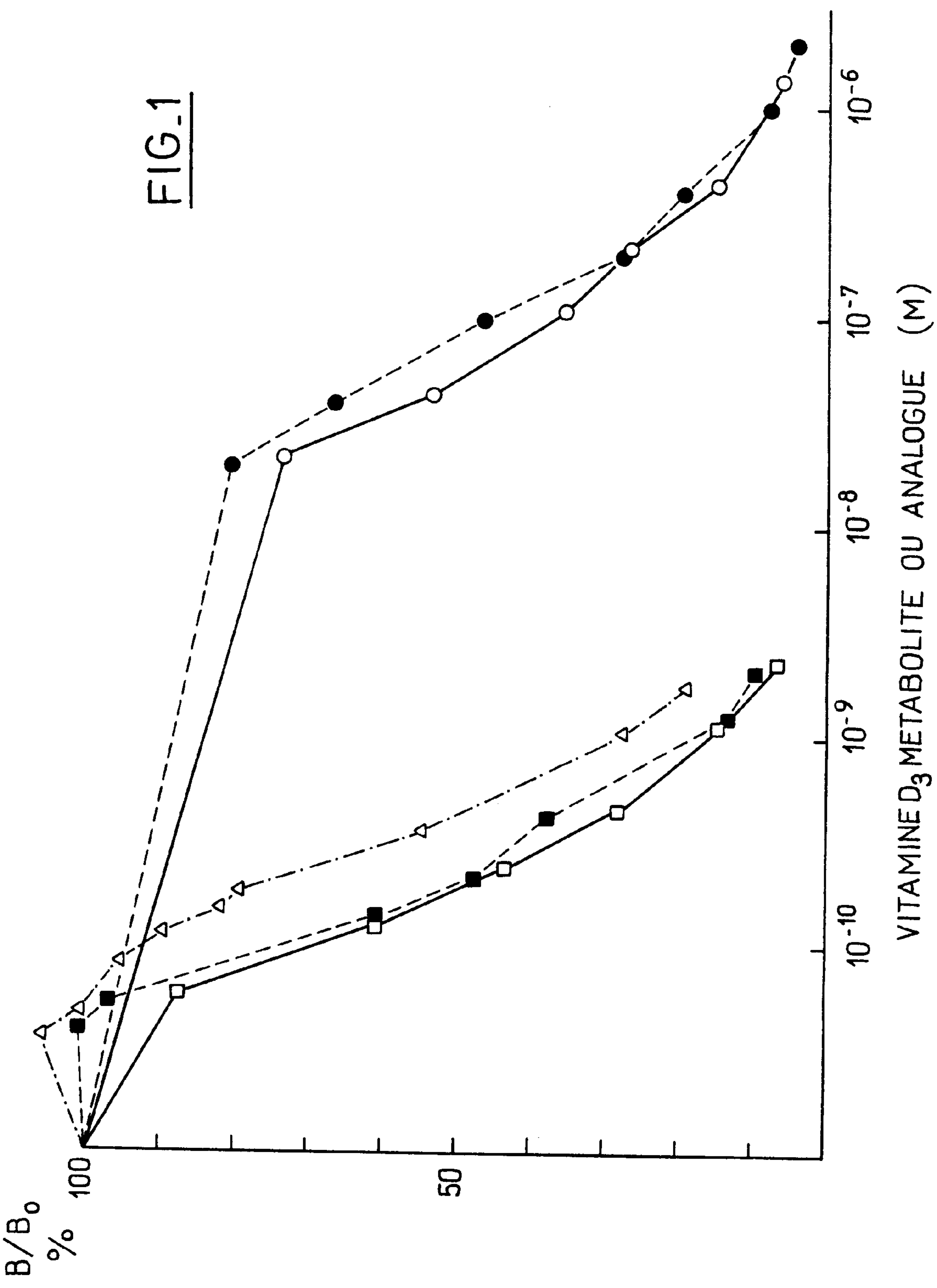
B/Bo
%
100
50
$10^{-10}$
$10^{-9}$
$10^{-8}$
$10^{-7}$
$10^{-6}$
VITAMINE $D_3$ METABOLITE OU ANALOGUE (M)

FIG_1

FIG_2

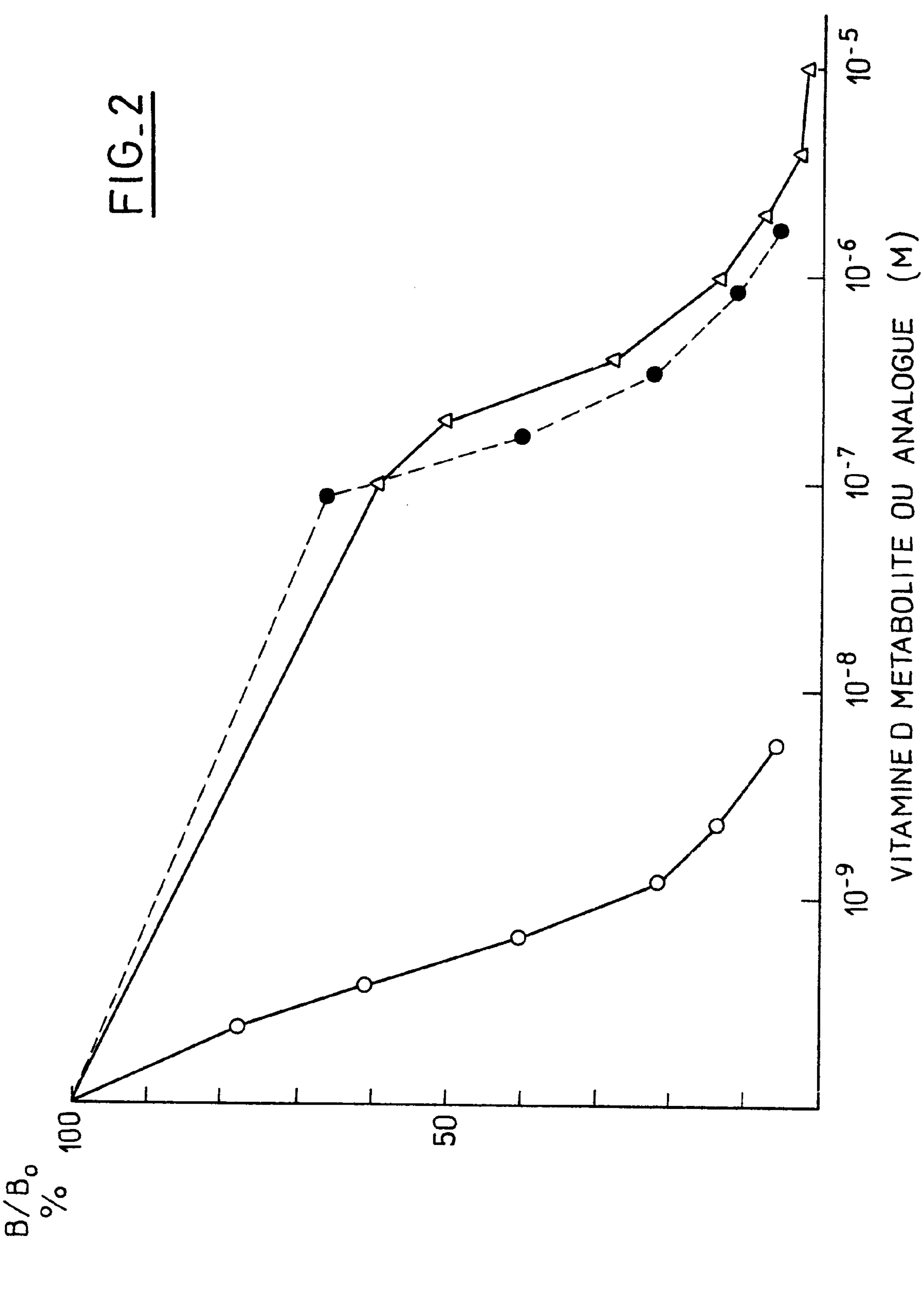
B/Bo %
100
50
$10^{-9}$
$10^{-8}$
$10^{-7}$
$10^{-6}$
$10^{-5}$
VITAMINE D METABOLITE OU ANALOGUE (M)

FIG_3

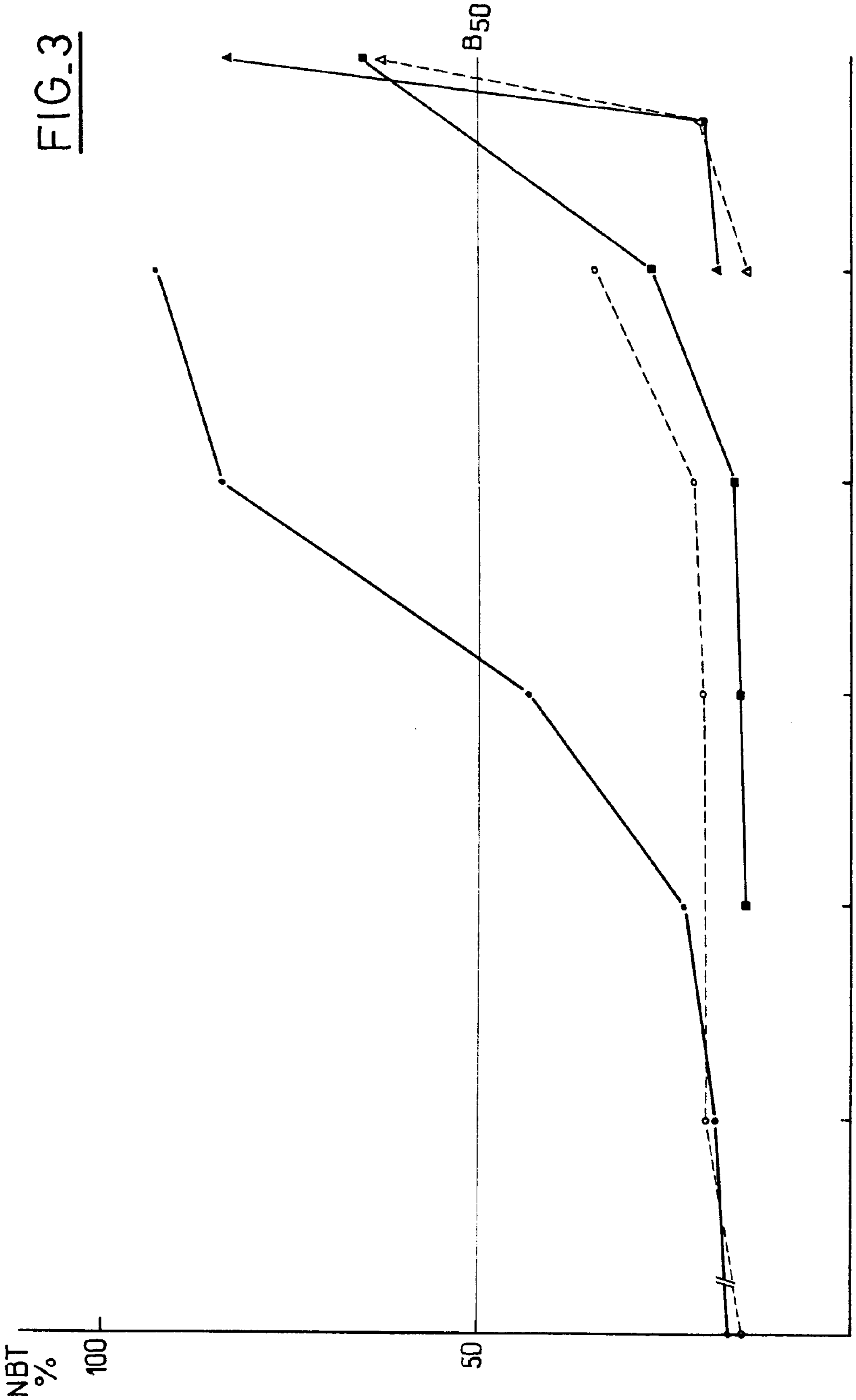

FIG_4